# EUROPEAN PATENT SPECIFICATION

(11) **EP 1 150 962 B1**
(45) Date of publication and mention of the grant of the patent: **28.04.2004**
(21) Application number: 00911810.0
(22) Date of filing: 14.02.2000
(51) Int. Cl.: C07D 239/54, C07D 417/04

(54) **PROCESS FOR THE PREPARATION OF 6-(PERFLUOROALKYL)URACIL COMPOUNDS FROM CARBAMATE COMPOUNDS**
VERFAHREN ZUR HERSTELLUNG VON 6-(PERFLUOROALKYL)URACILVERBINDUNGEN AUS CARBAMATVERBINDUNGEN
PREPARATION DE COMPOSES 6-(PERFLUOROALKYL)URACIL A PARTIR DE COMPOSES CARBAMATE

(30) Priority: 16.02.1999 US 250725
(43) Date of publication of application: 07.11.2001
(73) Proprietor: AMERICAN CYANAMID COMPANY, Madison, New Jersey 07940 (US)
(72) Inventor: KAMESWARAN, Venkataraman, Trenton, NJ 08638 (US)
(74) Representative: Meyer, Udo
(86) International application number: PCT/US2000/003795
(87) International publication number: WO 2000/049003

(56) References cited:
- DE-A- 19 508 590
- DE-A- 19 532 048
- US-A- 3 981 715

## Description

### BACKGROUND OF THE INVENTION

6-(Perfluoroalkyl)uracil compounds are useful as herbicidal agents and methods for their preparation are known in the art. 6-(Perfluoroalkyl)uracil compounds may be prepared by reacting 2-(N,N-disubstituted)amino-4-(perfluoroalkyl)-1,3-oxazin-6-one compounds with amine compounds.

Bull. Soc. Chem. Belg., *101*(4), pages 313-321 (1992) discloses that 2-(N,N-dialkyl)amino-4-(trifluoromethyl)-1,3-oxazin-6-one compounds are prepared by reacting ethyl 3-amino-4,4,4-trifluorocrotonate with phosgene iminium chloride compounds. However, this method is not entirely satisfactory because the required phosgene iminium chloride compounds are difficult to handle and relatively expensive. Accordingly, a need exists in the art for an improved process for the preparation of 6-(perfluoroalkyl)uracil compounds which avoids the use of 2-(N,N-disubstituted)-amino-4-(perfluoroalkyl)-1,3-oxazin-6-one compounds.

It is, therefore, an object of the present invention to provide an improved process for the preparation of 6-(perfluoroalkyl)uracil compounds which avoids the use of 2-(N,N-disubstituted)amino-4-(perfluoroalkyl)-1,3-oxazin-6-one compounds.

Other objects and advantages of the present invention will be apparent to those skilled in the art from the description below and the appended claims.

### SUMMARY OF THE INVENTION

The present invention provides a new process for the preparation of 6-(perfluoroalkyl)uracil compounds having the structural formula I 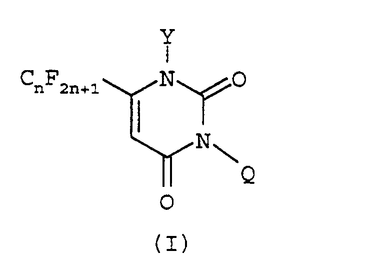 wherein
- n: is an integer of 1, 2, 3, 4, 5 or 6;
- Y: is hydrogen or C₁-C₆alkyl; and
- Q: is a C₁-C₆alkyl group or an optionally substituted phenyl, benzyl, heteroaryl or methyleneheteroaryl group,
which process comprises:
(a) reacting a carbamate compound having the structural formula II 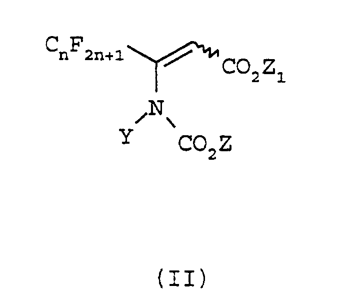 wherein
   - Z and Z₁: are each independently C₁-C₆alkyl or benzyl optionally substituted on the phenyl ring with any combination of from one to three halogen, C₁-C₄alkyl or C₁-C₄haloalkyl groups; and
   - n and Y: are as described above,
   with an amine compound having the structural formula III

   QNH₂ (III)

   wherein Q is as described above, in the presence of a base, to form the 6-(perfluoroalkyl)uracil compound of formula I
   wherein Y is hydrogen or C₁-C₆alkyl; and
(b) optionally alkylating the formula I compound
wherein Y is hydrogen to form the formula I compound
wherein Y is C₁-C₆alkyl.

### DETAILED DESCRIPTION OF THE INVENTION

In a preferred embodiment of the present invention, a carbamate compound of formula II is reacted with an amine compound of formula III and a base, preferably at a temperature ranging from about 20°C to 150°C, in the presence of a solvent.

In another preferred embodiment of the present invention, the double bond in the formula II compounds is predominately in the (Z)- configuration.

Advantageously, the present invention provides an improved process for the preparation of 6-(perfluoroalkyl)-uracil compounds which avoids the use of 2-(N,N-disubstituted)amino-4-(perfluoroalkyl)-1,3-oxazin-6-one compounds.

The product formula I compounds may be isolated using conventional isolation procedures such as diluting the reaction mixture with water and separating the product or extracting the product with a suitable extraction solvent. In the isolation procedure, conventional extraction solvents such as diethyl ether, ethyl acetate, toluene, methylene chloride, and the like, and mixtures thereof may be utilized.

Bases suitable for use in the process of the present invention include, but are not limited to, tri(C₁-C₆-alkyl)amines such as trimethylamine, triethylamine, tripropylamine, tributylamine, diisopropylethylamine and the like; heterocyclic tertiary amines such as 1,8-diazabicyclo[5.4.0]undec-7-ene (DBU), 1,5-diazabicyclo-[4.3.0]non-5-ene (DBN), 1,4-diazabicyclo[2.2.2]octane, pyridine, substituted pyridines, quinoline, substituted quinolines and the like; and alkali metal C₁-C₆alkoxides such as potassium tert-butoxide, sodium tert-butoxide and the like. Preferred bases include 1,8-diazabicyclo-[5.4.0]undec-7-ene and 1,5-diazabicyclo[4.3.0]non-5-ene.

Solvents suitable for use in step (a) of the process of this invention include, but are not limited to, carboxylic acid amides such as N,N-dimethylformamide, N,N-dimethylacetamide and the like; dialkyl sulfoxides such as dimethyl sulfoxide and the like; aromatic hydrocarbons such as toluene, benzene, xylenes, mesitylene and the like; halogenated aromatic hydrocarbons such as chlorobenzene, fluorobenzene and the like; aliphatic hydrocarbons such as pentane, hexane, heptane and the like; halogenated aliphatic hydrocarbons such as methylene chloride, chloroform, carbon tetrachloride, 1,2-dichloroethane and the like; alcohols such as methanol, ethanol, *n*-propanol, *sec*-propanol and the like; ketones such as acetone, methyl ethyl ketone and the like; ethers such as diethyl ether, tetrahydrofuran, dioxane, 1,2-dimethoxyethane and the like; nitriles such as acetonitrile, propionitrile and the like; and water; and mixtures thereof.

Alkylation procedures suitable for use in this invention include conventional procedures known in the art. In a preferred embodiment of this invention, the step (b) alkylation procedure comprises reacting the formula I compound wherein Y is hydrogen with an alkyl halide having the structural formula IV or a dialkylsulfate ester having the structural formula V

XY (IV)

or 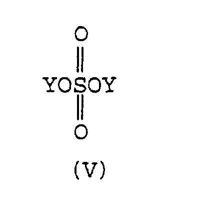 wherein X is chlorine, bromine or iodine, and Y is C₁-C₆alkyl in the presence of a base.

Bases suitable for use in the alkylation procedures of this invention include conventional bases known in the art including, but not limited to, alkali metal hydrides such as sodium hydride and the like; alkali metal C₁-C₆alkoxides such as potassium tert-butoxide, sodium tert-butoxide and the like; alkali metal hydroxides such as potassium hydroxide, sodium hydroxide and the like; alkali metal carbonates such as sodium carbonate, potassium carbonate and the like; alkaline earth metal hydroxides such as calcium hydroxide and the like; and alkaline earth metal carbonates such as calcium carbonate and the like.

Preferred formula II compounds for use in the process of this invention are those wherein
- Y: is hydrogen or C₁-C₄alkyl;
- Z and Z₁: are each independently C₁-C₄alkyl; and
- n: is 1.

More preferred carbamate compounds of formula II for use in the process of the present invention are those
wherein
- Y: is hydrogen or methyl;
- Z and Z₁: are each independently methyl or ethyl; and
- n: is 1.

Preferred formula I compounds which may be prepared by the process of the present invention are those wherein
n is 1;
Y is hydrogen or C₁-C₄alkyl;
Q is 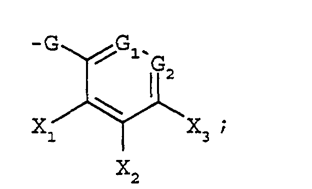
G is CH₂ or a bond;
G₁ is CX₅ or N;
G₂ is CX₄ or N;
X₁ is hydrogen, halogen or a C₁-C₆alkyl group optionally substituted with one epoxy group,
X₂ is hydrogen, halogen NRR₁, CO₂R₂, C(O)R₃, OR₄, SO₂R₅, SO₂NR₆R₇, C(R₈)(OR₉)₂, C(R₁₀)=NOR₁₁, C(R₁₂)=C(R₁₃)-C (OR₁₄)=NOR₁₅, CH₂O-NCO₂R₁₆,
   1,3-dioxolane optionally substituted with one C₁-C₆alkoxy group or one or two C₁-C₄alkyl groups,
   1,3-dioxolinone optionally substituted with one C₁-C₆alkoxy group or one or two C₁-C₄alkyl groups, or
   C₁-C₄alkyl optionally substituted with one CO₂R₂ group and one halogen atom, and
X₃ is hydrogen, halogen, C₁-C₄haloalkyl, CO₂R₁₇, cyano, C₁-C₄haloalkoxy, OR₁₈ or C₁-C₄alkyl, or
when X₁ and X₂ are taken together with the atoms to which they are attached, they may form a five- or six-membered ring wherein X₁X₂ or X₂X₁ is represented by: -OC(R₂₀)(R₂₁)O-, -CH₂S(O)ₚN(R₂₂)-, -SC(R₂₃)=N-, -CH=CH-CH(R₁₁)O-, -OC(O)N-, -SC(R₂₄)=N-, -ON(R₂₅)C(O)-, -OC(CO₂R₂₆)=C(R₂₇)-, -NC(R₂₈)=C(SR₂₉)-, -CH=C(CO₂R₃₀)O-, - CH₂CH(R₃₁)O- or -OC(R₃₂)(R₃₃)C(O)-, or
when X₂ and X₃ are taken together with the atoms to which they are attached, they may form a five- or six-membered ring wherein X₂X₃ or X₃X₂ is represented by : -NC(R₃₄)=NC(S)-, -N(R₃₅)N=C(R₃₆)-, -N(R₃₇)C(R₃₈)=N-, - N(R₃₈)C(O)CH₂O-, -N(R₃₉)C(O)CH=CH-, -S-N=C(R₄₀)-, - O-N=C(R₄₁)-, -N=N-N(R₄₂)-, -C(R₄₃)(R₄₄)C(O)N(R₄₅)- or -N(R₄₆)C(O)C(R₄₇)(R₄₈)-;
X₄ is hydrogen, halogen or OR₁₉;
X₅ is hydrogen or halogen;
R, R₅₆, R₆₄, R₆₉, R₇₀, R₇₇ and R₉₁ are each independently hydrogen, SO₂R₄₉, C₁-C₄alkyl, C₃-C₇cycloalkyl, C₃-C₆alkenyl, C₃-C₆alkynyl, phenyl or benzyl;
R₁ is hydrogen, SO₂R₅₀, C(O)R₅₁, amino or C₁-C₄alkyl optionally substituted with CO₂R₅₂ or C(O)R₅₃;
R₂, R₁₆, R₁₇, R₂₆, R₃₀, R₆₈, R₇₅, R₇₆, R₈₂ and R₈₈ are each independently hydrogen, C₁-C₈haloalkyl, C₃-C₈alkenyl, C₃-C₆alkynyl, phenyl, benzyl, furfuryl, pyridyl, thienyl,
   C₁-C₈alkyl optionally substituted with CO₂R₅₄, morpholine or C(O)R₅₅, or
   an alkali metal, an alkaline earth metal, ammonium or organic ammonium cation;
R₃, R₆₆, R₆₇, R₈₁, R₈₅ and R₈₉ are each independently hydrogen, C₁-C₆alkyl, C₃-C₆alkenyl, C₃-C₆alkynyl, NR₅₆R₅₇, phenyl or benzyl;
R₄, R₁₈, R₁₉ and R₆₅ are each independently hydrogen, C₁-C₆alkyl, C₃-C₆alkenyl, C₃-C₆alkynyl, C₁-C₄haloalkyl, C(O)R₅₈, C(S)R₅₉ or benzyl;
R₅ and R₇₂ are each independently C₁-C₆alkyl, C₁-C₆haloalkyl, NR₆₀R₆₁, imidazole or indazole;
R₆, R₁₁, R₁₂, R₁₄, R₁₅, R₂₀, R₂₁, R₂₂, R₂₅, R₂₈, R₂₉, R₃₁, R₃₂, R₃₃, R₃₅, R₄₅, R₄₆, R₆₃ and R₈₀ are each independently hydrogen or C₁-C₄alkyl;
R₇ is hydrogen, C₃-C₆alkenyl, C₃-C₆alkynyl, benzyl, or C₁-C₄alkyl optionally substituted with cyano or C(O) R₆₂;
R₈ and R₂₇ are each independently hydrogen, C₁-C₄alkyl or C₁-C₄alkoxy;
R₉ and R₉₀ are each independently C₁-C₆alkyl;
R₁₀ is hydrogen, C₁-C₆alkyl, phenyl or benzyl;
R₁₃, R₂₄ and R₃₆ are each independently hydrogen, C₁-C₆alkyl or halogen;
R₂₃ is hydrogen or NR₆₃R₆₄;
R₃₄ is hydrogen, C₁-C₄alkyl or C₁-C₄haloalkyl;
R₃₇ is hydrogen, C₁-C₄alkyl or C₂-C₈alkoxyalkyl;
R₃₈ and R₃₉ are each independently hydrogen, C₁-C₄alkyl, C₁-C₄haloalkyl, C₃-C₆alkenyl or C₃-C₆alkynyl;
R₄₀, R₄₁ and R₄₂ are each independently hydrogen, halogen, cyano, OR₆₅, C(O)R₆₆, C(S)R₆₇, CO₂R₆₈, C(=NOR₆₉),
   a C₁-C₈alkyl, C₃-C₇cycloalkyl, C₂-C₈alkenyl or C₂-C₈alkynyl group, wherein each group is optionally substituted with any combination of one to six halogen atoms, one to three C₁-C₁₀alkoxy groups, one or two C₁-C₆haloalkoxy groups, one or two NR₇₀R₇₁ groups, one or two S(O)_{q}R₇₂ groups, one or two cyano groups, one or two C₃-C₇cycloalkyl groups, one OSO₂R₇₃ group, one or two C(O)R₇₄ groups, one or two CO₂R₇₅ groups, one or two C(O)SR₇₆ groups, one or two C(O)NR₇₇R₇₈ groups, one to three OR₇₉ groups, one or two P(O) (OR₈₀)₂ groups, one 1,3-dioxolane optionally substituted with one to three C₁-C₄alkyl groups, or one 1,3-dioxane optionally substituted with one to three C₁-C₄alkyl groups, or
   phenyl or benzyl optionally substituted with any combination of one to three halogen atoms, one to three C₁-C₆alkyl groups, one to three C₁-C₆alkoxy groups, one C₃-C₇cycloalkyl group, one C₁-C₄haloalkyl group, one C₁-C₄alkylthio group, one cyano group, one nitro group, one C(O)R₈₁ group, one CO₂R₈₂ group, one OR₈₃ group, one SR₈₄ group, one C₁-C₆alkoxymethyl group, one hydroxymethyl group, one C₃-C₈alkenyloxymethyl group, or one C₁-C₈haloalkoxymethyl group;
R₄₃, R₄₄, R₄₇ and R₄₈ are each independently hydrogen, C₁-C₄alkyl, C₁-C₄haloalkyl, C₃-C₆alkenyl, C₃-C₆alkynyl or C₃-C₇cycloalkyl, or R₄₃ and R₄₄ or R₄₇ and R₄₈ may be taken together with the atom to which they are attached to form a C₃-C₇cycloalkyl group;
R₄₉, R₅₀ and R₈₆ are each independently C₁-C₆alkyl, NR₉₃R₉₄, C₁-C₄haloakyl, C₃-C₆alkenyl, C₃-C₆alkynyl or benzyl;
R₅₁, R₅₂, R₅₃, R₅₄, R₅₅, R₅₇, R₅₈, R₅₉,R₆₀, R₆₁, R₆₂, R₇₁, R₇₃, R₇₄, R₇₈, R₈₇ and R₉₂ are each independently hydrogen, C₁-C₆alkyl, C₃-C₇cycloalkyl, C₁-C₆haloalkyl, C₃-C₆alkenyl, C₃-C₆alkynyl, phenyl or benzyl;
R₇₉, R₈₃ and R₈₄ are each independently hydrogen, C(O)R₈₅, SO₂R₈₆, C₁-C₆haloalkyl, C₂-C₆alkenyl, C₅-C₈cycloalkenyl, C₂-C₆alkynyl, phenyl, benzyl, or C₁-C₁₀alkyl optionally substituted with one hydroxyl, benzyloxy, OC(O)R₈₇, C₁-C₆alkoxy, CO₂R₈₈, C(O)R₈₉, C(OR₉₀)₂, C(O)NR₉₁R₉₂ or cyano group;
R₉₃ and R₉₄ are each independently hydrogen, C₁-C₄haloalkyl, C₂-C₆alkenyl, C₃-C₈cycloalkyl,
   C₁-C₈alkyl optionally substituted with one or two C₁-C₄alkoxy groups or one cyanoalkyl group, or benzyl or phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one cyano group or one nitro group, and
   when R₉₃ and R₉₄ are taken together with the atom to which they are attached, they form a 5- to 12-membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy and C₁-C₉haloalkylsulfonyl groups; and
p and q are each independently 0, 1 or 2; and
the optical isomers, diastereomers and/or tautomers thereof.

More preferred formula I herbicidal agents which may be prepared by the process of this invention are those
wherein
n is 1;
Y is hydrogen or methyl;
Q is 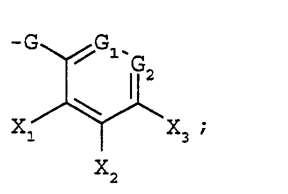
G is CH₂ or a bond;
G₁ is CX₅ or N;
G₂ is CX₄ or N;
X₁ is hydrogen, fluorine or C₁-C₃alkyl optionally substituted with one epoxy group;
X₂ is hydrogen, halogen NRR₁, CO₂R₂, C(O)R₃, OR₄, SO₂R₅, SO₂NR₆R₇, C(R₈)(OR₉)₂, C(R₁₀)=NOR₁₁, C(R₁₂)=C(R₁₃)-C(OR₁₄)=NOR₁₅⁻; CH₂O-NCO₂R₁₆,
   1,3-dioxolane optionally substituted with one C₁-C₆alkoxy group or one or two C₁-C₄alkyl groups,
   1,3-dioxolinone optionally substituted with one C₁-C₆alkoxy group or one or two C₁-C₄alkyl groups, or
   C₁-C₄alkyl optionally substituted with one CO₂R₂ group and one halogen atom, and
X₃ is hydrogen, halogen, C₁-C₄haloalkyl, CO₂R₁₇, cyano, C₁-C₄haloalkoxy, OR₁₈ or C₁-C₄alkyl, or
when X₁ and X₂ are taken together with the atoms to which they are attached, they may form a five- or six-membered ring wherein X₁X₂ or X₂X₁ is represented by: -OC(R₂₀)(R₂₁)O-, -CH₂S(O)ₚN(R₂₂)-, -SC(R₂₃)=N-, -CH=CH-CH(R₁₁)O-, -OC(O)N-, -SC(R₂₄)=N-, -ON(R₂₅)C(O)-, - OC(CO₂R₂₆)=CH-, -NC(R₂₈)=C(SR₂₉)-, -CH=C(CO₂R₃₀)O-, - CH₂CH(R₃₁)O- or -OC(R₃₂)(R₃₃)C(O)-, or
when X₂ and X₃ are taken together with the atoms to which they are attached, they may form a five- or six-membered ring wherein X₂X₃ or X₃X₂ is represented by: -NC(R₃₄)=NC(S)-, -N(R₃₅)N=C(R₃₆)-, -N(R₃₇)C(R₃₈)=N-, -N(R₃₈)C(O)CH₂O-, -N(R₃₉)C(O)CH=CH-, -S-N=C(R₄₀)-, -O-N=C(R₄₁)-, -N=N-N(R₄₂)-, -C(R₄₃)(R₄₄)C(O)N(R₄₅)- or -N(R₄₆)C(O)C(R₄₇)(R₄₈)-;
X₄ is hydrogen, halogen or OR₁₉;
X₅ is hydrogen or halogen;
R, R₆₄, R₆₉ and R₇₇ are each independently hydrogen, SO₂R₄₉ or C₁-C₄alkyl;
R₁ is hydrogen, SO₂R₅₀, C(O)R₅₁, amino or C₁-C₄alkyl optionally substituted with CO₂R₅₂ or C(O)R₅₃;
R₂, R₁₆, R₁₇, R₂₆, R₃₀, R₆₈, R₇₅, R₇₆, R₈₂ and R₈₈ are each independently hydrogen, C₃-C₆alkenyl or C₁-C₄alkyl optionally substituted with CO₂R₅₄, morpholine or C(O)R₅₅;
R₃, R₆₆, R₆₇, R₈₅ and R₈₉ are each independently hydrogen, C₁-C₄alkyl or NR₅₆R₅₇;
R₄, R₁₈ and R₁₉ are each independently hydrogen, C₁-C₄alkyl, C₁-C₄haloalkyl, C(O)R₅₈, C₃-C₄alkenyl or C₃-C₄alkynyl;
R₅₆ is SO₂R₄₉;
R₅₇ is hydrogen or C₁-C₄alkyl;
R₅ and R₇₂ are each independently NR₆₀R₆₁ or indazole;
R₆, R₁₁, R₁₂, R₁₄, R₁₅, R₂₀, R₂₁, R₂₂, R₂₅, R₂₈, R₂₉, R₃₁, R₃₂, R₃₃, R₃₅, R₄₅, R₄₆ and R₈₀ are each independently hydrogen or methyl;
R₇ is C₁-C₄alkyl optionally substituted with cyano or C(O)R₆₂;
R₈ is hydrogen or C₁-C₄alkoxy;
R₉ and R₉₀ are each independently C₁-C₄alkyl;
R₁₀ is hydrogen or C₁-C₃alkyl;
R₁₃, R₂₄ and R₃₆ are each independently hydrogen or chlorine;
R₂₃ is NR₆₃R₆₄;
R₃₄ is C₁-C₃haloalkyl;
R₃₇ is C₂-C₄alkoxyalkyl;
R₃₈ and R₃₉ are each independently C₁-C₃haloalkyl, C₁-C₃alkyl or propargyl;
R₄₀, R₄₁ and R₄₂ are each independently hydrogen, C(O)R₆₆, C(S)R₆₇, CO₂R₆₈, C(=NOR₆₉),
   C₁-C₃alkyl optionally substituted with any combination of one or two halogen atoms, one or two C₁-C₃alkoxy groups, one or two C₁-C₃haloalkoxy groups, one SO₂R₇₂ group, one or two cyano groups, one C₃-C₅cycloalkyl group, one OSO₂R₇₃ group, one C(O)R₇₄ group, one CO₂R₇₅ group, one C(O)SR₇₆ group, one C(O)NR₇₇R₇₈ group, one to two OR₇₉ groups, one P(O) (OR₈₀)₂ group, one 1,3-dioxolane group or one 1,3-dioxane group, or
   phenyl optionally substituted with any combination of one halogen atom, one or two methyl groups, one methoxy group, one halomethyl group or one OR₈₃ group;
R₄₃, R₄₄, R₄₇ and R₄₈ are each independently hydrogen or methyl, or R₄₃ and R₄₄ or R₄₇ and R₄₈ may be taken together with the atom to which they are attached to form a cyclopropyl group;
R₄₉, R₅₀ and R₈₆ are each independently C₁-C₄alkyl or NR₉₃R₉₄;
R₅₁, R₅₂, R₅₃, R₅₄, R₅₅, R₅₈, R₆₀, R₆₁, R₆₂, R₇₃, R₇₄, R₇₈ and R₈₇ are each independently hydrogen, C₁-C₄alkyl or C₁-C₄haloalkyl;
R₇₉ and R₈₃ are each independently hydrogen, C(O)R₈₅, SO₂R₈₆, C₁-C₄haloalkyl, C₃-C₄alkenyl or C₁-C₃alkyl substituted with one OC(O)R₈₇, CO₂R₈₈, C(O)R₈₉, C(OR₉₀)₂ or cyano group;
R₉₃ and R₉₄ are each independently hydrogen or C₁-C₈alkyl; and
p is 0, 1 or 2.

The process of the present invention is especially useful for the preparation of 6-(trifluoromethyl)uracil compounds having the structural formula VI 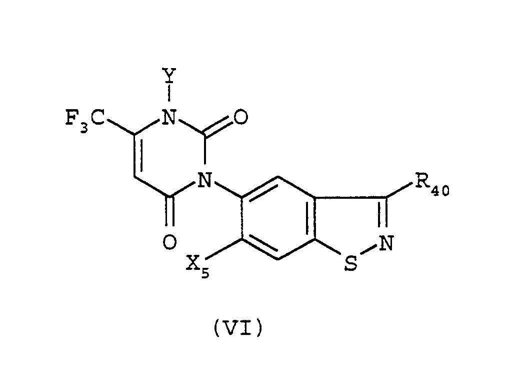 wherein
- Y: is hydrogen or methyl;
- X₅: is hydrogen or halogen;
- R₄₀: is hydrogen, C(O)R₆₆, C(S)R₆₇, CO₂R₆₈,
C₁-C₃alkyl optionally substituted with any combination of one or two halogen atoms, one or two C₁-C₃alkoxy groups, one or two C₁-C₃haloalkoxy groups, one SO₂R₇₂ group, one or two cyano groups, one C₃-C₅cycloalkyl group, one OSO₂R₇₃ group, one or two OR₇₉ groups, one P(O)(OR₈₀)₂ group, one 1,3-dioxolane group or one 1,3-dioxane group, or
phenyl optionally substituted with any combination of one halogen atom, one or two methyl groups, one methoxy group, one halomethyl group or one OR₈₃ group;
- R₆₆, R₆₇, R₈₅ and R₈₉: are each independently hydrogen, C₁-C₄alkyl or NR₅₆R₅₇;
- R₅₆: is SO₂R₄₉;
- R₅₇: is hydrogen or C₁-C₄alkyl;
- R₄₉ and R₈₆: are each independently C₁-C₄alkyl or NR₉₃R₉₄;
- R₉₃ and R₉₄: are each independently hydrogen or C₁-C₈alkyl;
- R₆₈ and R₈₈: are each independently hydrogen, C₃-C₆alkenyl or C₁-C₄alkyl optionally substituted with CO₂R₅₄, morpholine or C(O)R₅₅;
- R₅₄, R₅₅, R₆₀, R₆₁, R₇₃ and R₈₇: are each independently hydrogen, C₁-C₄alkyl or C₁-C₄haloalkyl;
- R₇₂: is NR₆₀R₆₁ or indazole;
- R₇₉ and R₈₃: are each independently hydrogen C(O)R₈₅, SO₂R₈₆, C₁-C₄haloalkyl, C₃-C₄alkenyl or C₁-C₃alkyl substituted with one OC(O)R₈₇, CO₂R₈₈, C(O)R₈₉, C(OR₉₀)₂ or cyano group;
- R₈₀: is hydrogen or methyl; and
- R₉₀: is C₁-C₄alkyl.

Exemplary of halogen hereinabove are fluorine, chlorine, bromine and iodine. The terms 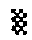 halomethyl 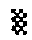 , 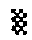 C₁-C₄haloalkyl 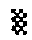 , 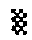 C₁-C₈haloalkyl 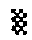 , 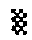 C₁-C₃haloalkoxy 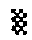 , 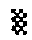 C₁-C₄haloalkoxy 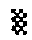 and 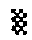 C₁-C₈haloalkoxymethyl 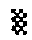 are defined as a methyl, C₁-C₄alkyl, C₁-C₈alkyl, C₁-C₃alkoxy, C₁-C₄alkoxy or C₁-C₈alkoxymethyl group substituted with one or more halogen atoms. In formula I above, alkali metals include sodium, potassium and lithium, and alkaline earth metals include calcium and magnesium. Organic ammonium cations suitable for use in the present invention include, but are not limited to, a group consisting of a positively charged nitrogen atom joined to from one to four aliphatic groups, each containing from one to sixteen carbon atoms.

In formula I above, 5- to 12-membered monocyclic or fused bicyclic, heterocyclic rings include, but are not limited to, benzimidazole, imidazole, imidazoline-2-thione, indole, isatoic anhydride, morpholine, piperazine, piperidine, purine, pyrazole, pyrrole, pyrrolidine and 1,2,4-triazole rings wherein each ring is optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy, or C₁-C₄haloalkylsulfonyl groups.

Starting formula II carbamate compounds may be prepared by reacting a β-amino-β-(perfluoroalkyl)acrylate compound having the structural formula VII 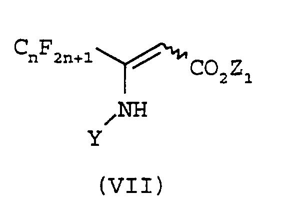 wherein n, Y and Z₁ are as described hereinabove with a base and a chloroformate compound having the structural formula VIII

ClCO₂Z (VIII)

wherein Z is as described hereinabove.

Formula II carbamate compounds may also be prepared according to the procedure described in Tetrahedron Letters, No.4, pages 243-246 (1976).

Formula VII β-amino-β-(perfluoroalkyl)acrylate compounds are known in the art and may be prepared according to the procedures described in U.S. 5,777,154; Journal of Heterocyclic Chemistry, *9*, pages 513-522 (1972); and Institute of Chemistry, Urals Scientific Center, Academy of Sciences of the USSR, Sverdlovsk, pages 1442-1447 (1987) - English translation of Zhurnal Organicheskoi Khimii, 22(8), pages 1603-1609 (1986).

Chloroformate compounds of formula VIII are known in the art and may be prepared by conventional procedures. In addition, certain formula VIII chloroformate compounds are commercially available.

Amine compounds having the structural formula IIIa 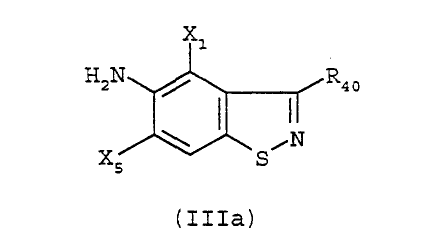 wherein X₁, X₅ and R₄₀ are as described hereinabove, may be prepared, as shown in Flow Diagram I, by cyclizing a ketone of formula IX with sulfur and ammonium hydroxide or ammonia to form a nitrobenzisothiazole of formula X, and reducing the formula X compound using conventional reducing agents such as iron in acetic acid.

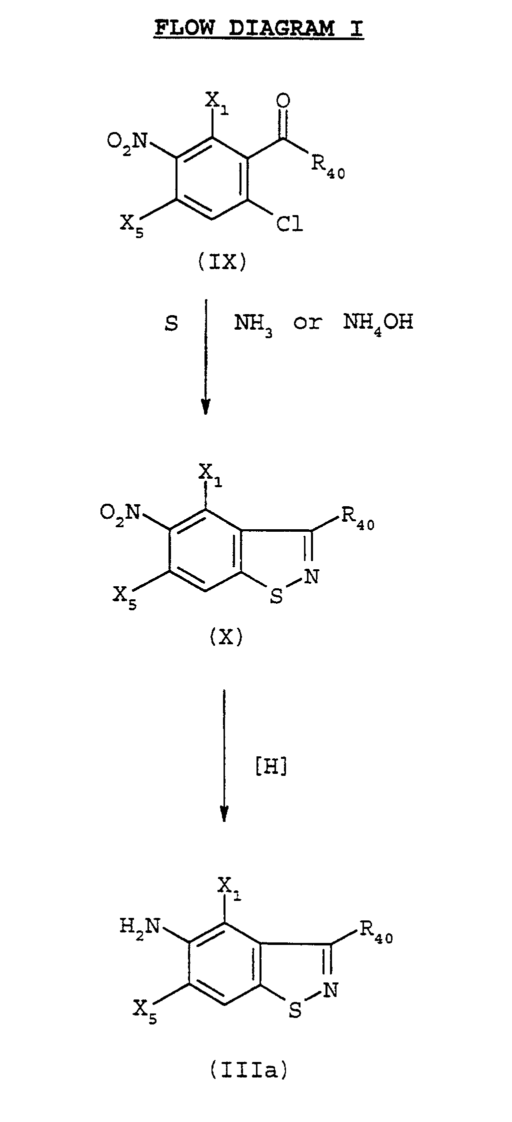

Starting amine compounds having the structural formula IIIb 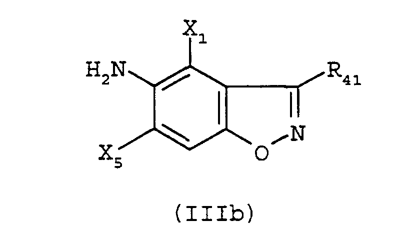 wherein X₁, X₅ and R₄₁ are as described hereinabove, may be prepared, as illustrated in Flow Diagram II, by reacting a ketone of formula XI with hydroxylamine hydrochloride optionally in the presence of sodium acetate to form an oxime of formula XII, cyclizing the formula XII compound with a base such as potassium hydroxide to form a nitrobenzisoxazole of formula XIII, and reducing the formula XIII compound using conventional reducing agents such as tin(II) chloride in acetic acid.

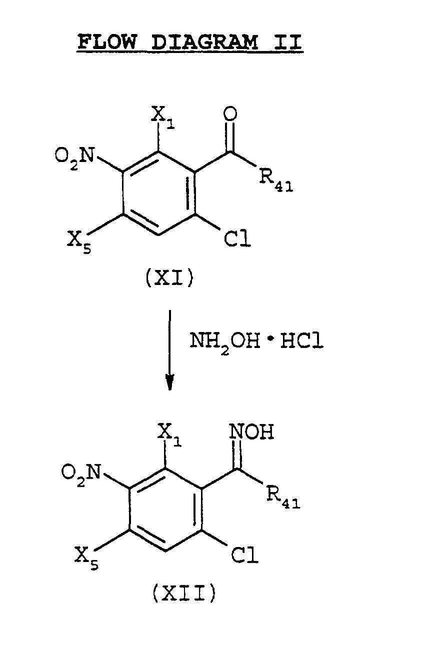

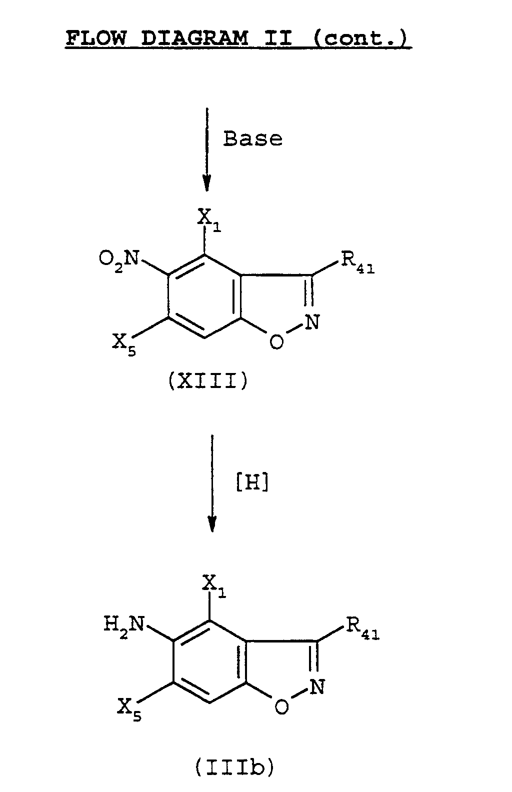

Alternatively, formula XIII nitrobenzisoxazole compounds may be prepared, as shown in Flow Diagram III, by reacting a ketone of formula XIV with hydroxylamine hydrochloride optionally in the presence of a base such as sodium acetate to form an oxime of formula XV, cyclizing the formula XV compound with 1,1'-carbonyldiimidazole in the presence of a base such as triethylamine to form a benzisoxazole of formula XVI, and nitrating the formula XVI compound using conventional methods such as a nitric acid/sulfuric acid mixture.

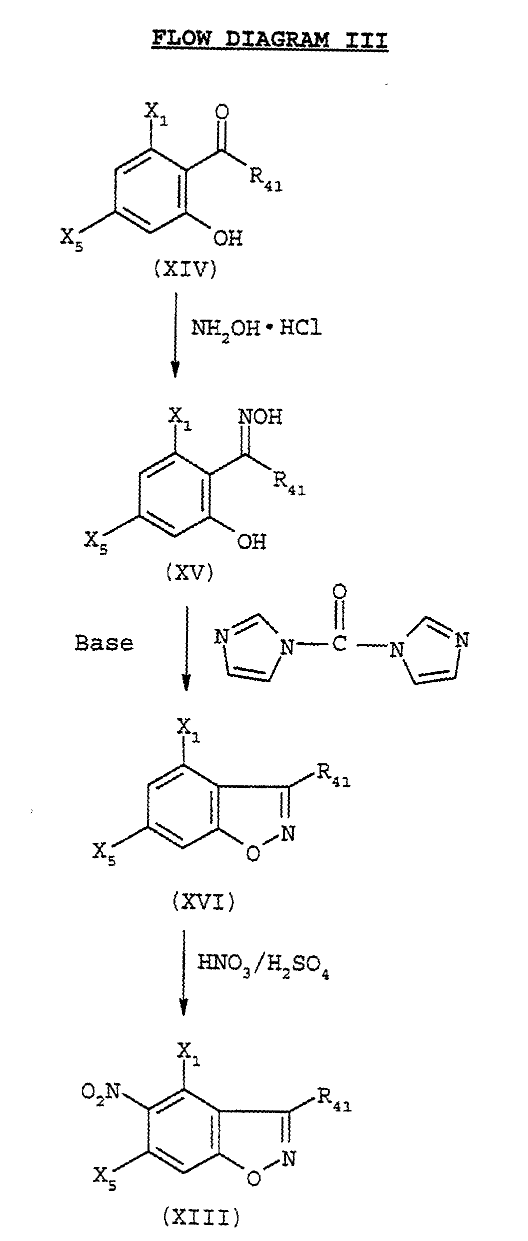

Intermediate compounds of formulas X and XIII wherein R₄₀ and R₄₁ are OR₆₅ may be prepared, as shown in Flow Diagram IV, by nitrating a benzisoxazol-3-ol or benzisothiazol-3-ol of formula XVII with a conventional nitrating agent such as a nitric acid/sulfuric acid mixture to form a 5-nitrobenzisoxazol-3-ol or 5-nitrobenzisothiazol-3-ol of formula XVIII, and reacting the formula XVIII compound with an electrophile of formula XIX in the presence of a base such as potassium carbonate.

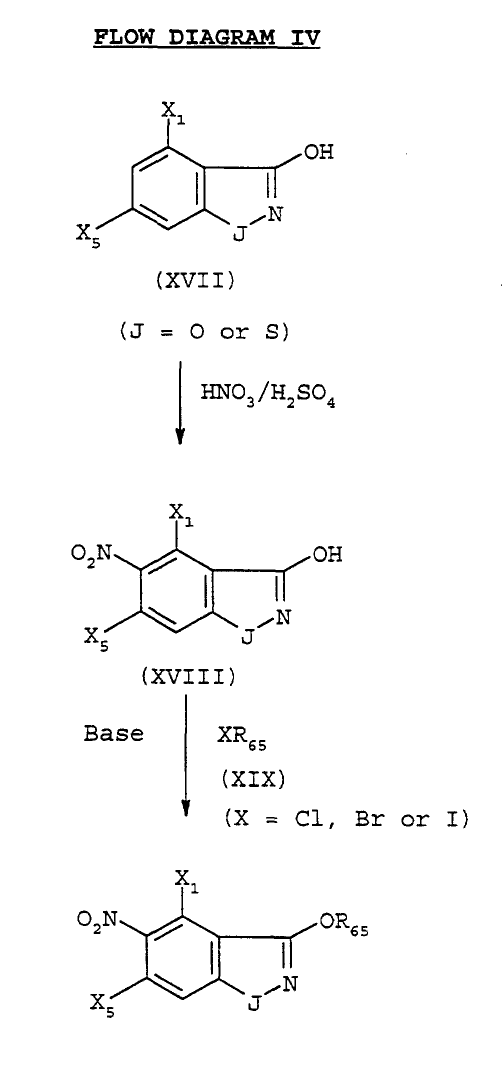

Formula X and XIII intermediate compounds wherein R₄₀ and R₄₁ are Cl or Br may be prepared, as shown in Flow Diagram V, by reacting a 5-nitrobenzisoxazol-3-ol or 5-nitrobenzisothiazol-3-ol of formula XVIII with phosphorous oxychloride, phosphorous oxybromide or phosphorous pentabromide.

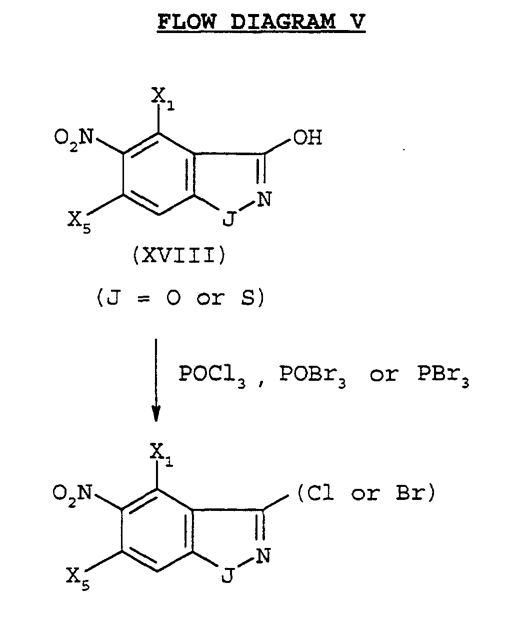

Other methods for the preparation of formula IIIa and IIIb amine compounds will become apparent from the examples set forth below. In addition, certain compounds of formulas IIIa, IIIb, X and XIII may be converted into other compounds of formulas IIIa, IIIb, X and XIII by using conventional procedures known to those skilled in the art.

Other formula III amine compounds are known in the art and may be prepared according to the procedures described in EP 561319-A; EP 540023-A; EP 545206-A; EP 542685-A; EP 473551-A; EP 476697-A; EP 489480-A; EP 496595-A; EP 420194-A; EP 648749-A; EP 705829-A; EP 714602-A; JP 9241245; JP 9301973; U.S. 5,169,430; U.S. 5,310,723; U.S. 5,324,854; U.S. 5,391,541; U.S. 5,399,543; U.S. 5,484,763; U.S. 5,523,278; U.S. 5,602,077; U.S. 5,661,108; WO 93/14073; WO 94/10155; WO 94/24128; WO 91/07393; WO 91/107392; WO 95/04461; WO 95/05079; WO 95/05080; WO 95/17096; WO 95/25725; WO 95/29168; WO 95/32952; WO 95/33746; WO 96/02518; WO 96/08151; WO 96/14315; WO 96/28442; WO 96/34859; WO 96/35679; WO 97/01541; WO 97/01542; WO 97/05118; WO 97/07105; WO 97/08170; WO 97/08171; WO 97/08953; WO 97/12884; WO 97/12886; WO 97/29094; WO 97/29105; WO 97/34484; WO 97/35845; WO 97/42176; WO 97/42188; WO 97/45418; WO 97/47607; WO 98/02422; WO 98/06706; WO 98/08824; WO 98/27057; WO 98/27067; WO 98/27082; and WO 98/27088, among others.

In order to facilitate a further understanding of this invention, the following examples are presented primarily for the purpose of illustrating more specific details thereof. The scope of the invention should not be deemed limited by the examples but encompasses the entire subject matter defined in the claims.

### REFERENCE EXAMPLE 1

### Preparation of Ethyl 3-[(ethoxycarbonyl)amino]-4,4,4-trifluorocrotonate, (Z)-

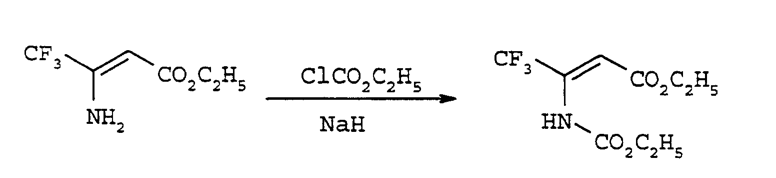

A solution of 3-amino-4,4,4-trifluorocrotonate (18.3 g, 100 mmol) in N,N-dimethylformamide (30 mL) is added dropwise to a stirred mixture of sodium hydride (60% in mineral oil, 9.6 g, 240 mmol) in N,N-dimethylformamide (20 mL) at 0°C under nitrogen. Upon cessation of the evolution of hydrogen gas, a solution of ethyl chloroformate (13.02 g, 120 mmol) in anhydrous ether (30 mL) is added at 0°C over 30 minutes. The resultant reaction mixture is then stirred at room temperature for one hour, and quenched with water and ethyl acetate. The resultant mixture is extracted with ethyl acetate. The organic extract is washed sequentially with water and aqueous 2 N hydrochloric acid, dried over anhydrous sodium sulfate, filtered, and concentrated *in vacuo* to obtain a residue. Fractional distillation of the residue under reduced pressure gives the title product as a liquid (18 g, 70.5% yield, bp 95-97°C (4.5 mm)) which is identified by ¹H and ¹⁹F NMR spectral analyses.

### REFERENCE EXAMPLE 2

### Preparation of Ethyl 3-[(N-ethoxycarbonyl)-N-methylamino)-4,4,4-trifluorocrotonate, (Z)-

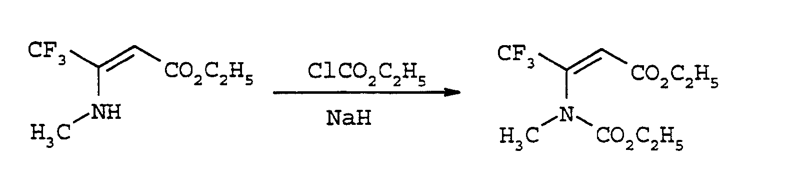

A solution of 3-methylamino-4,4,4-trifluorocrotonate (9.86 g, 50 mmol) in N,N-dimethylformamide (30 mL) is added dropwise to a stirred mixture of sodium hydride (60% in mineral oil, 4.5 g, 112 mmol) in N,N-dimethylformamide (30 mL) at -10°C under nitrogen. The resultant mixture is stirred at 0°C for 2 hours, treated with a solution of ethyl chloroformate (8.14 g, 75 mmol) in anhydrous ether (40 mL) at -10°C over 40 minutes, stirred at room temperature overnight, and quenched with water and ethyl acetate. The resultant mixture is extracted with ethyl acetate. The organic extract is washed sequentially with water and aqueous 2 N hydrochloric acid, dried over anhydrous sodium sulfate, filtered, and concentrated *in vacuo* to obtain a residue. Fractional distillation of the residue under reduced pressure gives the title product as a liquid (6.5 g, 48.3% yield, bp 60-62°C (0.2 mm)) which is identified by ¹H and ¹⁹F NMR spectral analyses.

### EXAMPLE 3

### Preparation of 3-Isopropyl-6-(trifluoromethyl)-2,4-(1H,3H)-pyrimidinedione

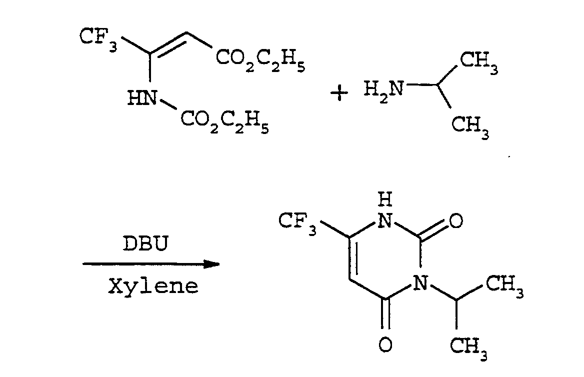

A stirred mixture of ethyl 3-[(ethoxycarbonyl)amino]-4,4,4-trifluorocrotonate, (Z)- (2.04 g, 8 mmol), isopropylamine (0.567 g, 9.6 mmol), and 1,8-diazabicyclo[5,4,0]undec-7-ene (DBU, 1.46 g, 9.6 mmol) in xylene (15 mL) is heated at 100°C for 3 hours, cooled, and diluted with ethyl acetate. Water is added, and the resultant aqueous mixture is extracted with ethyl acetate. The organic extracts are combined, washed sequentially with water and aqueous 2 N hydrochloric acid, dried over anhydrous sodium sulfate, filtered, and concentrated *in vacuo* to obtain a solid. The solid is crystallized from methanol/water to give the title product as a solid (1.08 g, 61% yield) which is identified by ¹H and ¹⁹F NMR spectral analyses.

### EXAMPLE 4

### Preparation of 3-[3-(6-Methoxy-m-tolyl)-1,2-benzisothiazol-5-yl)-6-(trifluoromethyl)-2,4(1H,3H)-pyrimidinedione

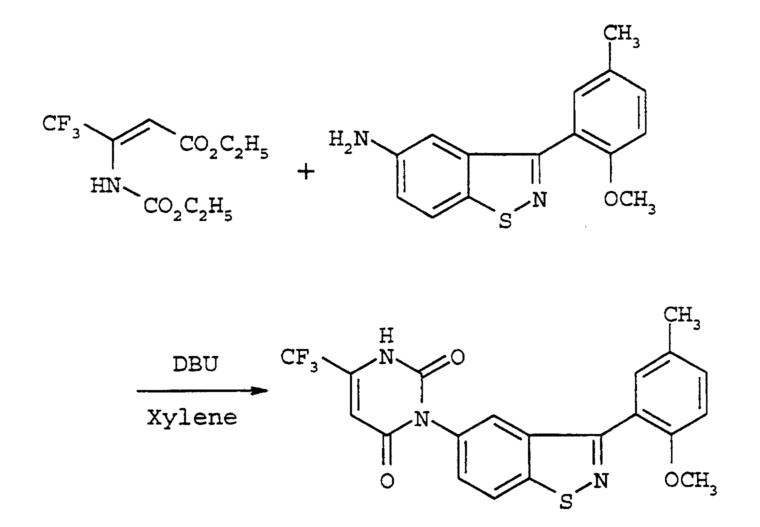

A mixture of ethyl 3-[(ethoxycarbonyl)amino]-4,4,4-trifluorocrotonate, (Z)- (2.04 g, 8 mmol), 5-amino-3-(6-methoxy-*m*-tolyl)-1,2-benzisothiazole (2.59 g, 9.6 mmol) and 1,8-diazabicyclo[5,4,0]undec-7-ene (DBU, 1.46 g, 9.6 mmol) in xylene (15 mL) is heated at 110°C for 6 hours, cooled, and quenched with ethyl acetate and water. The resultant mixture is extracted with ethyl acetate. The organic extracts are combined, washed with aqueous 2 N hydrochloric acid, dried over anhydrous sodium sulfate, filtered, and concentrated *in vacuo* to obtain a foam. Flash chromatography of the foam using silica gel and a 3:7 ethyl acetate/hexanes solution gives the title product as a solid (2.5 g, 72% yield) which is identified by ¹H and ¹⁹F NMR spectral analyses.

### REFERENCE EXAMPLE 5

### Preparation of 2'-Chloro-2-methoxy-5-methyl-5'-nitrobenzophenone

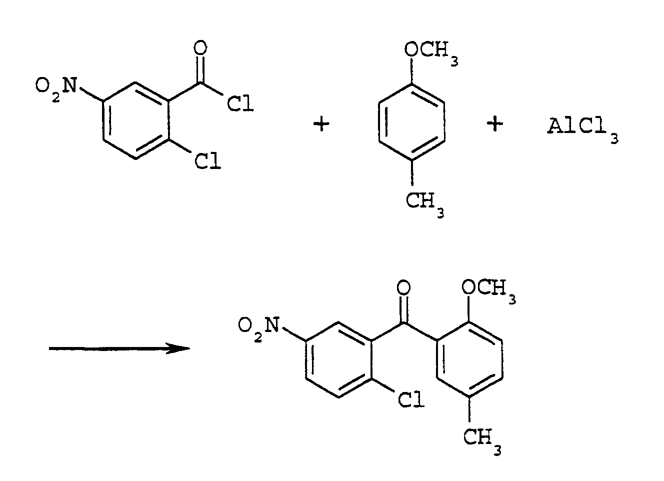

A mixture of aluminum chloride (33.3 g, 25.0 mmol) in methylene chloride is cooled to about 5 °C, treated over one hour with p-methylanisole (31.6 g, 25.0 mmol) while maintaining the reaction mixture temperature below 10 °C, treated over 20 minutes with a solution of 2-chloro-5-nitrobenzoyl chloride (50.0 g, 22.7 mmol) in methylene chloride while maintaining the reaction mixture temperature below 10 °C, warmed to and stirred at room temperature for 60 minutes, and poured onto ice. The resultant aqueous mixture is treated with concentrated hydrochloric acid (50 mL) and extracted with methylene chloride. The organic extract is dried over anhydrous magnesium sulfate and concentrated *in vacuo* to give a yellow solid. After placing the solid in a Kugelrohr apparatus at 40 °C to remove residual p-methylanisole, the title product is obtained as a beige solid (68.8 g, 99.1%) which is identified by NMR spectral analyses.

Using essentially the same procedure, the following compounds are obtained:

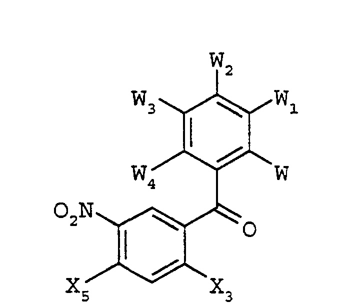

| **X**_{**3**} | **X**_{**5**} | **W** | **W**_{**1**} | **W**_{**2**} | **W**_{**3**} | **W**_{**4**} | **mp °C** |
|---|---|---|---|---|---|---|---|
| Cl | H | | | | | | |
| Cl | H | H | I | H | H | OCH₃ | 115-116.5 |
| Cl | H | H | H | CH₃ | H | OCH₃ | |
| Cl | H | H | H | C₂H₅ | H | H | |
| Cl | H | H | CH₃ | CH₃ | H | OCH₃ | |
| Cl | H | H | H | OCH₃ | H | H | 108-112 |
| Cl | H | H | C₂H₅ | H | H | OCH₃ | 98-99.5 |
| Cl | H | H | H | OCH₃ | H | CH₃ | 91-92 |
| Cl | H | H | H | CH₃ | H | H | 95.5-96.5 |
| Cl | H | H | H | SCH₃ | H | H | 127-128 |
| Cl | H | H | H | CH₃ | H | OCH₃ | 91-92.5 |
| Cl | H | H | H | C₂H₅ | H | H | |
| Cl | H | H | H | Cl | H | H | 88.5-90.5 |
| Cl | H | H | H | F | H | H | 68-69.5 |
| Cl | H | H | Cl | H | H | OCH₃ | 124-126 |
| Cl | H | H | OCH₃ | H | H | OCH₃ | 71-73 |
| Cl | H | H | H | OCH₃ | H | OCH₃ | 98-100 |
| Cl | H | H | CH₃ | CH₃ | H | OCH₃ | 127-129 |
| Cl | H | H | H | Cl | H | OCH₃ | 96-99 |
| Cl | H | CH₃ | H | CH₃ | H | OCH₃ | 108.5-110 |
| Cl | H | H | H | H | CH₃ | OCH₃ | 71-74 |
| Cl | H | H | H | N(CH₃)- SO₂CH₃ | H | H | |
| Cl | H | H | CH₃ | Cl | H | OCH₃ | 126-128 |
| Cl | H | H | CH₃ | H | CH₃ | OCH₃ | 110-112 |
| Cl | H | CH₃ | CH₃ | CH₃ | H | OCH₃ | 104-106 |
| Cl | H | H | CH(CH₃)₂ | H | H | OCH₃ | 69-71 |
| Cl | H | H | CH₃ | H | H | H | |
| Cl | H | H | H | H | H | CN | |
| Cl | H | H | H | H | H | OCH₃ | |
| Cl | H | H | OCH₃ | H | H | H | |
| Cl | H | H | F | H | H | OCH₃ | |
| Cl | H | H | H | F | H | OCH₃ | |
| Cl | H | H | H | H | H | SCH₃ | |
| Cl | H | H | H | H | H | CH₃ | |
| Cl | H | H | H | H | H | F | |
| Cl | H | H | SCH₃ | H | H | H | |
| Cl | H | H | H | OCH₃ | H | H | |
| Cl | H | H | -(CH₂)₃- | | H | OCH₃ | |
| Cl | F | H | H | H | H | H | |
| F | F | H | CH₃ | H | H | OCH₃ | |

### REFERENCE EXAMPLE 6

### Preparation of 3-(6-Methoxy-m-tolyl)-5-nitro-1,2-benzisothiazole

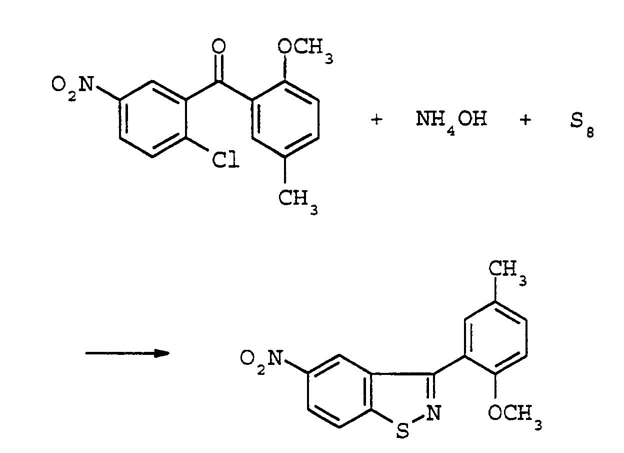

Ammonium hydroxide (350 mL of a 30% solution, 270 mmol) is added to a mixture of 2'-chloro-2-methoxy-5-methyl-5'-nitrobenzophenone (68.7 g, 22.5 mmol) and sulfur (7.57 g, 23.6 mmol) in N,N-dimethylformamide. The resultant reaction mixture is stirred at 80 °C for 19.5 hours, cooled to 40 °C, treated with additional ammonium hydroxide (50 mL of a 30% solution), stirred at 80 °C for 25 hours, cooled, and poured onto ice. The resultant aqueous mixture is filtered to obtain the title product as a yellow solid (63.5 g, 93.9%) which is identified by NMR spectral analyses.

Using essentially the same procedure, the following compounds are obtained:

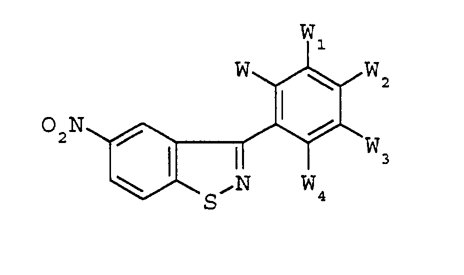

| **W** | **W**_{**1**} | **W**_{**2**} | **W**_{**3**} | **W**_{**4**} | **mp °C** |
|---|---|---|---|---|---|
| H | H | CH₃ | H | OCH₃ | 201-203 |
| H | CH₃ | CH₃ | H | OCH₃ | 199-200 |
| H | CH₃ | H | H | H | 116.5-117.5 |
| H | H | Cl | H | OCH₃ | 229-231 |
| H | H | H | CH₃ | OCH₃ | 134-136 |
| H | H | H | H | CN | 187.5-189 |
| H | H | H | H | OCH₃ | 193-198 |
| H | H | OCH₃ | H | H | 201-203 |
| H | OCH₃ | H | H | H | 174-175 |
| H | F | H | H | OCH₃ | 224-226 |
| H | C₂H₅ | H | H | OCH₃ | 153-154.5 |
| H | H | CH₃ | H | H | 188-189 |
| H | H | N (CH₃) SO₂CH₃ | H | H | |
| H | CH₃ | Cl | H | OCH₃ | 230-234 |
| H | I | H | H | OCH₃ | |
| H | H | SCH₃ | H | H | 177.5-178.5 |
| H | H | OCH₃ | H | CH₃ | 131-135 |
| H | H | F | H | H | 226-228 |
| H | H | Cl | H | H | 217.5-219 |
| H | H | F | H | OCH₃ | 224-225 |
| H | H | H | H | SCH₃ | 114.5-115.5 |
| H | H | CH₃ | H | OCH₃ | 201-203 |
| H | OCH₃ | H | H | OCH₃ | 195-196 |
| H | H | H | H | CH₃ | 145-146 |
| H | H | H | H | F | 181-182 |
| H | H | OCH₃ | H | OCH₃ | 171-172.5 |
| H | SCH₃ | H | H | H | 139-140.5 |
| H | CH₃ | H | CH₃ | OCH₃ | |
| CH₃ | CH₃ | CH₃ | H | OCH₃ | |
| H | CH(CH₃)₂ | H | H | OCH₃ | |
| H | H | CH₃ | CH₃ | OCH₃ | |
| H | -(CH₂)₃- | | H | OCH₃ | |

### REFERENCE EXAMPLE 7

### Preparation of 3-Methyl-5-nitro-1,2-benzisothiazole

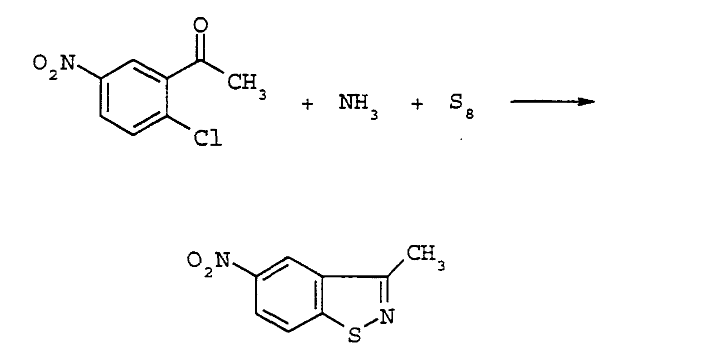

Ammonia (45 g, 2,642 mmol) is bubbled into methanol at -40 °C in a steel bomb. Sulfur (30.5 g, 95.0 mmol) and 2'-chloro-5'-nitroacetophenone (19 g, 95.0 mmol) are then added. The bomb is sealed and heated at about 90 °C overnight. After cooling, the reaction mixture is removed from the bomb and concentrated *in vacuo* to obtain a residue. The residue is diluted with methylene chloride, passed through a plug of silica gel and concentrated *in vacuo* to give the title product as an orange solid (12.0 g) which is identified by NMR spectral analyses.

Using essentially the same procedure, the following compounds are obtained:

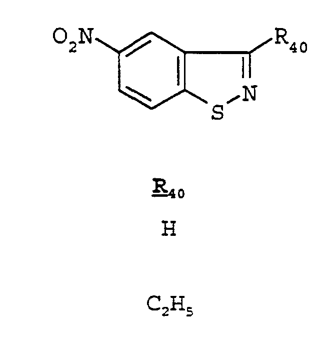

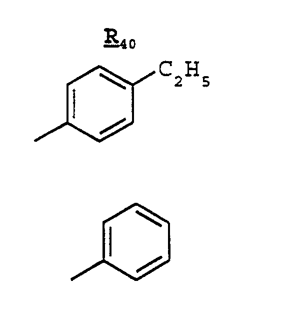

### REFERENCE EXAMPLE 8

### Preparation of 5-Amino-3-(6-methoxy-m-tolyl)-1,2-benzisothiazole

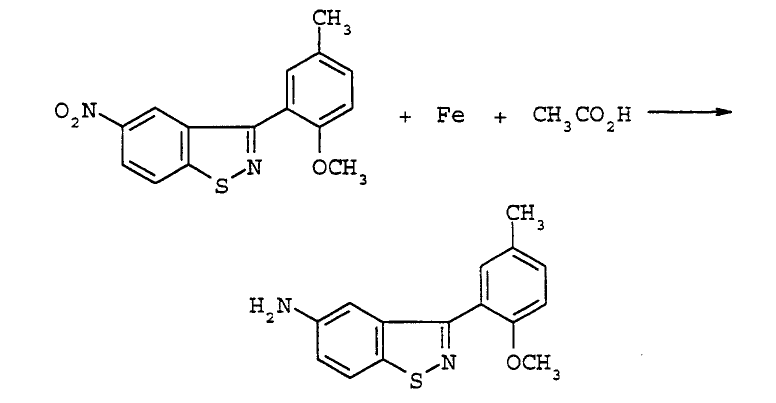

A mixture of 3-(6-methoxy-m-tolyl)-5-nitro-1,2-benzisothiazole (63.0 g, 0.210 mol), 5% acetic acid (1.52 L, 1.21 mol) and ethyl acetate (975 mL) is heated to 65 °C, treated portionwise with iron powder (58.6 g, 1.05 mol), stirred at 65 °C, and filtered through quartz filter paper. The filtrate phases are separated and the aqueous phase is extracted with ethyl acetate. The organic phase and extracts are combined, washed sequentially with water and brine, dried over anhydrous magnesium sulfate, and concentrated *in vacuo* to obtain the title product as an orange oil (55.7 g, 98.1%) which is identified by NMR spectral analyses.

Using essentially the same procedure, the following compounds are obtained:

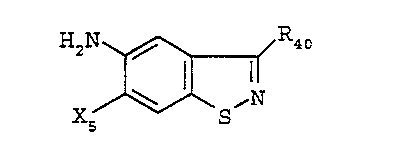

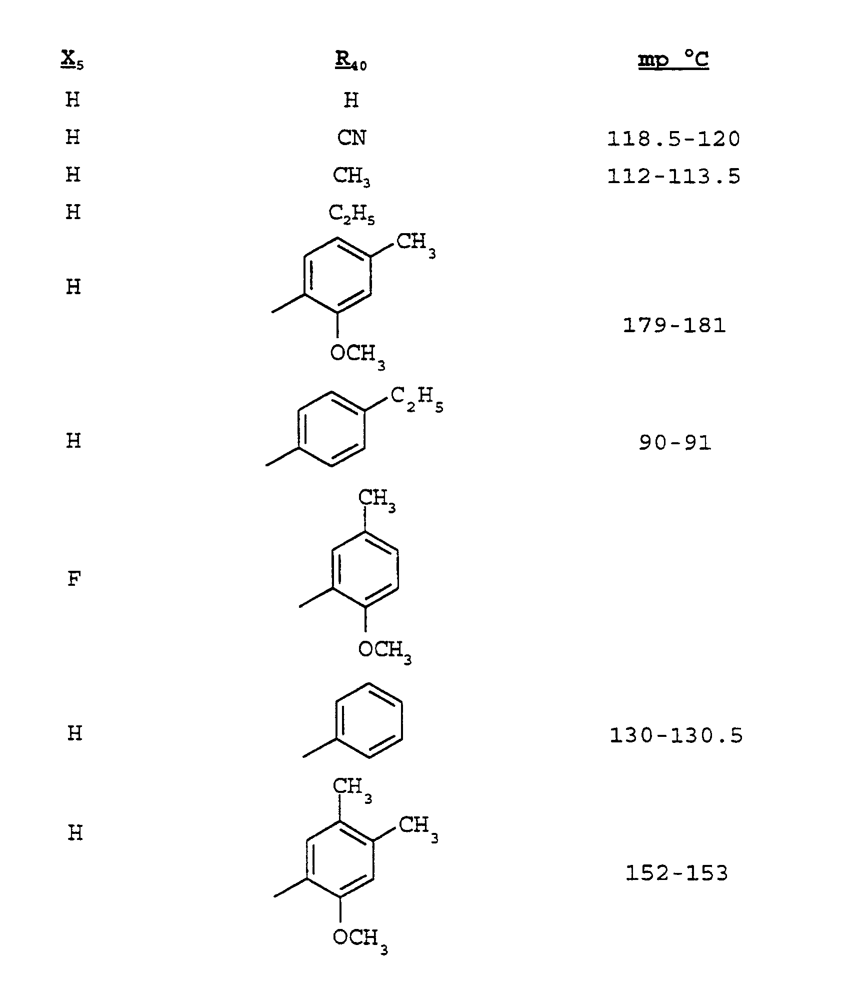

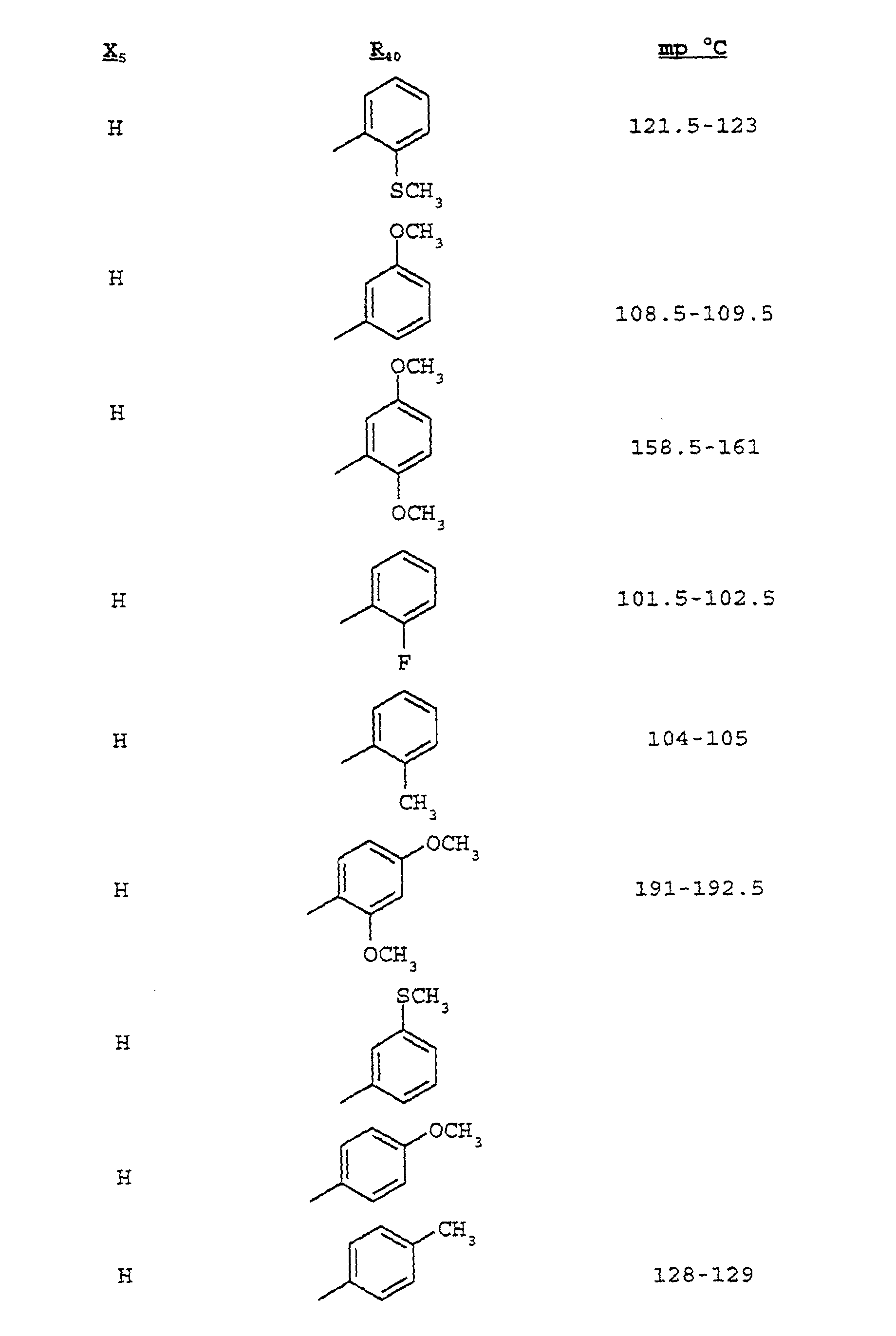

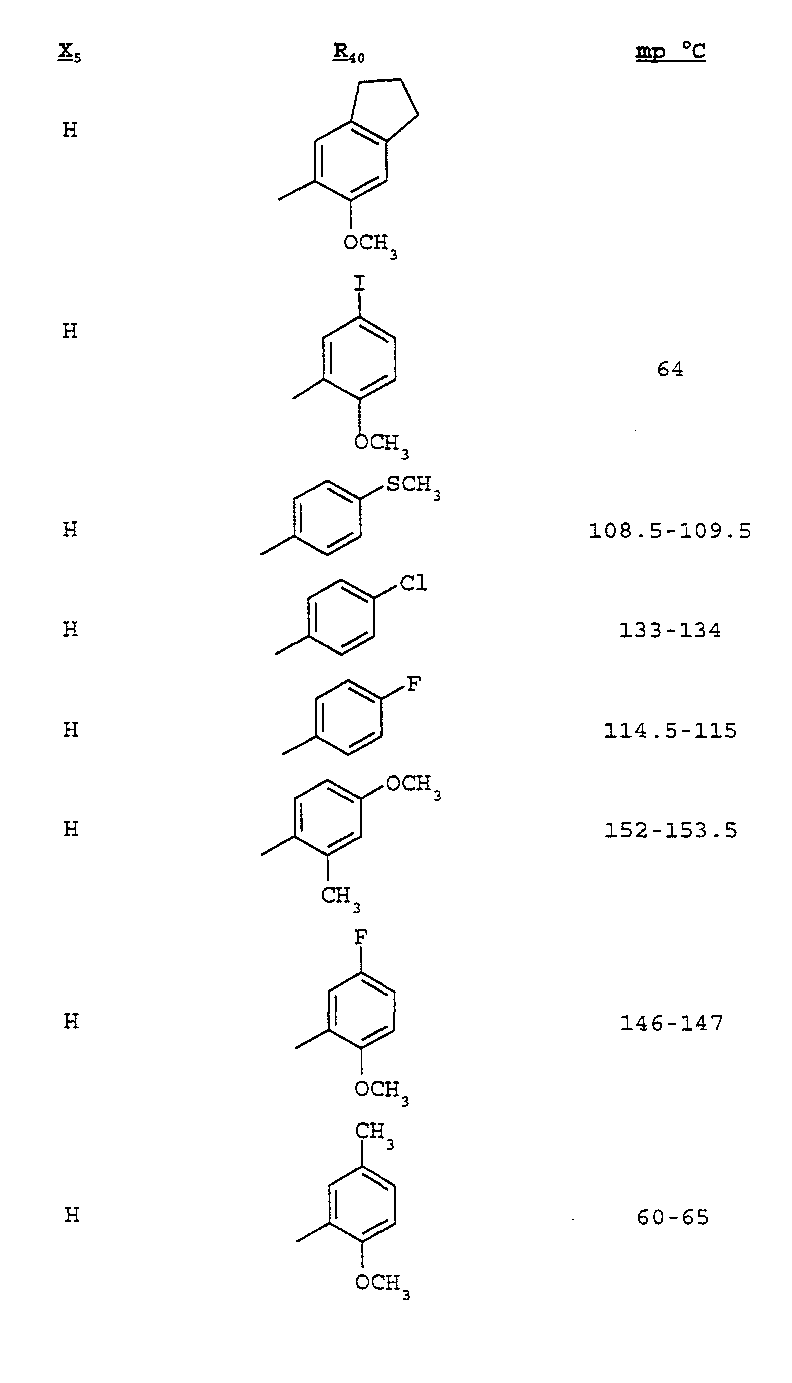

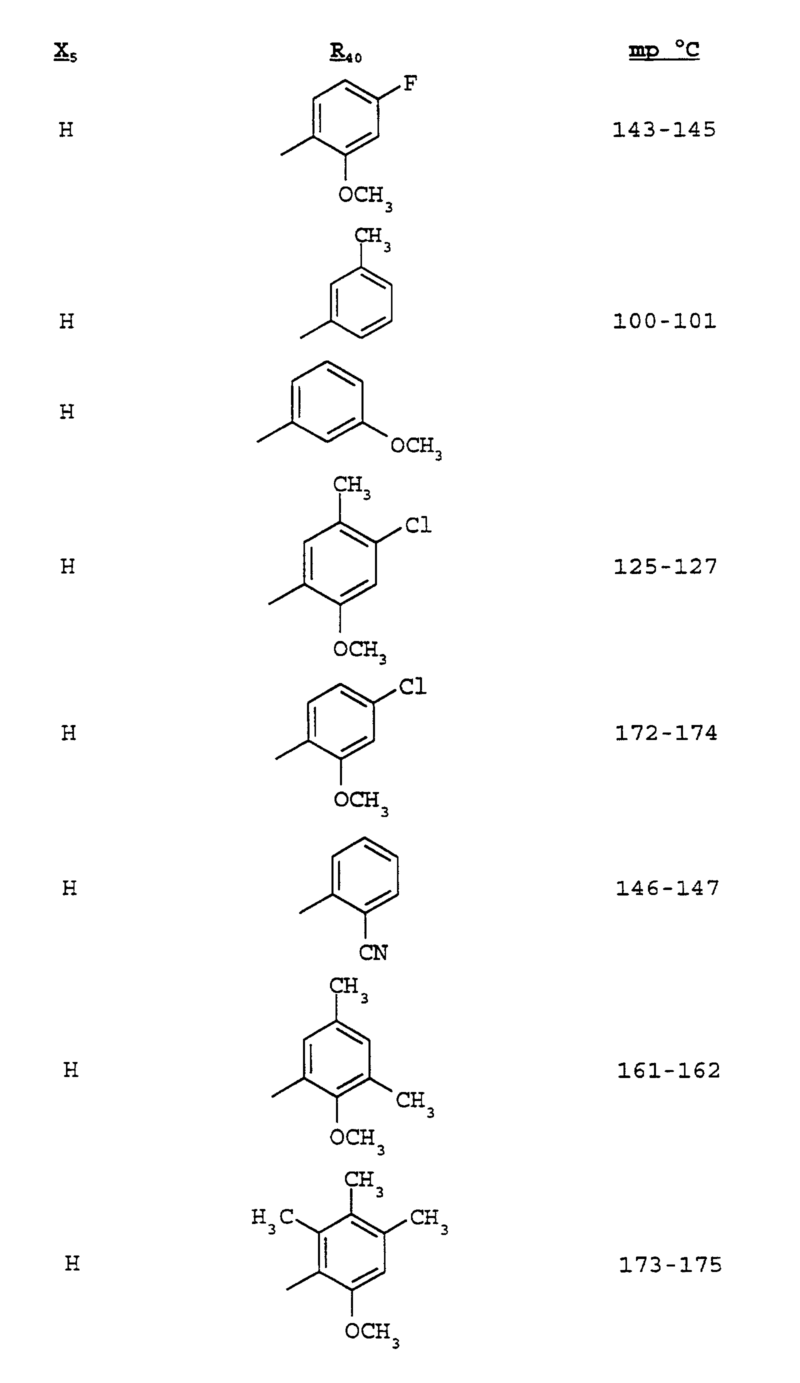

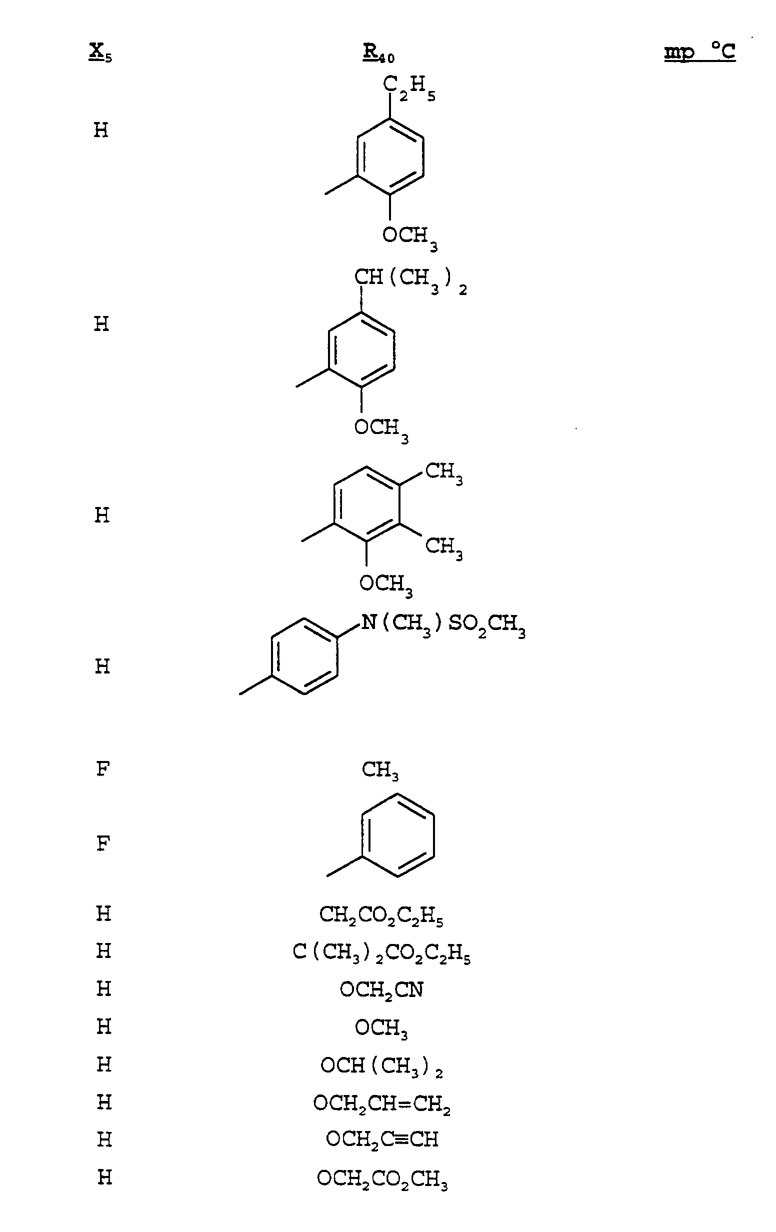

### REFERENCE EXAMPLE 9

### Preparation of 2-Chloro-2'-methoxy-5'-methyl-5-nitrobenzophenone, oxime

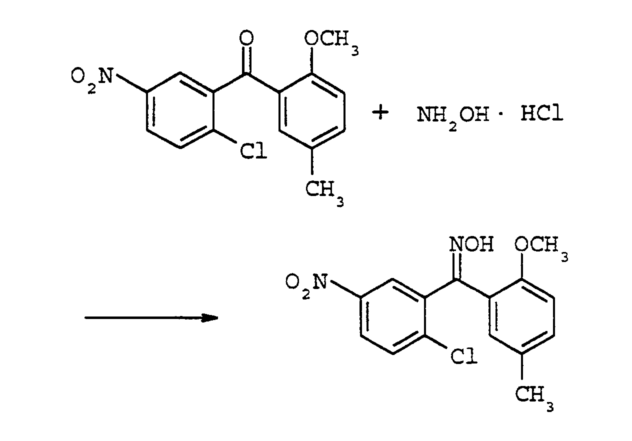

A mixture of 2-chloro-2'-methoxy-5'-methyl-5-nitrobenzophenone (90.0 g, 0.294 mol) in ethanol is treated with a solution of hydroxylamine hydrochloride (102.3 g, 1.47 mol) in water, refluxed overnight, and poured onto ice. The resultant aqueous mixture is filtered to obtain a solid. The solid is washed with water and dried in a hot vacuum oven overnight to give the title product as a white solid (84.2 g) which is identified by ¹H NMR spectral analysis.

Using essentially the same procedure, the following compounds are obtained:

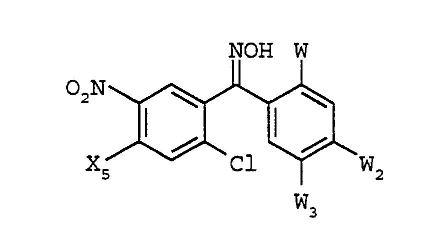

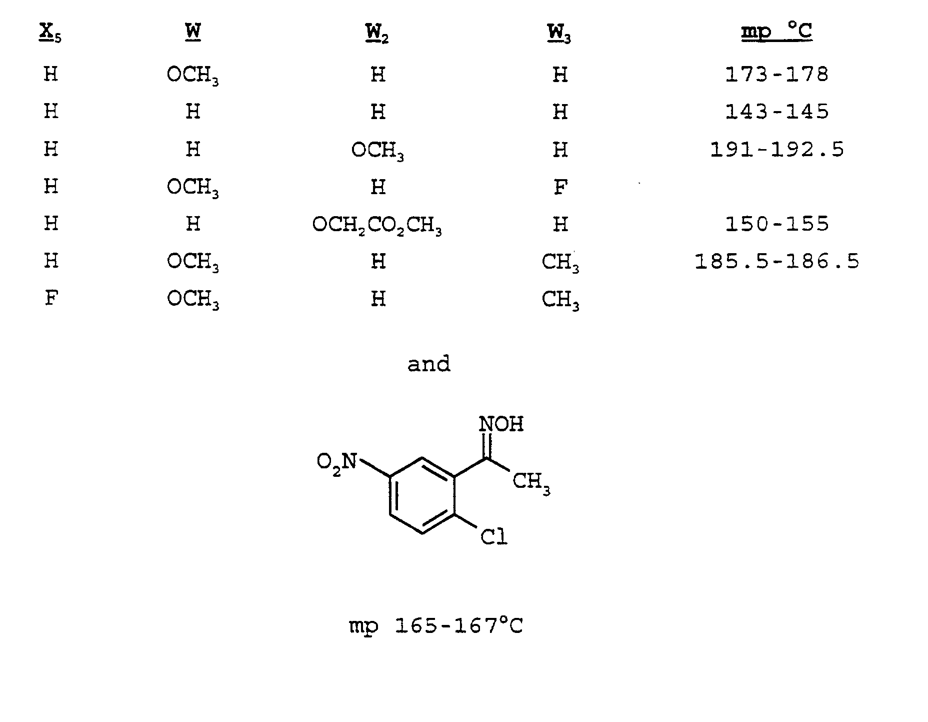

### REFERENCE EXAMPLE 10

### Preparation of 3-(6-Methoxy-m-tolyl)-5-nitro-1,2-benzisoxazole

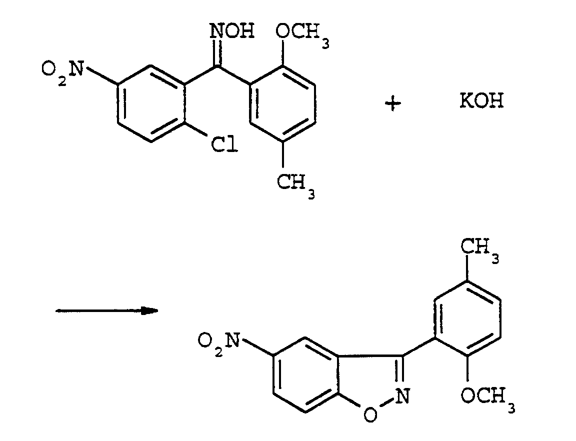

A mixture of 2-chloro-2'-methoxy-5'-methyl-5-nitro-benzophenone, oxime (84.0 g, 0.262 mol) in ethanol is warmed to 65 °C, treated with 150 mL of 10% potassium hydroxide solution over 25 minutes, heated to 78 °C over one hour, cooled, and poured onto ice. The resultant aqueous mixture is filtered to obtain a solid. The solid is washed with water, dried, recrystallized from N,N-dimethylformamide, washed sequentially with N,N-dimethylformamide and ethanol, and dried in a vacuum oven at 80 °C to give the title product as a solid (mp 225-226 °C) which is identified by ¹H NMR spectral analysis.

Using essentially the same procedure, the following compounds are obtained:

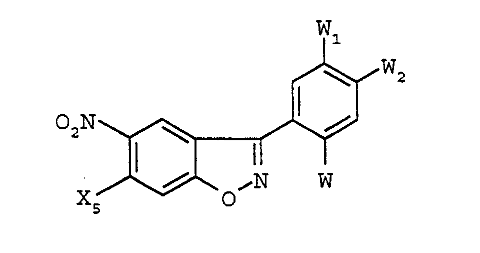

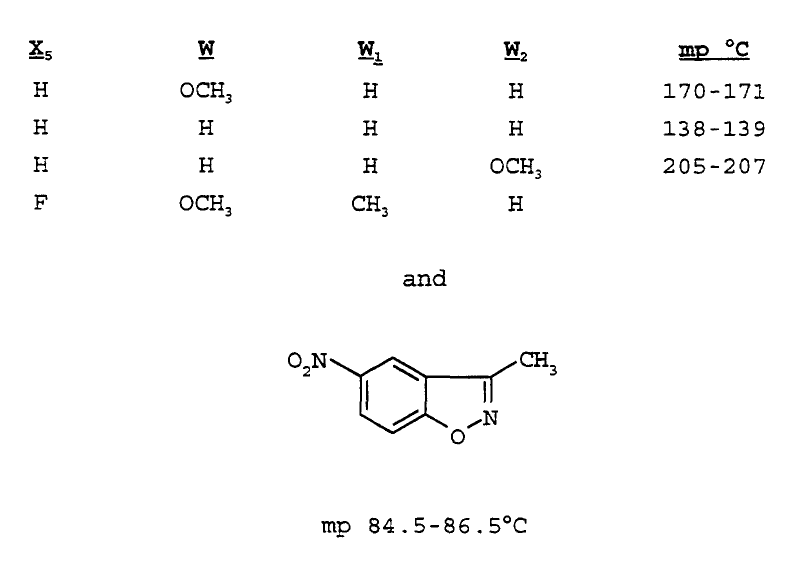

### REFERENCE EXAMPLE 11

### Preparation of 5-Amino-3-(6-methoxy-m-tolyl)-1,2-benzisoxazole and 5-Amino-4-chloro-3-(6-methoxy-m-tolyl)-1,2-benzisoxazole

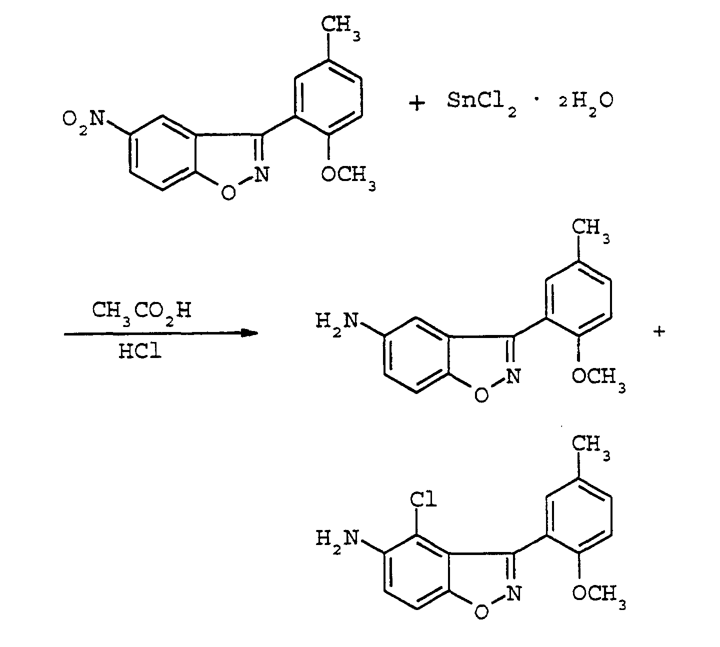

A mixture of 3-(6-methoxy-m-tolyl)-5-nitro-1,2-benzisoxazole (20.0 g, 0.0703 mol) in acetic acid (380 mL) is warmed, treated with a warm solution of tin(II) chloride dihydrate (47.4 g, 0.210 mol) in concentrated hydrochloric acid (110 mL), refluxed for one hour, cooled to 10 °C, and concentrated *in vacuo* to obtain a gum. The gum is added to water with stirring to obtain a slurry. The slurry is treated with 80 g of 50% sodium hydroxide solution, stirred at 60 °C to 80 °C over one hour, cooled, and decanted to obtain a residue. A mixture of the residue in ethanol is treated with potassium hydroxide (10 g), heated overnight, cooled to room temperature, neutralized with hydrochloric acid, and concentrated *in vacuo* to obtain a residue. The residue is diluted with ethyl acetate and filtered. The filtrate is concentrated *in vacuo* and chromatographed using silica gel and a 2% ethyl acetate in methylene chloride solution to give the title products as semi-solids which are identified by elemental and mass spectral analyses.

Using essentially the same procedure, the following compounds are obtained:

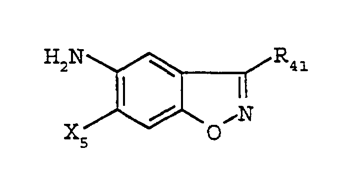

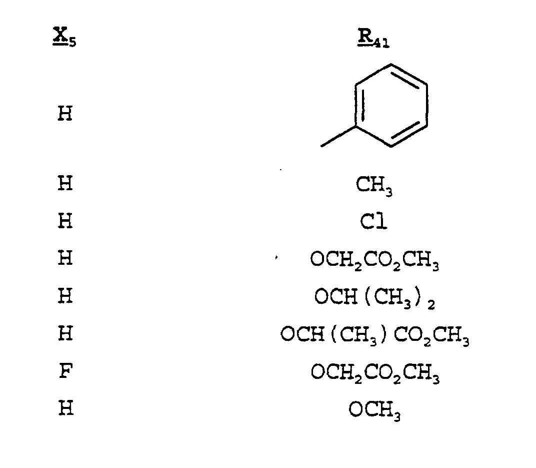

### REFERENCE EXAMPLE 12

### Preparation of m-Fluorophenyl acetate

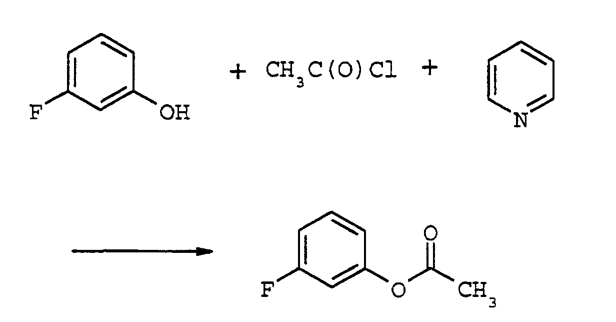

A solution of 3-fluorophenol (100 g, 0.890 mol) in methylene chloride is cooled to 0 °C to 5 °C, treated with pyridine (75.0 mL, 0.930 mol), stirred for several minutes, treated dropwise with acetyl chloride (66.0 mL, 0.930 mol) while maintaining the reaction mixture temperature below 17 °C, stirred at ice-bath temperature for two hours, warmed to room temperature, and poured into an ice-water mixture. The organic phase is separated, washed with brine, dried over anhydrous magnesium sulfate, and concentrated *in vacuo* to obtain the title product as a yellow oil which is identified by ¹H NMR spectral analysis.

### REFERENCE EXAMPLE 13

### Preparation of 4'-Fluoro-2'-hydroxyacetophenone

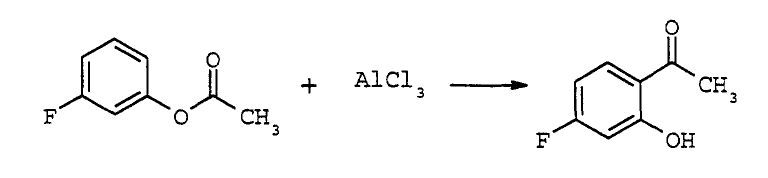

*m*-Fluorophenyl acetate (123 g, 0.798 mol) is cooled with an ice-bath, treated portionwise with aluminum chloride (150 g, 1.12 mol), stirred at 190 °C for one hour, and cooled to obtain a solid. A mixture of ice, water and hydrochloric acid, and methylene chloride are added to the solid. The resultant mixture is stirred for several minutes, and the phases are separated. The organic phase is washed sequentially with water, saturated sodium hydrogen carbonate solution and brine, dried over anhydrous magnesium sulfate, and concentrated *in vacuo* to obtain the title product (99.0 g) which is identified by ¹H NMR spectral analysis.

### REFERENCE EXAMPLE 14

### Preparation of 4'-Fluoro-2'-hydroxyacetophenone, oxime

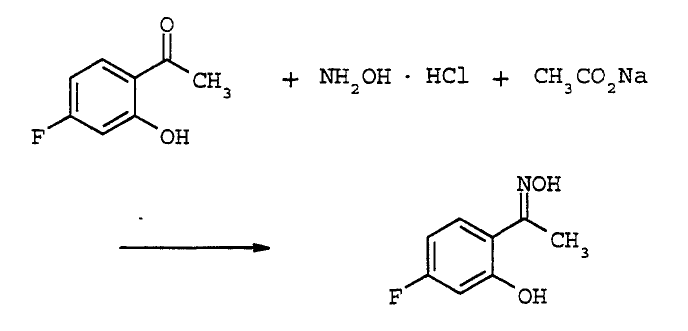

A mixture of 4'-fluoro-2'-hydroxyacetophenone (99.0 g, 0.640 mol), hydroxylamine hydrochloride (89.0 g, 1.28 mol), and sodium acetate (79.0 g, 0.960 mol) in methanol is refluxed for one hour and poured into an ice-water mixture. The resultant aqueous mixture is filtered to obtain a solid. The solid is dissolved in methylene chloride, and the resultant organic solution is dried over anhydrous magnesium sulfate, concentrated *in vacuo*, diluted with hexanes, and filtered to give the title product as a solid (55.0 g, mp 112-114 °C) which is identified by ¹H NMR spectral analysis.

### REFERENCE EXAMPLE 15

### Preparation of 6-Fluoro-3-methyl-1,2-benzisoxazole

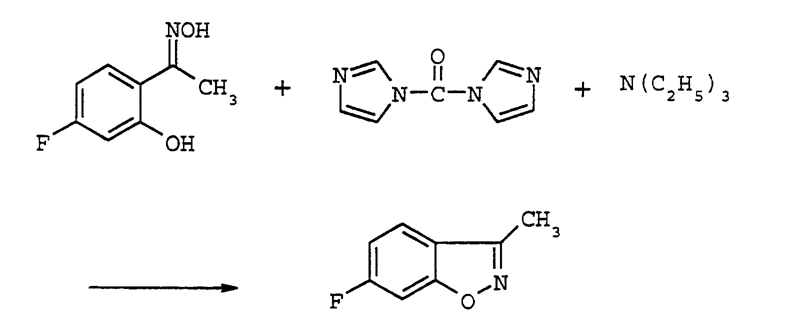

A mixture of 4'-fluoro-2'-hydroxyacetophenone, oxime (47.0 g, 0.278 mol) in tetrahydrofuran is heated to just under reflux, treated with a solution of 1,1'-carbonyldiimidazole (55.0 g, 0.340 mol) and triethylamine (39.0 g, 0.390 mol) in tetrahydrofuran, refluxed for one hour, cooled, concentrated *in vacuo*, and poured into an ice-water mixture. The resultant aqueous mixture is extracted with ether. The organic extracts are combined, washed sequentially with saturated ammonium chloride solution and brine, dried over anhydrous magnesium sulfate, and concentrated *in vacuo* to obtain an oil. Column chromatography of the oil using silica gel and a methylene chloride/hexanes solution (1:1) gives the title product as a yellow oil which is identified by ¹H NMR spectral analysis.

### REFERENCE EXAMPLE 16

### Preparation of 6-Fluoro-3-methyl-5-nitro-1,2-benzisoxazole

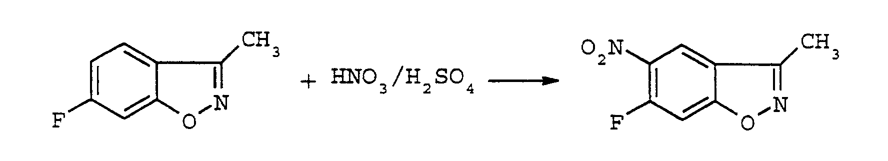

A mixture of 6-fluoro-3-methyl-1,2-benzisoxazole (23.5 g, 0.156 mol) in concentrated sulfuric acid is cooled with an ice-bath, treated dropwise with 90% nitric acid (8.50 mL) while maintaining the reaction mixture temperature below 15 °C, stirred for one hour at ice-bath temperature, treated with additional 90% nitric acid (5.80 mL), warmed to and stirred at room temperature overnight, and poured onto ice. The resultant aqueous mixture is filtered to obtain a solid. The solid is air-dried and dissolved in methylene chloride. The resultant organic solution is dried over anhydrous magnesium sulfate, diluted with hexanes, and filtered to give the title product as a purple solid which is identified by ¹H NMR spectral analysis.

Using essentially the same procedure, the following compounds are obtained:

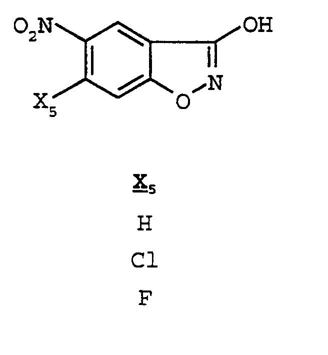

### REFERENCE EXAMPLE 17

### Preparation of Methyl [(5-nitro-1,2-benzisoxazol-3-yl)oxy]acetate

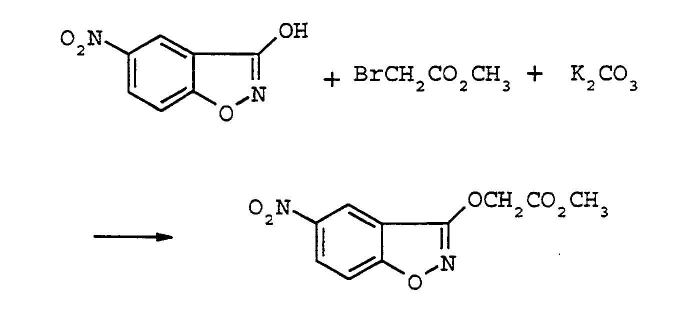

A mixture of 5-nitro-1,2-benzisoxazol-3-ol (3.90 g, 0.0220 mol) and potassium carbonate (4.17 g, 0.0300 mol) in N,N-dimethylformamide is stirred for 30 minutes, treated with methyl bromoacetate (3.96 g, 0.0260 mol), stirred overnight at room temperature, and poured into an acidic ice-water mixture. The resultant aqueous mixture is extracted with ethyl acetate. The organic extracts are combined, washed sequentially with water and brine, dried over anhydrous magnesium sulfate, and concentrated *in vacuo* to obtain a yellow oil. Column chromatography of the oil using silica gel and a (1:1) to (4:1) methylene chloride/hexanes gradient gives the title product as a white solid (2.80 g, mp 72-73.5 °C) which is identified by NMR spectral analyses.

Using essentially the same procedure, the following compounds are obtained:

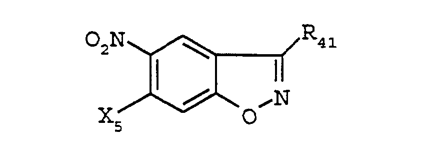

| **X**_{**5**} | **R**_{**41**} | **mp °C** |
|---|---|---|
| H | OCH (CH₃)₂ | 81-83 |
| H | OCH₂CH=CH₂ | 70-72 |
| H | OCH₃ | 101.5-103 |
| Cl | OCH(CH₃)CO₂CH₃ | 98-100 |
| F | OCH₂CO₂CH₃ | 104-106 |

### REFERENCE EXAMPLE 18

### Preparation of 3-Chloro-5-nitro-1,2-benzisoxazole

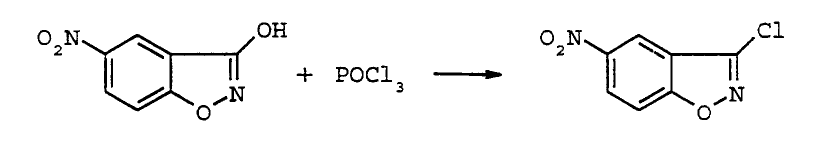

A mixture of 5-nitro-1,2-benzisoxazol-3-ol (4.00 g, 0.0220 mol) and phosphorus oxychloride (40.0 mL, 65.8 g, 0.429 mol) is placed in a glass bomb, heated at 150-155 °C for two hours, cooled overnight, concentrated *in vacuo*, diluted with methylene chloride, and brought to about pH 8 with sodium hydrogen carbonate solution. The phases are separated. The organic phase is washed sequentially with water and brine, dried over anhydrous magnesium sulfate, and concentrated *in vacuo* to obtain a residue. Column chromatography of the residue using silica gel and a methylene chloride/hexanes solution (1:1) gives the title product as an amber oil which is identified by NMR spectral analysis.

### REFERENCE EXAMPLE 19

### Preparation of 2-Chloro-2'-methoxy-5-nitrobenzophenone

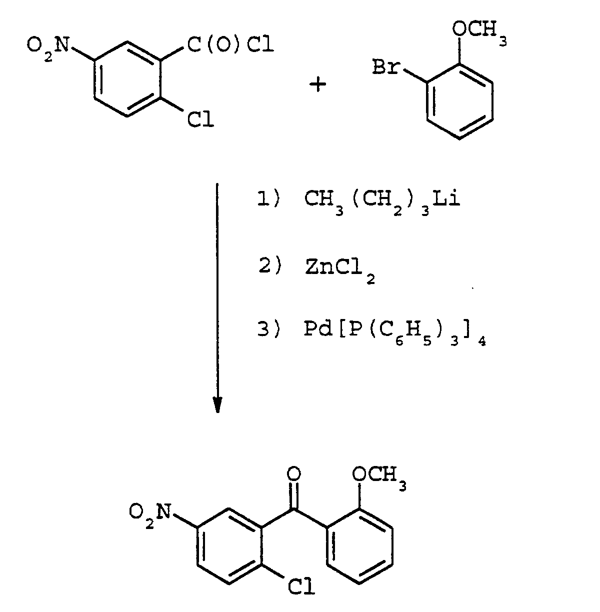

A solution of 2-bromoanisole (27.9 g, 145 mmol) in diethyl ether is cooled to -70 °C, treated with butyllithium (64.0 mL, 160 mmol), stirred at -70 °C for one hour, treated with 0.5 M zinc chloride in tetrahydrofuran solution (320 mL, 160 mmol), stirred for one hour at -70 °C, warmed to about 0 °C, and concentrated *in vacuo* to obtain a yellow-green oil. A solution of the oil in tetrahydrofuran is treated sequentially with tetrakis(triphenylphosphine)palladium(0) (5.00 g, 4.35 mmol) and a solution of 2-chloro-5-nitrobenzoyl chloride (35.0 g, 159 mmol) in tetrahydrofuran, stirred for three days, and poured into 10% hydrochloric acid. The resultant aqueous mixture is extracted with methylene chloride. The organic extracts are combined, washed sequentially with water and brine, dried over anhydrous magnesium sulfate, and concentrated *in vacuo* to obtain a semi-solid. The solid is triturated with diethyl ether to give the title product as a yellow solid which is identified by NMR spectral analyses.

Using essentially the same procedure, the following compounds are obtained:

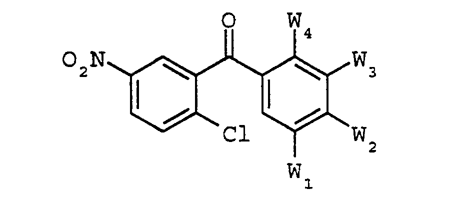

| **W**_{**1**} | **W**_{**2**} | **W**_{**3**} | **W**_{**4**} | **mp °C** |
|---|---|---|---|---|
| H | Cl | H | OCH₃ | 96-99 |
| H | H | CH₃ | OCH₃ | 71-74 |
| F | H | H | OCH₃ | |
| Cl | H | H | OCH₃ | 124-126 |
| OCH₃ | H | H | OCH₃ | 71-73 |
| H | OCH₃ | H | OCH₃ | 98-100 |
| H | F | H | OCH₃ | |
| H | H | CH₃ | H | 65-66.5 |
| H | H | SCH₃ | H | 87-88 |
| H | H | H | F | 118-120 |
| H | H | H | CH₃ | 78-79.5 |
| H | H | H | SCH₃ | 123-124.5 |
| H | F | H | H | |
| H | H | OCH₃ | H | |
| H | H | H | OCH₃ | |
| H | CH₃ | CH₃ | OCH₃ | |

### REFERENCE EXAMPLE 20

### Preparation of 2-Chloro-2'-methoxy-5-nitrobenzhydrol

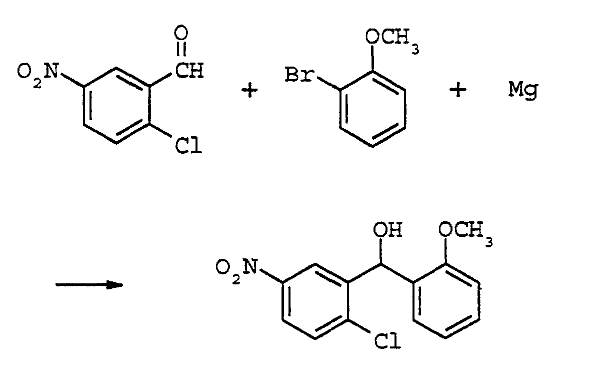

A solution of 2-bromoanisole (50.0 g, 0.267 mol) in ether is added portionwise to a mixture of magnesium (7.10 g, 0.293 mol) in ether. After the addition is complete, the reaction mixture is heated at reflux for one hour, diluted with ether, cooled to 0 °C, treated with a solution of 2-chloro-5-nitrobenzaldehyde (39.0 g, 0.210 mol) in tetrahydrofuran, warmed to room temperature, and diluted with an ice-water mixture. After acidifying the aqueous mixture with hydrochloric acid (pH 2 - pH 3), the organic phase is separated and the aqueous phase is extracted with ether. The organic extracts are combined, washed sequentially with 10% sodium hydrogen carbonate solution and brine, dried over anhydrous sodium sulfate, and concentrated *in vacuo* to give the title product as a brown gum.

Using essentially the same procedure, the following compounds are obtained:

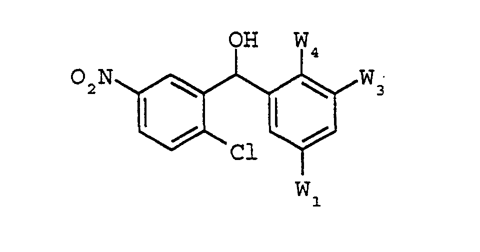

| **W** | **W**_{**3**} | **W**_{**4**} |
|---|---|---|
| OCH₃ | H | OCH₃ |
| CH₃ | H | OCH₃ |
| F | H | OCH₃ |
| H | OCH₃ | H |

### REFERENCE EXAMPLE 21

### Preparation of 2-Chloro-2'-methoxy-5-nitrobenzophenone

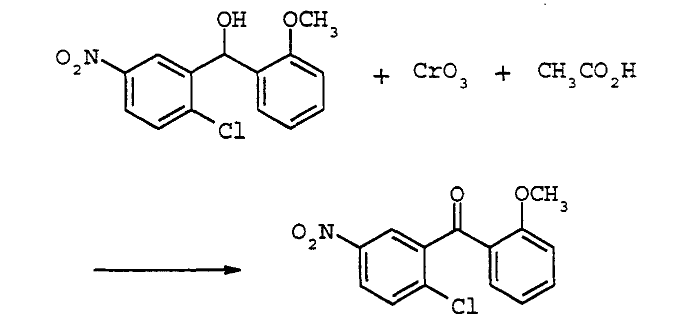

A solution of chromium(VI) oxide (91.0 g, 0.919 mol) in a water/acetic acid solution (1:4) is added portionwise to 2-chloro-2'-methoxy-5-nitrobenzhydrol (64.2 g, 0.219 mol) while maintaining the reaction mixture temperature at 25 °C to 35 °C. The reaction mixture is then stirred at 25 °C to 35 °C for one hour, cooled, diluted with water, and concentrated *in vacuo* to obtain a residue. The residue is diluted with water, and extracted with methylene chloride. The organic extracts are combined, dried over anhydrous sodium sulfate, mixed with silica gel (10 g), and filtered. The filtrate is concentrated *in vacuo* to obtain an oil. A solution of the oil in a methanol/water solution is decolorized with charcoal and concentrated *in vacuo* to yield a residue. Column chromatography of the residue using silica gel and methylene chloride/hexanes solutions gives the title product as a white solid.

Using essentially the same procedure, the following compounds are obtained:

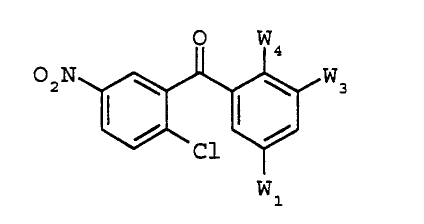

| **W**_{**1**} | **W**_{**3**} | **W**_{**4**} | **mp °C** |
|---|---|---|---|
| OCH₃ | H | OCH₃ | |
| CH₃ | H | OCH₃ | 109-111 |
| F | H | OCH₃ | 94-95 |
| H | OCH₃ | H | 79-81 |

### REFERENCE EXAMPLE 22

### Preparation of 2-Chloro-4-fluoro-5-nitrobenzoyl chloride

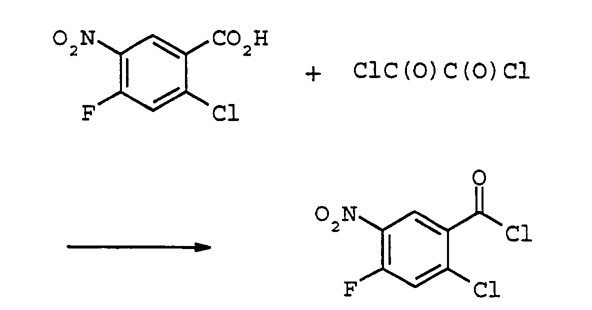

A mixture of 2-chloro-4-fluoro-5-nitrobenzoic acid (50.0 g, 0.228 mol) and N,N-dimethylformamide (5 drops) in 1,2-dichloroethane is treated dropwise with oxalyl chloride (30.8 mL, 0.353 mol), refluxed for 3 hours, cooled, and concentrated *in vacuo* to obtain the title product as an orange solid which is identified by NMR spectral analyses.

Following essentially the same procedure, but using 2,4-difluoro-5-nitrobenzoic acid, 2,4-difluoro-5-nitrobenzoyl chloride is obtained as a brown oil.

### REFERENCE EXAMPLE 23

### Preparation of 2'-Chloro-4'-fluoro-5'-nitroacetophenone

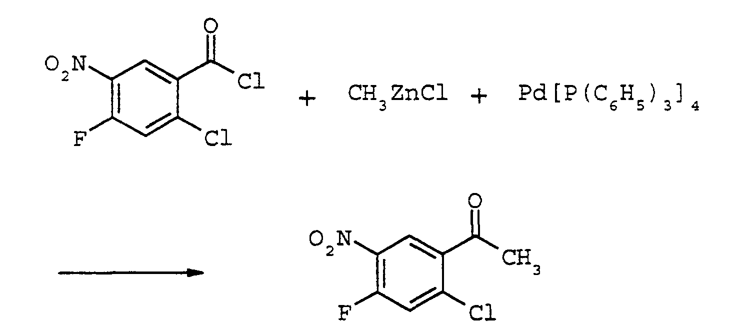

A 2 M solution of methylzinc chloride in tetrahydrofuran (5.00 mL, 10.1 mmol) is treated dropwise with a solution of 2-chloro-4-fluoro-5-nitrobenzoyl chloride (2.00 g, 8.40 mmol) in tetrahydrofuran, treated with tetrakis(triphenylphosphine)palladium(0) (0.400 g, 0.350 mmol), stirred at room temperature for one hour, and poured into 3 N hydrochloric acid. The resultant aqueous mixture is extracted with ethyl acetate. The organic extracts are combined, washed sequentially with water and saturated sodium hydrogen carbonate solution, dried over anhydrous magnesium sulfate, and concentrated *in vacuo* to obtain a dark liquid. Flash column chromatography of the liquid using silica gel and a methylene chloride in hexanes solution (6:4) gives the title product as an off-white solid (mp 66-68 °C) which is identified by NMR spectral analyses.

### REFERENCE EXAMPLE 24

### Preparation of 6-Amino-3-methyl-5-nitro-1,2-benzisothiazole

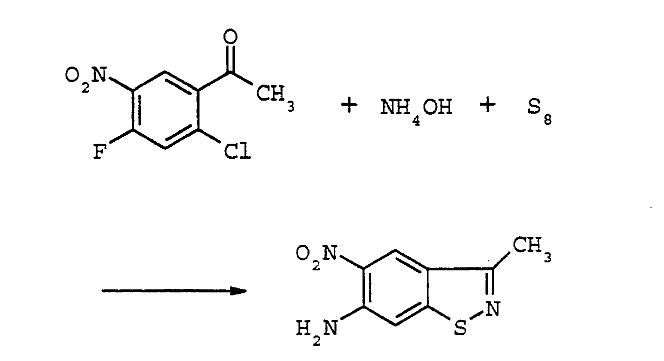

A mixture of 2'-chloro-4'-fluoro-5'-nitroacetophenone (12.0 g, 0.0552 mol), sulfur (1.77 g, 0.0552 mol), 30% ammonium hydroxide solution (100 mL, 0.856 mol), and methanol is placed in a steel bomb, heated at 85 °C overnight, cooled, treated with additional sulfur (0.270 g) and 30% ammonium hydroxide solution (50 mL), heated at 85 °C overnight, cooled, filtered to remove solids, and extracted with ethyl acetate. The organic extracts are combined, washed sequentially with water and brine, dried over anhydrous magnesium sulfate, and concentrated *in vacuo* to obtain a solid. Flash column chromatography of the solid using silica gel, and 0%, 1% and 2% diethyl ether in methylene chloride solutions gives the title product as an orange solid (4.19 g, mp 189-191 °C) which is identified by NMR spectral analyses.

Using essentially the same procedure, the following compound is obtained:

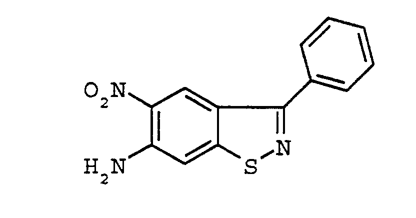

### REFERENCE EXAMPLE 25

### Preparation of 6-Chloro-3-methyl-5-nitro-1,2-benzisothiazole

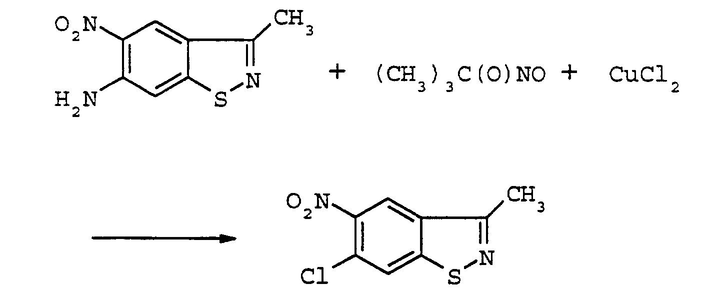

A mixture of tert-butyl nitrite (3.30 mL, 0.0278 mol) and copper(II) chloride (2.98 g, 0.0222 mol) in acetonitrile is heated to 65 °C, treated portionwise with 6-amino-3-methyl-5-nitro-1,2-benzisothiazole (3.88 g, 0.0185 mol), stirred at 65 °C, cooled to room temperature, and poured into 20% hydrochloric acid. The resultant aqueous mixture is extracted with ethyl acetate. The organic extracts are combined, washed with 20% hydrochloric acid, dried over anhydrous magnesium sulfate, and concentrated *in vacuo* to obtain a solid. Flash column chromatography of the solid using silica gel and methylene chloride/hexanes solutions (1:1 and 3:1) gives the title product as a pale, yellow solid (2.54 g, mp 156-158 °C) which is identified by NMR spectral analyses.

Using essentially the same procedure, the following compound is obtained:

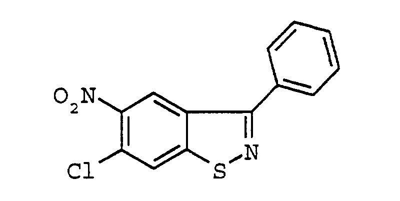

### REFERENCE EXAMPLE 26

### Preparation of 6-Fluoro-3-methyl-5-nitro-1,2-benzisothiazole

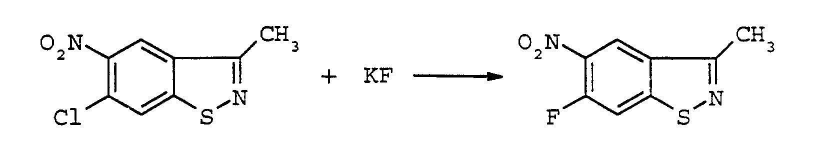

A mixture of 6-chloro-3-methyl-5-nitro-1,2-benzisothiazole (2.25 g, 9.80 mmol), potassium fluoride (2.85 g, 49.0 mmol), and 18-crown-6 (1.50 g, 5.70 mmol) in acetonitrile is heated in a sealed tube for 29 days, filtered to remove solids, and partially concentrated *in vacuo* to obtain a liquid. The liquid is diluted with ethyl acetate, washed sequentially with water and brine, dried over anhydrous magnesium sulfate, and concentrated *in vacuo* to obtain a dark, brown solid. Flash column chromatography of the solid using silica gel and a 10% to 50% ethyl acetate in hexanes gradient gives a yellow solid containing two components. Flash column chromatography of the yellow solid using silica gel and a 50% to 70% methylene chloride in hexanes gradient gives the title product as a pale, yellow solid (0.870 g, mp 118-119 °C) which is identified by NMR spectral analyses.

Using essentially the same procedure, the following compound is obtained:

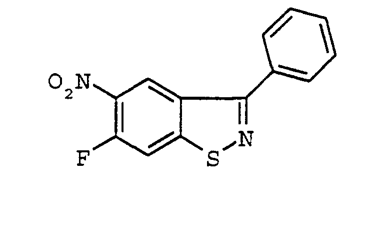

### REFERENCE EXAMPLE 27

### Preparation of 2,2'-Dithiobis[5-nitrobenzoic acid]

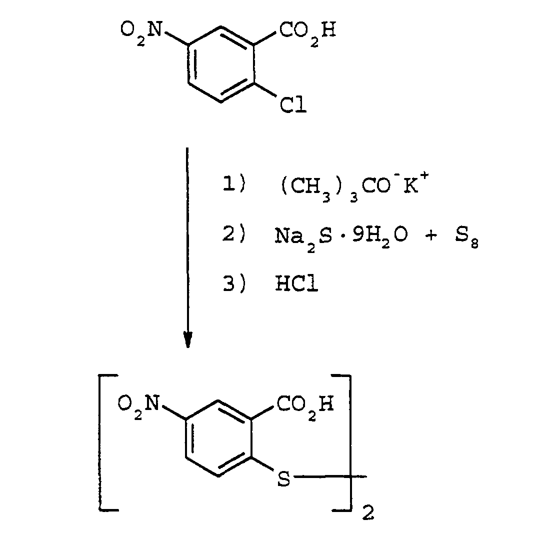

A mixture of 2-chloro-5-nitrobenzoic acid (100 g, 0.496 mol) in ethanol is treated portionwise with potassium tert-butoxide (55.5 g, 0.495 mol), diluted with additional ethanol, heated to reflux, treated portionwise with a solution prepared from sodium sulfide nonahydrate (60.0 g, 0.249 mol), sulfur (8.80 g, 0.274 mol) and water, refluxed for two hours, cooled to room temperature, and treated with concentrated hydrochloric acid. The resultant acidic mixture is stirred for one hour and filtered to obtain a solid. The solid is washed with water and air-dried to give the title product as a yellow powder which is identified by NMR spectral analysis.

### REFERENCE EXAMPLE 28

### Preparation of 5-Nitro-1,2-benzisothiazol-3(2H)-one

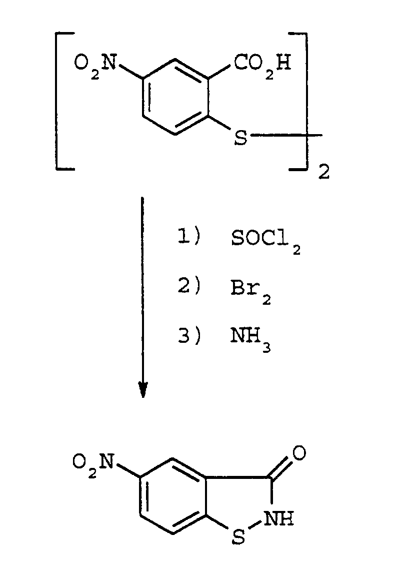

A mixture of 2,2'-dithiobis[5-nitrobenzoic acid] (44.6 g, 0.113 mol) and thionyl chloride (49.0 mL, 0.670 mol) in methylene chloride is treated with N,N-dimethylformamide (0.800 mL), refluxed overnight, concentrated *in vacuo,* and diluted with 1,2-dichloroethane. The resultant organic solution is treated with bromine (22.5 mL, 0.436 mol), stirred at room temperature for 20 minutes, refluxed for 3.5 hours, and concentrated *in vacuo* to obtain a residue. A solution of the residue in 1,2-dichloroethane is cooled with an ice-water bath, treated with concentrated ammonia (112 mL) over 15 minutes, stirred at room temperature for 16 hours, cooled with an ice-water bath, and treated with concentrated hydrochloric acid. The resultant aqueous mixture is stirred at room temperature for one hour and filtered to obtain a solid. The solid is washed with water and air-dried to give the title product as a yellow solid which is identified by NMR spectral analysis.

### REFERENCE EXAMPLE 29

### Preparation of 3-Chloro-5-nitro-1,2-benzisothiazole

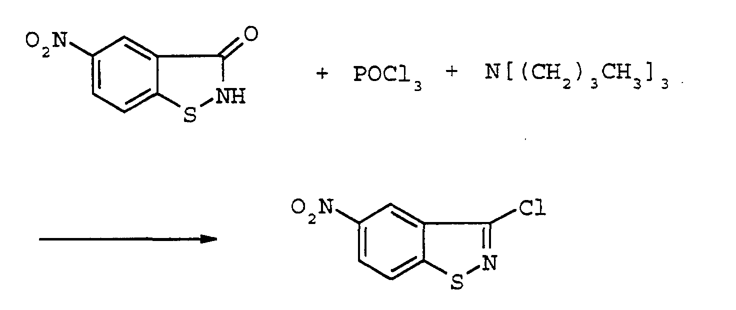

A mixture of 5-nitro-1,2-benzisothiazol-3(2H)-one (10.0 g, 0.0510 mol), phosphorus oxychloride (40.0 mL, 0.429 mol) and tributylamine (12.0 mL, 0.050 mol) is heated at 103-115 °C for six hours, stirred at room temperature overnight, and poured into an ice-water mixture. The resultant aqueous mixture is extracted with methylene chloride. The combined organic extracts are washed sequentially with water and saturated sodium hydrogen carbonate solution, dried over anhydrous sodium sulfate, and concentrated *in vacuo* to obtain a gum. Column chromatography of the gum using silica gel and methylene chloride gives the title product as an orange-yellow solid which is identified by NMR spectral analysis.

### REFERENCE EXAMPLE 30

### Preparation of Ethyl α-cyano-5-nitro-1,2-benzisothiazole-3-acetate

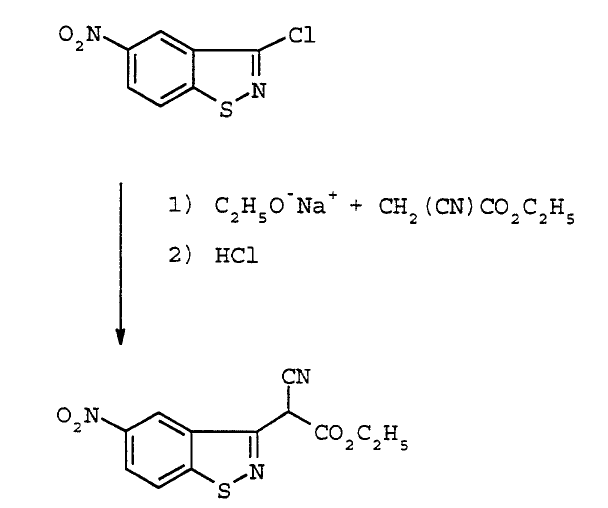

A sodium ethoxide solution (previously prepared from ethanol and sodium (1.00 g, 0.0430 mol)) is cooled with an ice-acetone bath, treated portionwise with ethyl cyanoacetate (4.51 g, 0.0398 mol), stirred at room temperature for 30 minutes, treated with 3-chloro-5-nitro-1,2-benzisothiazole (4.27 g, 0.0199 mol), stirred at room temperature overnight, cooled to 0 °C, and treated dropwise with 10% hydrochloric acid (15.0 mL). The resultant aqueous mixture is stirred at room temperature for one hour and filtered to obtain a solid. The solid is washed with ethanol and air-dried to give the title product as a yellow solid which is identified by NMR spectral analysis.

### REFERENCE EXAMPLE 31

### Preparation of Ethyl 5-nitro-1,2-benzisothiazole-3-acetate

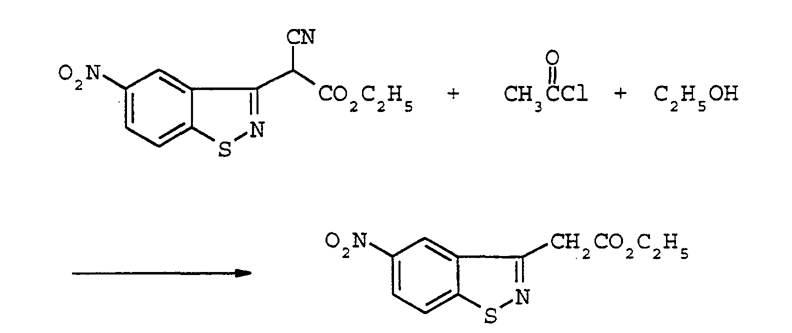

Ethyl α-cyano-5-nitro-1,2-benzisothiazole-3-acetate (6.67 g, 0.0229 mol) is added to a solution of acetyl chloride (67.0 mL) in ethanol. The reaction mixture is refluxed overnight, cooled, and filtered to remove solids. The resultant filtrate is concentrated *in vacuo* to obtain a brown semi-solid. A mixture of the semi-solid in diethyl ether is stirred for two hours and filtered to obtain a solid. The solid is washed with diethyl ether and air-dried to give the title product as yellow crystals (1.04 g, mp 91-92 °C).

### REFERENCE EXAMPLE 32

### Preparation of 5-Nitro-1,2-benzisothiazole-3-acetonitrile

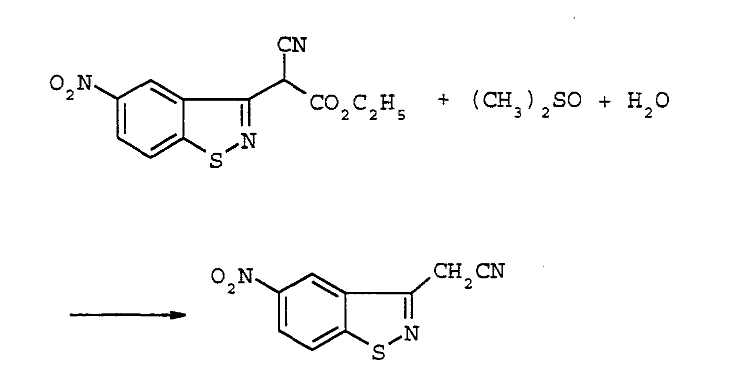

A mixture of ethyl 5-nitro-1,2-benzisothiazole-3-acetate (5.00 g, 17.2 mmol), water (1.00 mL), and methyl sulfoxide (35.0 mL) is stirred at 107 °C for 24 hours, stirred at room temperature for two days, and poured into an ice-water mixture. The resultant aqueous mixture is stirred for two hours and filtered to obtain a solid. The solid is washed with water and air-dried to give the title product as a tan solid.

### REFERENCE EXAMPLE 33

### Preparation of α,α-Dimethyl-5-nitro-1,2-benzisothiazole-3-acetonitrile

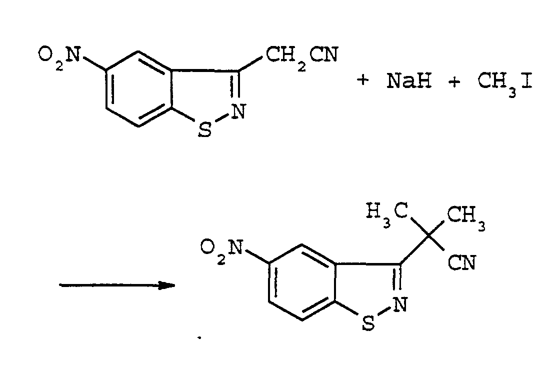

A mixture of 5-nitro-1,2-benzisothiazole-3-acetonitrile (1.29 g, 5.89 mmol) in N,N-dimethylformamide is cooled to -9 °C, treated with sodium hydride (1.00 g of a 60% dispersion in oil), stirred at -3 °C for 20 minutes, treated with iodomethane (5.00 mL), stirred at room temperature for four hours, and poured onto ice. The resultant aqueous mixture is treated with 10% hydrochloric acid and extracted with methylene chloride. The combined organic extracts are washed sequentially with water, saturated sodium hydrogen carbonate solution and water, dried over anhydrous sodium sulfate, and concentrated *in vacuo* to obtain a solid. column chromatography of the solid using silica gel and methylene chloride gives the title product as a yellow solid which is identified by NMR spectral analysis.

### REFERENCE EXAMPLE 34

### Preparation of Ethyl α,α-dimethyl-5-nitro-1,2-benzisothiazole-3-acetate

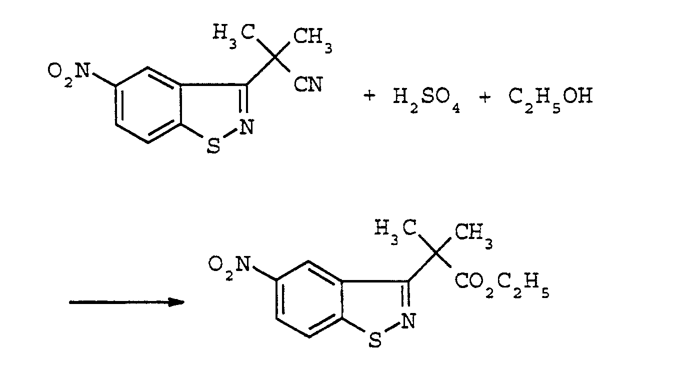

A mixture of α,α-dimethyl-5-nitro-1,2-benzisothiazole-3-acetonitrile (0.913 g, 3.69 mmol), water (0.450 mL), concentrated sulfuric acid (4.55 mL) and ethanol (9.10 mL) is refluxed for one hour, cooled, and poured onto ice. The resultant aqueous mixture is neutralized with saturated sodium bicarbonate solution and extracted with methylene chloride. The organic extract is washed with water, dried over anhydrous sodium sulfate, and concentrated *in vacuo* to obtain a solid. Column chromatography of the solid using silica gel and methylene chloride gives the title product as pale yellow crystals.
spectral analysis.

### REFERENCE EXAMPLE 35

### Preparation of 5-Amino-3-chloro-1,2-benzisothiazole

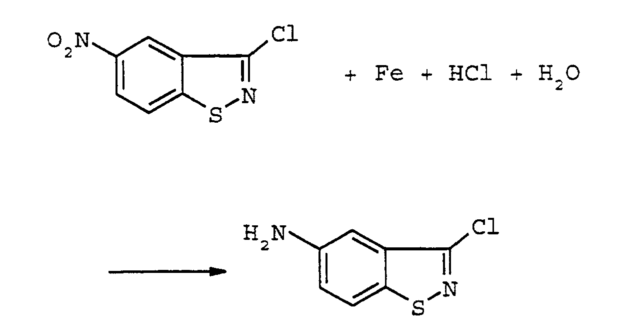

A solution of 3-chloro-5-nitro-1,2-benzisothiazole (2.00 g) in toluene is treated with iron powder (8.40 g, 325 mesh) and concentrated hydrochloric acid (8 drops), heated to reflux, treated dropwise with water (8.00 mL), refluxed for 35 minutes, cooled to room temperature, and filtered through diatomaceous earth. The resultant filtrate is concentrated *in vacuo* to obtain a residue. Flash column chromatography of the residue using silica gel and an ethyl acetate/hexanes solution (1:1) gives the title product.

### REFERENCE EXAMPLE 36

### Preparation of [(5-Nitro-1,2-benzisothiazol-3-yl)-oxy]acetonitrile

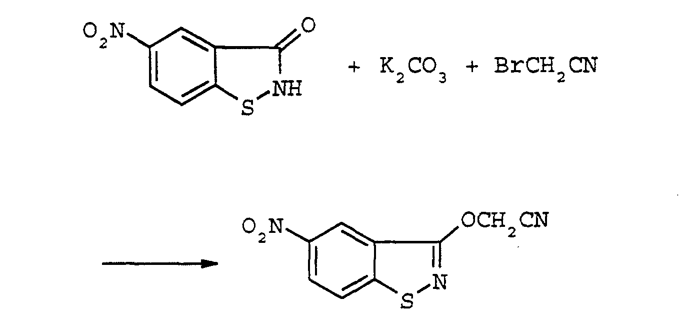

A mixture of 5-nitro-1,2-benzisothiazol-3(2H)-one (17.5 g, 89.2 mmol) in N,N-dimethylformamide is treated with potassium carbonate (18.5 g, 134 mmol), stirred at room temperature for 30 minutes, treated with bromoacetonitrile (16.0 g, 133 mmol), stirred at room temperature overnight, and poured onto ice. The resultant aqueous mixture is acidified to pH 3 with hydrochloric acid and extracted with ethyl acetate. The combined organic extracts are washed sequentially with water and brine, dried over anhydrous magnesium sulfate, and concentrated *in vacuo* to obtain a solid. Column chromatography of the solid using silica gel and methylene chloride gives the title product as a yellow solid (15.0 g, mp 123-124.5 °C).

Using essentially the same procedure, the following compounds are obtained:

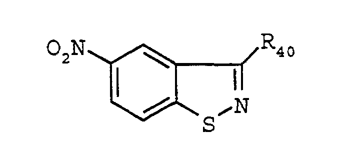

| **R**_{**40**} | **mp °C** |
|---|---|
| OCH₃ | 108-109 |
| OCH(CH₃)₂ | |
| OCH₂CH=CH₂ | |
| OCH₂C≡CH | 115-117 |
| OCH₂CO₂CH₃ | |

### REFERENCE EXAMPLE 37

### Preparation of 5-Nitro-1,2-benzisothiazole

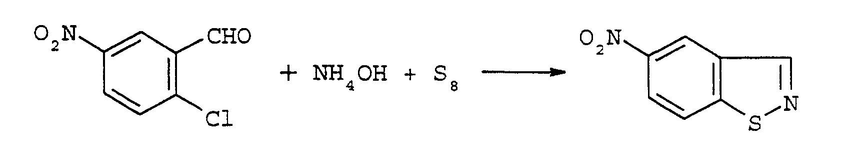

To a mixture of ammonium hydroxide (1000 ml) and N,N-dimethylformamide is added 2-chloro-5-nitrobenzaldehyde (300 g, 1.62 mol) and sulfur (54.4 g, 1.70 mol). The mixture is heated slowly to and stirred at 90 °C for one hour, cooled to room temperature, poured onto ice, and diluted with water. Filtration affords the title compound as a yellow solid (277.1 g, 94.9%).

Using essentially the same procedure, but using 2'-chloro-5'-nitro-2-methyl-2-carboethoxypropiophenone, ethyl α,α-dimethyl-5-nitro-1,2-benzisothiazole-3-acetate is obtained as a solid (mp 75-77°C).

### REFERENCE EXAMPLE 38

### Preparation of 3-Chloro-5-nitro-1,2-benzisothiazole

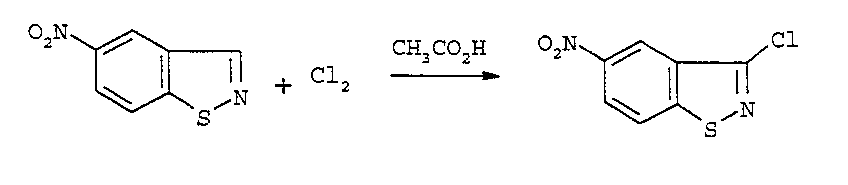

A suspension of 5-nitro-1,2-benzisothiazole (271 g, 1.50 mol) in acetic acid is heated to 80 °C to form a solution. The heating source is removed and chlorine gas is added continuously over six hours at 70-80 °C until saturation of the mixture occurs. The mixture is cooled t room temperature and stirred overnight. Filtration afford the title compound as a yellow crystalline solid (237 g, 73.6%) which is identified by NMR spectral analysis.

### REFERENCE EXAMPLE 39

### Preparation of 2'-Chloro-2-methyl-2-carboethoxy propiophenone

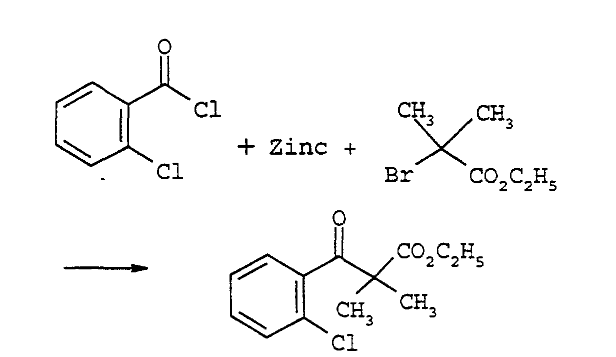

A mixture of 2-chlorobenzoyl chloride (52.2 g, 0.298 mol), ethyl 2-bromoisobutyrate (58.2 g, 0.298 mol) and ether is added in portions to zinc foil (19.5 g, 0.298 mol) and the resultant mixture stirred at reflux for three hours and overnight at room temperature. The mixture is poured into cold, dilute sulfuric acid and the organic layer is washed with saturated sodium bicarbonate and brine, dried over anhydrous magnesium sulfate and concentrated *in vacuo* to a yellow oil. The oil is chromatographed on silica gel with hexanes:ethyl acetate to afford the title compound as a colorless oil (41.8 g, 55.1%).

### REFERENCE EXAMPLE 40

### Preparation of 2'-Chloro-5'-nitro-2-methyl-2-carboethoxypropiophenone

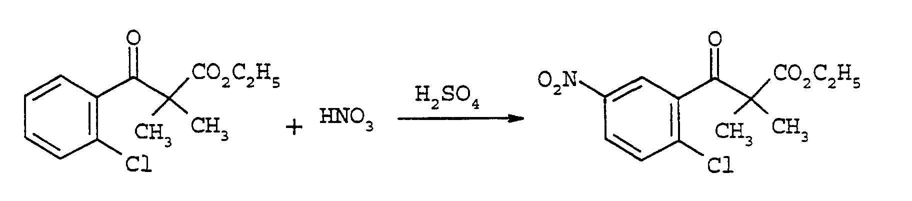

To concentrated sulfuric acid (15.0 ml) at 5 °C is added 2'-chloro-2-methyl-2-carboethoxypropiophenone (4.00 g, 0.01570 mol) followed by dropwise addition of concentrated nitric acid (90%, 0.740 ml, 0.0204 mol). After stirring 5 minutes, the mixture is poured onto ice and extracted with ethyl acetate. The organic layers are washed with saturated sodium bicarbonate and brine, dried over anhydrous magnesium sulfate, filtered and concentrated *in vacuo* to afford the title compound as a yellow oil (3.90 g, 83.0%) which is identified by NMR spectral analysis.

### REFERENCE EXAMPLE 41

### Preparation of 1-Benzothiophen-2,3-dione

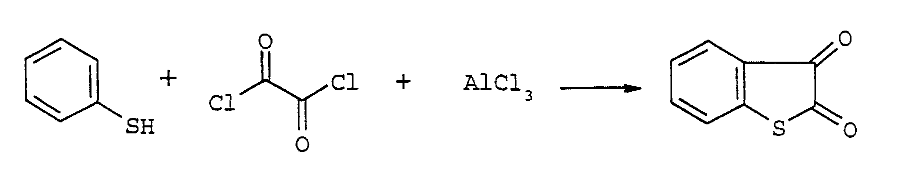

To a solution of thiophenol (100 g, 0.907 mol) in ether is added dropwise a solution of oxalyl chloride (175 g, 1.38 mol) in ether. The mixture is stirred two hours at reflux and concentrated *in vacuo*. The residue is taken up in methylene chloride and cooled to 0 °C. Aluminum chloride (145 g, 1.09 mol) is added in portions such that the temperature does not exceed 25 °C. The resultant mixture is stirred 30 minutes at reflux, cooled to room temperature and poured into ice water with stirring. The organic layer is washed with saturated sodium bicarbonate, water and brine, dried over anhydrous magnesium sulfate, filtered and concentrated *in vacuo* to an orange solid which is recrystallized from methylene chloride:hexanes to afford the title compound (102 g, 69.0%) which is identified by NMR spectral analysis.

### REFERENCE EXAMPLE 42

### Preparation of 1,2-Benzisothiazole-3-carboxamide

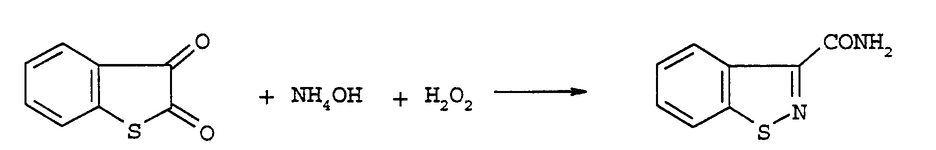

To ammonium hydroxide (1.78 1) is added 1-benzothiophen-2,3-dione (87.0 g, 0.530 mol) at 5-10 °C, followed by hydrogen peroxide (30% aqueous, 178 ml). The resultant mixture is filtered to obtain a yellow solid which is dried (77.0 g, 81.7%) and identified as the title compound by NMR and IR spectral analysis.

### REFERENCE EXAMPLE 43

### Preparation of 3-Cyano-5-nitro-2,2-benzisothiazole

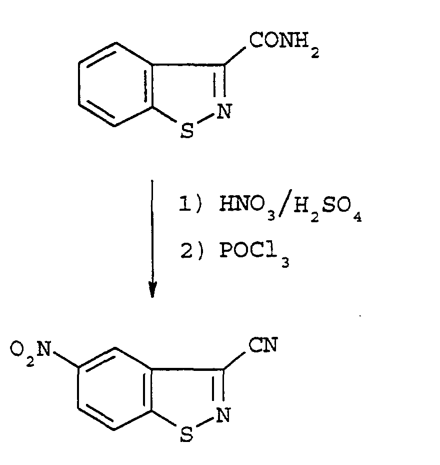

A solution of 1,2-benzisothiazole-3-carboxamide (12.0 g, 0.0674 mol) in concentrated sulfuric acid at 0-5 °C is treated dropwise with nitric acid (90%, 4.12 ml) such that the temperature does not exceed 10 °C, stirred one hour at 5 °C, and poured into ice water with vigorous stirring. The resultant suspension is filtered to obtain a solid. The solid is dried and recrystallized from acetonitrile to afford a white solid (10.0 g) which is treated with phosphorus oxychloride (60.0 ml). The resultant mixture is stirred at 90-100 °C for 90 minutes, cooled to room temperature, slowly poured into ice water with stirring, and filtered to obtain a solid. Recrystallization of the solid from methylene chloride:hexanes gives the title compound as an orange solid (8.00 g, 87.9%, mp 168-170 °C) which is identified by NMR and IR spectral analyses.

### REFERENCE EXAMPLE 44

### Preparation of 3-(6-Methoxy-m-tolyl)-6-amino-5-nitro-1,2-benzisothiazole

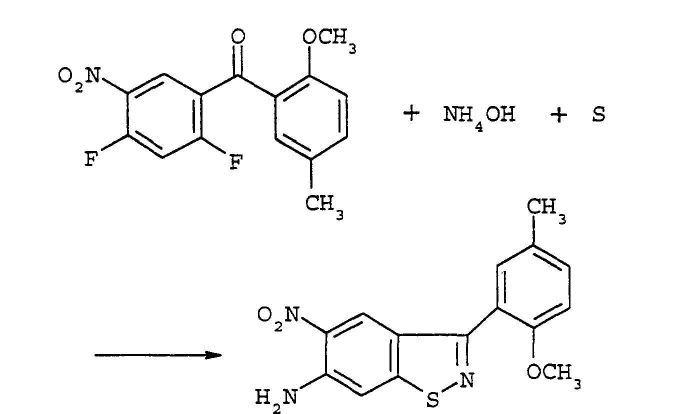

Ammonium hydroxide (330 ml) is added to a suspension of 2',4'-difluoro-2-methoxy-5-methyl-5'-nitrobenzophenone (60.0 g, 0.186 mol), sulfur (6.25 g, 0.195 mol) and N,N-dimethylformamide on an ice bath. The resultant mixture is allowed to warm to 35 °C, heated gradually to 81 °C over a two hour period, cooled to room temperature, and poured into water. The resultant solid is taken up in ethyl acetate and N,N-dimethylformamide, and washed with water. The organic layer is concentrated *in vacuo* to afford the title compound which is identified by NMR spectral analysis.

### REFERENCE EXAMPLE 45

### Preparation of 3-(6-Methoxy-m-tolyl)-6-chloro-5-nitro-1,2-benzisothiazole

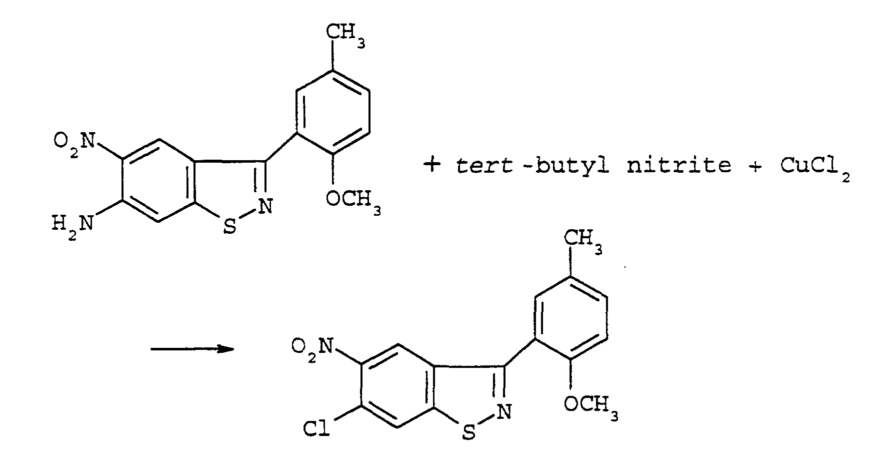

A mixture of *tert*-butyl nitrite (5.90 g, 0.0571 mol), copper chloride (6.20 g, 0.0457 mol) and acetonitrile is heated to 65-75 °C, treated with 3-(6-methoxy-*m*-tolyl)-6-amino-5-nitro-1,2-benzisothiazole (12.0 g, 0.0381 mol) over 10 minutes, stirred for two hours at 67-75 °C, treated with *tert*-butyl nitrite (1.50 ml) and copper chloride (1.00 g), stirred 40 minutes at 67-75°C, cooled to room temperature, and diluted with ethyl acetate. The organic layer is washed with 10% hydrochloric acid and filtered. The filtrate is washed with water and concentrated *in vacuo* to afford the title compound as a solid (10.6 g, 83.1%) which is identified by NMR and IR spectral analyses.

### REFERENCE EXAMPLE 46

### Preparation of 3-(6-Methoxy-m-tolyl)-6-fluoro-5-nitro-1,2-benzisothiazole

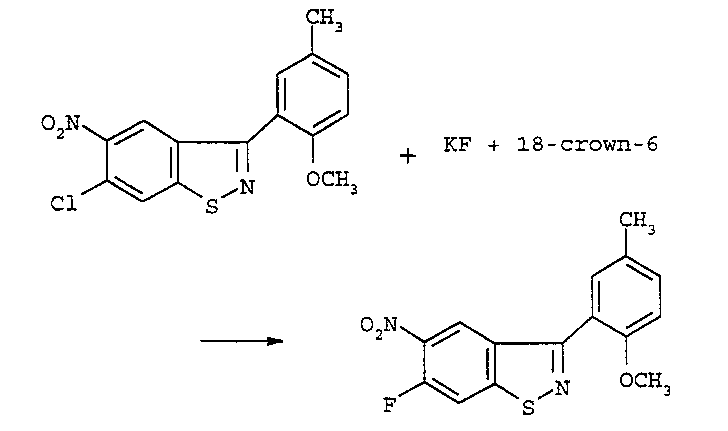

A mixture of 3-(6-methoxy-*m*-tolyl)-6-chloro-5-nitro-1,2-benzisothiazole (7.30 g, 0.0218 mol), potassium fluoride (6.33 g, 0.109 mol) 18-crown-6 (2.31 g, 0.0872 mol) and sulfolane is stirred 19 hours at 154°C, cooled to room temperature, and poured into ice water. The resultant solid is filtered and chromatographed on silica gel with methylene chloride to afford a solid which is recrystallized from acetonitrile to afford a tan powder. The powder is recrystallized from ethyl acetate to give the title compound as a tan solid (2.09 g, 29.9%) which is identified by NMR spectral analysis.

### REFERENCE EXAMPLE 47

### Preparation of 5-Amino-4-bromo-6-fluoro-3-methyl-1,2-benzisothiazole

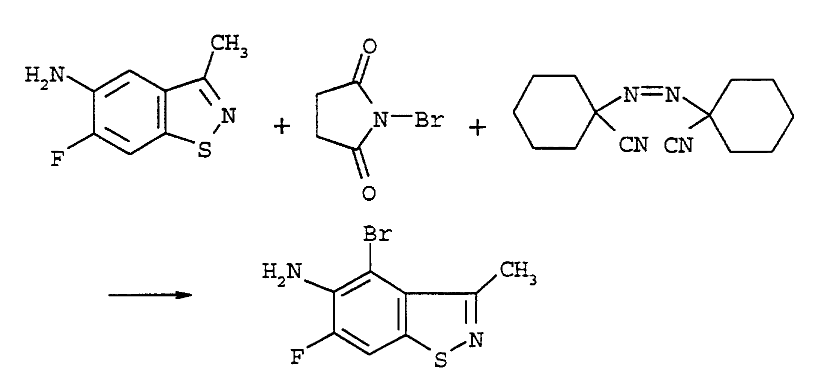

To a solution of 5-amino-6-fluoro-3-methyl-1,2-benzisothiazole (0.600 g, 0.00329 mol) in 1,2-dichloroethane is added N-bromosuccinimide (0.586 g, 0.00329 mol) followed by 1,1'-azobis(cyclohexanecarbonitrile) (0.0200 g). The mixture is stirred two hours at 70 °C, additional N-bromosuccinimide (0.240 g, 0.00135 mol) is added, and the mixture is stirred 40 minutes at 70°C. The mixture is then cooled to room temperature, filtered and concentrated *in vacuo* to obtain a residue. The residue is chromatographed on silica gel to give the title compound (0.870 g, 100%) which is identified by NMR spectral analysis.

## Claims

1. A process for the preparation of a 6-(perfluoroalkyl)uracil compound having the structural formula I 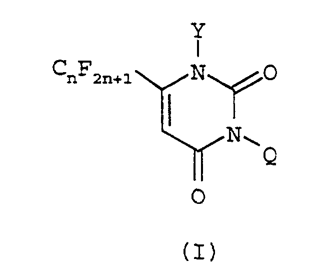 wherein
n is an integer of 1, 2, 3, 4, 5 or 6;
Y is hydrogen or C₁-C₆alkyl; and
Q is a C₁-C₆alkyl group or an optionally substituted phenyl, benzyl, heteroaryl or methyleneheteroaryl group,
which process comprises
(a) reacting a carbamate compound having the structural formula II 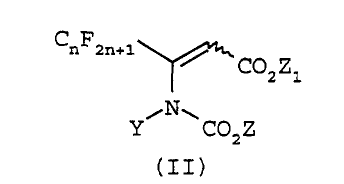 wherein
Z and Z₁ are each independently C₁-C₆alkyl or benzyl optionally substituted on the phenyl ring with any combination of from one to three halogen, C₁-C₄alkyl or C₁-C₄haloalkyl groups; and
n and Y are as described above,
with an amine compound having the structural formula III
QNH₂ (III)
wherein Q is as described above, in the presence of a base, to form the 6-(perfluoroalkyl)uracil compound of formula I wherein Y is hydrogen or C₁-C₆alkyl; and
(b) optionally alkylating the formula I compound
wherein Y is hydrogen to form the formula I compound
wherein Y is C₁-C₄alkyl.

2. A process according to claim 1 wherein the double bond in the formula II compound is predominately in the (Z)- configuration.

3. A process according to claim 1 wherein the base is selected from the group consisting of a tri(C₁-C₆alkyl)-amine, a heterocyclic tertiary amine and an alkali metal C₁-C₆alkoxide.

4. A process according to claim 3 wherein the base is selected from the group consisting of 1,8-diazabicyclo-[5.4.0]undec-7-ene and 1,5-diazabicyclo[4.3.0]non-5-ene.

5. A process according to claim 1 wherein the carbamate compound is reacted with the amine compound in the presence of a solvent.

6. A process according to claim 5 wherein the solvent is selected from the group consisting of a carboxylic acid amide, a dialkyl sulfoxide, an aromatic hydrocarbon, a halogenated aromatic hydrocarbon, an aliphatic hydrocarbon, a halogenated aliphatic hydrocarbon, an alcohol, a ketone, an ether, a nitrile and water and mixtures thereof.

7. A process according to claim 1 wherein the carbamate compound is reacted with the amine compound and the base at a temperature of about 20°C to 150°C.

8. A process according to claim 1 wherein step (b) comprises reacting the formula I compound wherein Y is hydrogen with an alkyl halide having the structural formula IV or a dialkylsulfate ester having the structural formula V
XY (IV)
or 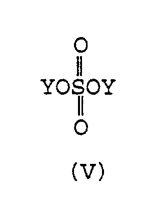 wherein X is chlorine, bromine or iodine, and Y is C₁-C₆alkyl, in the presence of a base.

9. A process according to claim 1 wherein
n is 1;
Y is hydrogen or C₁-C₄alkyl;
Q is 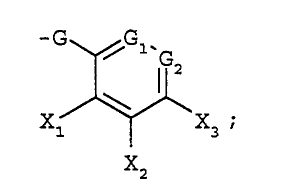
G is CH₂ or a bond;
G₁ is CX₅ or N;
G₂ is CX₄ or N;
X₁ is hydrogen, halogen or a C₁-C₆alkyl group optionally substituted with one epoxy group,
X₂ is hydrogen, halogen NRR₁, CO₂R₂, C(O)R₃, OR₄, SO₂R₅, SO₂NR₆R₇, C(R₈)(OR₉)₂, C(R₁₀)=NOR₁₁, C(R₁₂)=C(R₁₃)-C(OR₁₄)=NOR₁₅, CH₂O-NCO₂R₁₆,
1,3-dioxolane optionally substituted with one C₁-C₆alkoxy group or one or two C₁-C₄alkyl groups,
1,3-dioxolinone optionally substituted with one C₁-C₆alkoxy group or one or two C₁-C₄alkyl groups, or
C₁-C₄alkyl optionally substituted with one CO₂R₂ group and one halogen atom, and
X₃ is hydrogen, halogen, C₁-C₄haloalkyl, CO₂R₁₇, cyano, C₁-C₄haloalkoxy, OR₁₈ or C₁-C₄alkyl, or
when X₁ and X₂ are taken together with the atoms to which they are attached, they may form a five- or six-membered ring wherein X₁X₂ or X₂X₁ is represented by: -OC(R₂₀)(R₂₁)O-, -CH₂S(O)ₚN(R₂₂)-, -SC(R₂₃)=N-, -CH=CH-CH(R₁₁)O-, -OC(O)N-, -SC(R₂₄)=N-, -ON(R₂₅)C(O)-, -OC(CO₂R₂₆)=C(R₂₇)-, -NC(R₂₈)=C(SR₂₉)-, -CH=C(CO₂R₃₀)O-, -CH₂CH(R₃₁)O- or -OC(R₃₂)(R₃₃)C(O)-, or
when X₂ and X₃ are taken together with the atoms to which they are attached, they may form a five- or six-membered ring wherein X₂X₃ or X₃X₂ is represented by: -NC(R₃₄)=NC(S)-, -N(R₃₅)N=C(R₃₆)-, -N(R₃₇)C(R₃₈)=N-, -N(R₃₈)C(O)CH₂O-, -N(R₃₉)C(O)CH=CH-, -S-N=C(R₄₀)-, -O-N=C(R₄₁)-, -N=N-N(R₄₂)-, -C(R₄₃)(R₄₄)C(O)N(R₄₅)- or -N(R₄₆)C(O)C(R₄₇)(R₄₈)-;
X₄ is hydrogen, halogen or OR₁₉;
X₅ is hydrogen or halogen;
R, R₅₆, R₆₄, R₆₉, R₇₀, R₇₇ and R₉₁ are each independently hydrogen, SO₂R₄₉, C₁-C₄alkyl, C₃-C₇cycloalkyl, C₃-C₆alkenyl, C₃-C₆alkynyl, phenyl or benzyl;
R₁ is hydrogen, SO₂R₅₀, C(O)R₅₁, amino or C₁-C₄alkyl optionally substituted with CO₂R₅₂ or C(O)R₅₃;
R₂, R₁₆, R₁₇, R₂₆, R₃₀, R₆₈, R₇₅, R₇₆, R₈₂ and R₈₈ are each independently hydrogen, C₁-C₈haloalkyl, C₃-C₈alkenyl, C₃-C₆alkynyl, phenyl, benzyl, furfuryl, pyridyl, thienyl,
C₁-C₈alkyl optionally substituted with CO₂R₅₄, morpholine or C(O)R₅₅, or
an alkali metal, an alkaline earth metal, ammonium or organic ammonium cation;
R₃, R₆₆, R₆₇, R₈₁, R₈₅ and R₈₉ are each independently hydrogen, C₁-C₆alkyl, C₃-C₆alkenyl, C₃-C₆alkynyl, NR₅₆R₅₇, phenyl or benzyl;
R₄, R₁₈, R₁₉ and R₆₅ are each independently hydrogen, C₁-C₆alkyl, C₃-C₆alkenyl, C₃-C₆alkynyl, C₁-C₄haloalkyl, C(O)R₅₈ , C(S)R₅₉ or benzyl;
R₅ and R₇₂ are each independently C₁-C₆alkyl, C₁-C₆haloalkyl, NR₆₀R₆₁, imidazole or indazole;
R₆, R₁₁, R₁₂, R₁₄, R₁₅, R₂₀, R₂₁, R₂₂, R₂₅, R₂₈, R₂₉, R₃₁, R₃₂, R₃₃, R₃₅, R₄₅, R₄₆, R₆₃ and R₈₀ are each independently hydrogen or C₁-C₄alkyl;
R₇ is hydrogen, C₃-C₆alkenyl, C₃-C₆alkynyl, benzyl, or C₁-C₄alkyl optionally substituted with cyano or C(O)R₆₂;
R₈ and R₂₇ are each independently hydrogen, C₁-C₄alkyl or C₁-C₄alkoxy;
R₉ and R₉₀ are each independently C₁-C₆alkyl;
R₁₀ is hydrogen, C₁-C₆alkyl, phenyl or benzyl;
R₁₃, R₂₄ and R₃₆ are each independently hydrogen, C₁-C₆alkyl or halogen;
R₂₃ is hydrogen or NR₆₃R₆₄;
R₃₄ is hydrogen, C₁-C₄alkyl or C₁-C₄haloalkyl;
R₃₇ is hydrogen, C₁-C₄alkyl or C₂-C₈alkoxyalkyl;
R₃₈ and R₃₉ are each independently hydrogen, C₁-C₄alkyl, C₁-C₄haloalkyl, C₃-C₆alkenyl or C₃-C₆alkynyl;
R₄₀, R₄₁ and R₄₂ are each independently hydrogen, halogen, cyano, OR₆₅, C (O)R₆₆, C (S) R₆₇, CO₂R₆₈, C (=NOR₆₉),
a C₁-C₈alkyl, C₃-C₇cycloalkyl, C₂-C₈alkenyl or C₂-C₈alkynyl group, wherein each group is optionally substituted with any combination of one to six halogen atoms, one to three C₁-C₁₀-alkoxy groups, one or two C₁-C₆haloalkoxy groups, one or two NR₇₀R₇₁ groups, one or two S(O)_{q}R₇₂ groups, one or two cyano groups, one or two C₃-C₇cycloalkyl groups, one OSO₂R₇₃ group, one or two C(O)R₇₄ groups, one or two CO₂R₇₅ groups, one or two C(O)SR₇₆ groups, one or two C(O)NR₇₇R₇₈ groups, one to three OR₇₉ groups, one or two P(O)(OR₈₀)₂ groups, one 1,3-dioxolane optionally substituted with one to three C₁-C₄alkyl groups, or one 1,3-dioxane optionally substituted with one to three C₁-C₄alkyl groups, or
phenyl or benzyl optionally substituted with any combination of one to three halogen atoms, one to three C₁-C₆alkyl groups, one to three C₁-C₆alkoxy groups, one C₃-C₇cycloalkyl group, one C₁-C₄haloalkyl group, one C₁-C₄alkylthio group, one cyano group, one nitro group, one C(O)R₈₁ group, one CO₂R₈₂ group, one OR₈₃ group, one SR₈₄ group, one C₁-C₆alkoxymethyl group, one hydroxymethyl group, one C₃-C₈alkenyloxymethyl group, or one C₁-C₈haloalkoxymethyl group;
R₄₃, R₄₄, R₄₇ and R₄₈ are each independently hydrogen, C₁-C₄alkyl, C₁-C₄haloalkyl, C₃-C₆alkenyl, C₃-C₆alkynyl or C₃-C₇cycloalkyl, or R₄₃ and R₄₄ or R₄₇ and R₄₈ may be taken together with the atom to which they are attached to form a C₃-C₇cycloalkyl group;
R₄₉, R₅₀ and R₈₆ are each independently C₁-C₆alkyl, NR₉₃R₉₄, C₁-C₄haloakyl, C₃-C₆alkenyl, C₃-C₆alkynyl or benzyl;
R₅₁, R₅₂, R₅₃, R₅₄, R₅₅, R₅₇, R₅₈, R₅₉, R₆₀, R₆₁, R₆₂, R₇₁, R₇₃, R₇₄, R₇₈, R₈₇ and R₉₂ are each independently hydrogen, C₁-C₆alkyl, C₃-C₇cycloalkyl, C₁-C₆haloalkyl, C₃-C₆alkenyl, C₃-C₆alkynyl, phenyl or benzyl;
R₇₉, R₈₃ and R₈₄ are each independently hydrogen, C(O)R₈₅, SO₂R₈₆, C₁-C₆haloalkyl, C₂-C₆alkenyl, C₅-C₈cycloalkenyl, C₂-C₆alkynyl, phenyl, benzyl, or C₁-C₁₀alkyl optionally substituted with one hydroxyl, benzyloxy, OC(O)R₈₇, C₁-C₆alkoxy, CO₂R₈₈, C(O)R₈₉, C(OR₉₀)₂, C (O)NR₉₁R₉₂ or cyano group;
R₉₃ and R₉₄ are each independently hydrogen, C₁-C₄haloalkyl, C₂-C₆alkenyl, C₃-C₈cycloalkyl,
C₁-C₈alkyl optionally substituted with one or two C₁-C₄alkoxy groups or one cyanoalkyl group, or benzyl or phenyl optionally substituted with any combination of one to three halogen atoms, one to three C₁-C₄alkyl groups, one to three C₁-C₄haloalkyl groups, one to three C₁-C₄alkoxy groups, one to three C₁-C₄haloalkoxy groups, one cyano group or one nitro group, and
when R₉₃ and R₉₄ are taken together with the atom to which they are attached, they form a 5- to 12-membered monocyclic or fused bicyclic, heterocyclic ring optionally substituted with one or more groups independently selected from halogen, cyano, nitro, amino, hydroxyl, C₁-C₄alkyl, C₁-C₄haloalkyl, C₁-C₄alkoxy, C₁-C₄haloalkoxy and C₁-C₄haloalkylsulfonyl groups;
p and q are each independently 0, 1 or 2; and
Z and Z₁ are each independently C₁-C₄alkyl.

10. A process according to claim 9 wherein
Y is hydrogen or methyl;
X₁ is hydrogen, fluorine or C₁-C₃alkyl optionally substituted with one epoxy group;
X₂ is hydrogen, halogen NRR₁, CO₂R₂, C(O)R₃, OR₄, SO₂R₅, SO₂NR₆R₇, C(R₈)(OR₉)₂, C (R₁₀) =NOR₁₁, C (R₁₂)=C(R₁₃)-C (OR₁₄)=NOR₁₅, CH₂O-NCO₂R₁₆,
1,3-dioxolane optionally substituted with one C₁-C₆alkoxy group or one or two C₁-C₄alkyl groups,
1,3-dioxolinone optionally substituted with one C₁-C₆alkoxy group or one or two C₁-C₄alkyl groups, or
C₁-C₄alkyl optionally substituted with one CO₂R₂ group and one halogen atom, and
X₃ is hydrogen, halogen, C₁-C₄haloalkyl, CO₂R₁₇, cyano, C₁-C₄haloalkoxy, OR₁₈ or C₁-C₄alkyl, or
when X₁ and X₂ are taken together with the atoms to which they are attached, they may form a five- or six-membered ring wherein X₁X₂ or X₂X₁ is represented by: -OC(R₂₀)(R₂₁)O-, -CH₂S(O)ₚN(R₂₂)-, -SC(R₂₃)=N-, -CH=CH-CH(R₁₁)O-, -OC(O)N-, -SC(R₂₄)=N-, -ON(R₂₅)C(O)-, -OC(CO₂R₂₆)=CH-, -NC(R₂₈)=C(SR₂₉)-, -CH=C(CO₂R₃₀)O-, -CH₂CH(R₃₁)O- or -OC(R₃₂)(R₃₃)C(O)-, or
when X₂ and X₃ are taken together with the atoms to which they are attached, they may form a five- or six-membered ring wherein X₂X₃ or X₃X₂ is represented by: -NC(R₃₄)=NC(S)-, -N(R₃₅)N=C(R₃₆)-, -N(R₃₇)C(R₃₈)=N-, -N(R₃₈)C(O)CH₂O-, -N(R₃₉)C(O)CH=CH-, -S-N=C(R₄₀)-, -O-N=C(R₄₁)-, -N=N-N(R₄₂)-, -C(R₄₃)(R₄₄)C(O)N(R₄₅)- or -N(R₄₆)C(O)C(R₄₇)(R₄₈)-;
X₄ is hydrogen, halogen or OR₁₉;
X₅ is hydrogen or halogen;
R, R₆₄, R₆₉ and R₇₇ are each independently hydrogen, SO₂R₄₉ or C₁-C₄alkyl;
R₁ is hydrogen, SO₂R₅₀, C(O)R₅₁, amino or C₁-C₄alkyl optionally substituted with CO₂R₅₂ or C(O)R₅₃;
R₂, R₁₆, R₁₇, R₂₆, R₃₀, R₆₈, R₇₅, R₇₆, R₈₂ and R₈₈ are each independently hydrogen, C₃-C₆alkenyl or C₁-C₄alkyl optionally substituted with CO₂R₅₄, morpholine or C(O)R₅₅;
R₃, R₆₆, R₆₇, R₈₅ and R₈₉ are each independently hydrogen, C₁-C₄alkyl or NR₅₆R₅₇;
R₄, R₁₈ and R₁₉ are each independently hydrogen, C₁-C₄alkyl, C₁-C₄haloalkyl, C(O)R₅₈, C₃-C₄alkenyl or C₃-C₄alkynyl;
R₅₆ is SO₂R₄₉;
R₅₇ is hydrogen or C₁-C₄alkyl;
R₅ and R₇₂ are each independently NR₆₀R₆₁ or indazole;
R₆, R₁₁, R₁₂, R₁₄, R₁₅, R₂₀, R₂₁, R₂₂, R₂₅, R₂₈, R₂₉, R₃₁, R₃₂, R₃₃, R₃₅, R₄₅, R₄₆ and R₈₀ are each independently hydrogen or methyl;
R₇ is C₁-C₄alkyl optionally substituted with cyano or C(O)R₆₂;
R₈ is hydrogen or C₁-C₄alkoxy;
R₉ and R₉₀ are each independently C₁-C₄alkyl;
R₁₀ is hydrogen or C₁-C₃alkyl;
R₁₃, R₂₄ and R₃₆ are each independently hydrogen or chlorine;
R₂₃ is NR₆₃R₆₄;
R₃₄ is C₁-C₃haloalkyl;
R₃₇ is C₂-C₄alkoxyalkyl;
R₃₈ and R₃₉ are each independently C₁-C₃haloalkyl, C₁-C₃alkyl or propargyl;
R₄₀, R₄₁ and R₄₂ are each independently hydrogen, C(O)R₆₆, C(S)R₆₇, CO₂R₆₈, C(=NOR₆₉),
C₁-C₃alkyl optionally substituted with any combination of one or two halogen atoms, one or two C₁-C₃alkoxy groups, one or two C₁-C₃haloalkoxy groups, one SO₂R₇₂ group, one or two cyano groups, one C₃-C₅cycloalkyl group, one OSO₂R₇₃ group, one C(O)R₇₄ group, one CO₂R₇₅ group, one C(O)SR₇₆ group, one C(O)NR₇₇R₇₈ group, one to two OR₇₉ groups, one P(O)(OR₈₀)₂ group, one 1,3-dioxolane group or one 1,3-dioxane group, or
phenyl optionally substituted with any combination of one halogen atom, one or two methyl groups, one methoxy group, one halomethyl group or one OR₈₃ group;
R₄₃, R₄₄, R₄₇ and R₄₈ are each independently hydrogen or methyl, or R₄₃ and R₄₄ or R₄₇ and R₄₈ may be taken together with the atom to which they are attached to form a cyclopropyl group;
R₄₉, R₅₀ and R₈₆ are each independently C₁-C₄alkyl or NR₉₃R₉₄;
R₅₁, R₅₂, R₅₃, R₅₄, R₅₅, R₅₈, R₆₀, R₆₁, R₆₂, R₇₃, R₇₄, R₇₈ and R₈₇are each independently hydrogen, C₁-C₄alkyl or C₁-C₄haloalkyl;
R₇₉ and R₈₃ are each independently hydrogen, C(O)R₈₅, SO₂R₈₆, C₁-C₄haloalkyl, C₃-C₄alkenyl or C₁-C₃alkyl substituted with one OC(O)R₈₇, CO₂R₈₈, C(O)R₈₉, C(OR₉₀)₂ or cyano group;
R₉₃ and R₉₄ are each independently hydrogen or C₁-C₈alkyl;
p is 0, 1 or 2; and
Z and Z₁ are each independently methyl or ethyl.

11. A process according to claim 1 for the preparation of a 6-(trifluoromethyl)uracil compound having the structural formula VI 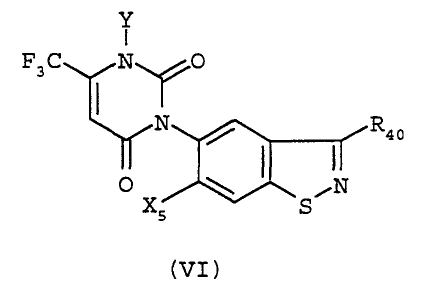 wherein
Y is hydrogen or methyl;
X₅ is hydrogen or halogen;
R₄₀ is hydrogen, C(O)R₆₆, C(S)R₆₇, CO₂R₆₈,
C₁-C₃alkyl optionally substituted with any combination of one or two halogen atoms, one or two C₁-C₃alkoxy groups, one or two C₁-C₃haloalkoxy groups, one SO₂R₇₂ group, one or two cyano groups, one C₃-C₅cycloalkyl group, one OSO₂R₇₃ group, one or two OR₇₉ groups, one P(O)(OR₈₀)₂ group, one 1,3-dioxolane group or one 1,3-dioxane group, or
phenyl optionally substituted with any combination of one halogen atom, one or two methyl groups, one methoxy group, one halomethyl group or one OR₈₃ group;
R₆₆, R₆₇, R₈₅ and R₈₉ are each independently hydrogen, C₁-C₄alkyl or NR₅₆R₅₇;
R₅₆ is SO₂R₄₉;
R₅₇ is hydrogen or C₁-C₄alkyl;
R₄₉ and R₈₆ are each independently C₁-C₄alkyl or NR₉₃R₉₄;
R₉₃ and R₉₄ are each independently hydrogen or C₁-C₈alkyl;
R₆₈ and R₈₈ are each independently hydrogen, C₃-C₆alkenyl or C₁-C₄alkyl optionally substituted with CO₂R₅₄, morpholine or C(O)R₅₅;
R₅₄, R₅₅, R₆₀, R₆₁, R₇₃ and R₈₇ are each independently hydrogen, C₁-C₄alkyl or C₁-C₄haloalkyl;
R₇₂ is NR₆₀R₆₁ or indazole;
R₇₉ and R₈₃ are each independently hydrogen C(O)R₈₅, SO₂R₈₆, C₁-C₄haloalkyl, C₃-C₄alkenyl or C₁-C₃alkyl substituted with one OC(O)R₈₇, CO₂R₈₈, C(O)R₈₉, C(OR₉₀)₂ or cyano group;
R₈₀ is hydrogen or methyl; and
R₉₀ is C₁-C₄alkyl.

## Patentansprüche

1. Verfahren zur Herstellung einer 6-(Perfluoralkyl)-uracil-Verbindung der Strukturformel I, 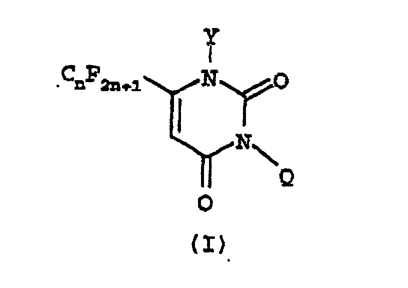 in der
n eine ganze Zahl 1, 2, 3, 4, 5 oder 6 bedeutet,
y Wasserstoff oder C₁-C₆-Alkyl bedeutet und
Q eine C₁-C₆-Alkylgruppe oder eine gegebenenfalls substituierte Phenyl-, Benzyl-, Heteroaryl- oder Methylenheteroarylgruppe bedeutet,
**dadurch gekennzeichnet, daß** man
(a) eine Carbamatverbindung der Strukturformel II, 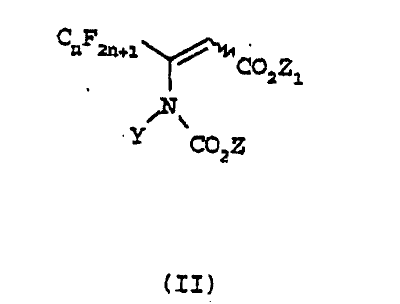 in der
Z und Z₁ jeweils unabhängig C₁-C₆-Alkyl oder Benzyl, das am Phenylring gegebenenfalls durch eine beliebige Kombination aus ein bis drei Halogen-, C₁-C₄-Alkyl- oder C₁-C₄-Halogenalkylgruppen substituiert ist, bedeuten und
n und Y wie oben angegeben sind,
mit einer Aminverbindung der Strukturformel III,
QNH₂ (III)
in der Q wie oben angegeben ist, in Gegenwart einer Base umsetzt, wodurch man zu der 6-(Perfluoralkyl)uracil-Verbindung der Formel I, in der Y Wasserstoff oder C₁-C₆-Alkyl bedeutet, gelangt, und
(b) gegebenenfalls die Verbindung der Formel I, in der Y Wasserstoff bedeutet, alkyliert, wodurch man zu der Verbindung der Formel I, in der Y C₁-C₄-Alkyl bedeutet, gelangt.

2. Verfahren nach Anspruch 1, wobei die Doppelbindung in der Verbindung der Formel II hauptsächlich in (Z)-Konfiguration vorliegt.

3. Verfahren nach Anspruch 1, wobei die Base aus der Gruppe Tri(C₁-C₆-alkyl)amin, heterocyclisches tertiäres Amin und Alkalimetall-C₁-C₆-alkoholat stammt.

4. Verfahren nach Anspruch 3, wobei die Base aus der Gruppe 1,8-Diazabicyclo[5.4.0]undec-7-en und 1,5-Diazabicyclo[4.3.0]non-5-en stammt.

5. Verfahren nach Anspruch 1, wobei die Carbamatverbindung mit der Aminverbindung in Gegenwart eines Lösungsmittels umgesetzt wird.

6. Verfahren nach Anspruch 5, wobei das Lösungsmittel aus der Gruppe Carbonsäureamid, Dialkylsulfoxid, aromatischer Kohlenwasserstoff, halogenierter aromatischer Kohlenwasserstoff, aliphatischer Kohlenwasserstoff, halogenierter aliphatischer Kohlenwasserstoff, Alkohol, Keton, Ether, Nitril und Wasser sowie deren Mischungen stammt.

7. Verfahren nach Anspruch 1, wobei die Carbamatverbindung mit der Aminverbindung und der Base bei einer Temperatur von ungefähr 20°C bis 150°C umgesetzt wird.

8. Verfahren nach Anspruch 1, wobei der Schritt (b) **dadurch gekennzeichnet ist, daß** man die Verbindung der Formel I, in der Y Wasserstoff bedeutet, mit einem Alkylhalogenid der Strukturformel IV oder einem Dialkylsulfatester der Strukturformel V
XY (IV)
oder 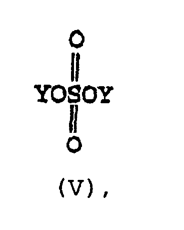 in der X Chlor, Brom oder Iod bedeutet und Y C₁-C₆-Alkyl bedeutet, in Gegenwart einer Base umsetzt.

9. Verfahren nach Anspruch 1, wobei
n 1 bedeutet,
Y Wasserstoff oder C₁-C₄-Alkyl bedeutet,
Q 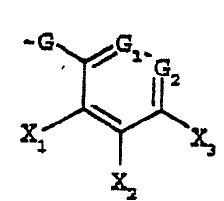 bedeutet,
G CH₂ oder eine Bindung bedeutet,
G₁ CX₅ oder N bedeutet,
G₂ CX₄ oder N bedeutet,
X₁ Wasserstoff, Halogen oder eine C₁-C₆-Alkylgruppe, die gegebenenfalls durch eine Epoxygruppe substituiert ist, bedeutet,
X₂ Wasserstoff, Halogen NRR₁, CO₂R₂, C(O)R₃, OR₄, SO₂R₅, SO₂NR₆R₇, C(R₈)(OR₉)₂, C(R₁₀)=NOR₁₁, C(R₁₂) =C(R₁₃) -C (OR₁₄) =NOR₁₅, CH₂O-NCO₂R₁₆,
1,3-Dioxolan, das gegebenenfalls durch eine C₁-C₆-Alkoxygruppe oder ein oder zwei C₁-C₄-Alkylgruppen substituiert ist,
1,3-Dioxolinon, das gegebenenfalls durch eine C₁-C₆-Alkoxygruppe oder eine oder zwei C₁-C₄-Alkylgruppen substituiert ist, oder
C₁-C₄-Alkyl, das gegebenenfalls durch eine CO₂R₂-Gruppe und ein Halogenatom substituiert ist, bedeutet, und
X₃ Wasserstoff, Halogen, C₁-C₄-Halogenalkyl, CO₂R₁₇, Cyano, C₁-C₄-Halogenalkoxy, OR₁₈ oder C₁-C₄-Alkyl bedeutet, oder,
X₁ und X₂ gemeinsam mit den Atomen, an die sie gebunden sind, einen fünf- oder sechsgliedrigen Ring bilden können, in dem X₁X₂ bzw. X₂X₁ -OC(R₂₀)(R₂₁)O-, -CH₂S(O)ₚN(R₂₂)-, -SC(R₂₃)=N-, -CH=CH-CH(R₁₁)O-, -OC(O)N-, -SC (R₂₄)=N-, -ON(R₂₅)C(O)-, -OC(CO₂R₂₆)=(C(R₂₇)-, -NC(R₂₈)=C(SR₂₉)-, -CH=C(CO₂R₃₀)O-, -CH₂CH (R₃₁)O- oder -OC(R₃₂) (R₃₃)C(O)- bedeutet, oder
X₂ und X₃ gemeinsam mit den Atomen, an die sie gebunden sind, einen fünf- oder sechsgliedrigen Ring bilden können, in dem X₂X₃ bzw. X₃X₂ -NC(R₃₄)=NC(S)-, -N(R₃₅)N=C(R₃₆)-, -N(R₃₇)C(R₃₈)=N-, -N(R₃₈)C(O)CH₂O-, -N(R₃₉)C(O)CH=CH-, -S-N=C(R₄₀)-, -O-N=C(R₄₁)-, -N=N-N(R₄₂)-, -C(R₄₃)(R₄₄)C(O)N(R₄₅)- oder -N(R₄₆)C(O)C(R₄₇)(R₄₈)- bedeutet,
X₄ Wasserstoff, Halogen oder OR₁₉ bedeutet,
X₅ Wasserstoff oder Halogen bedeutet,
R, R₅₆, R₆₄, R₆₉, R₇₀, R₇₇ und R₉₁ jeweils unabhängig Wasserstoff, SO₂R₄₉, C₁-C₄-Alkyl, C₃-C₇-Cycloalkyl, C₃-C₆-Alkenyl, C₃-C₆-Alkinyl, Phenyl oder Benzyl bedeuten,
R₁ Wasserstoff, SO₂R₅₀, C(O)R₅₁, Amino oder C₁-C₄-Alkyl, das gegebenenfalls durch CO₂R₅₂ oder C(O)R₅₃ substituiert ist, bedeutet,
R₂, R₁₆, R₁₇, R₂₆, R₃₀, R₆₈, R₇₅, R₇₆, R₈₂ und R₈₈ jeweils unabhängig Wasserstoff, C₁-C₈-Halogenalkyl, C₃-C₈-Alkenyl, C₃-C₆-Alkinyl, Phenyl, Benzyl, Furfuryl, Pyridyl, Thienyl, C₁-C₈-Alkyl, das gegebenenfalls durch CO₂R₅₄, Morpholin oder C(O)R₅₅ substituiert ist, oder ein Alkalimetall, ein Erdalkalimetall, Ammoniumkation oder organisches Ammoniumkation bedeuten,
R₃, R₆₆, R₆₇, R₈₁, R₈₅ und R₈₉ jeweils unabhängig Wasserstoff, C₁-C₆-Alkyl, C₃-C₆-Alkenyl, C₃-C₆-Alkinyl, NR₅₆R₅₇, Phenyl oder Benzyl bedeuten,
R₄, R₁₈, R₁₉ und R₆₅ jeweils unabhängig Wasserstoff, C₁-C₆-Alkyl, C₃-C₆-Alkenyl, C₃-C₆-Alkinyl, C₁-C₄-Halogenalkyl, C(O)R₅₈, C(S)R₅₉ oder Benzyl bedeuten,
R₅ und R₇₂ jeweils unabhängig C₁-C₆-Alkyl, C₁-C₆-Halogenalkyl, NR₆₀R₆₁, Imidazol oder Indazol bedeuten,
R₆, R₁₁, R₁₂, R₁₄, R₁₅, R₂₀, R₂₁, R₂₂, R₂₅, R₂₈, R₂₉, R₃₁, R₃₂, R₃₃, R₃₅, R₄₅, R₄₆, R₆₃ und R₈₀ jeweils unabhängig Wasserstoff oder C₁-C₄-Alkyl bedeuten,
R₇ Wasserstoff, C₃-C₆-Alkenyl, C₃-C₆-Alkinyl, Benzyl oder C₁-C₄-Alkyl, das gegebenenfalls durch Cyano oder C(O)R₆₂ substituiert ist, bedeutet,
R₈ und R₂₇ jeweils unabhängig Wasserstoff, C₁-C₄-Alkyl oder C₁-C₄-Alkoxy bedeuten,
R₉ und R₉₀ jeweils unabhängig C₁-C₆-Alkyl bedeuten,
R₁₀ Wasserstoff, C₁-C₆-Alkyl, Phenyl oder Benzyl bedeutet,
R₁₃, R₂₄ und R₃₆ jeweils unabhängig Wasserstoff, C₁-C₆-Alkyl oder Halogen bedeuten,
R₂₃ Wasserstoff oder NR₆₃R₆₄ bedeutet,
R₃₄ Wasserstoff, C₁-C₄-Alkyl oder C₁-C₄-Halogenalkyl bedeutet,
R₃₇ Wasserstoff, C₁-C₄-Alkyl oder C₂-C₈-Alkoxyalkyl bedeutet,
R₃₈ und R₃₉ jeweils unabhängig Wasserstoff, C₁-C₄-Alkyl, C₁-C₄-Halogenalkyl, C₃-C₆-Alkenyl oder C₃-C₆-Alkinyl bedeuten,
R₄₀, R₄₁ und R₄₂ jeweils unabhängig Wasserstoff, Halogen, Cyano, OR₆₅, C(O)R₆₆, C(S)R₆₇, CO₂R₆₈, C(=NOR₆₉),
eine C₁-C₈-Alkyl-, C₃-C₇-Cycloalkyl-, C₂-C₈-Alkenyl- oder C₂-C₈-Alkinylgruppe, in der jede Gruppe gegebenenfalls durch eine beliebige Kombination aus einem bis sechs Halogenatomen, einer bis drei C₁-C₁₀-Alkoxygruppen, einer oder zwei C₁-C₆-Halogenalkoxygruppen, einer oder zwei NR₇₀R₇₁-Gruppen, einer oder zwei S(O)_{q}R₇₂-Gruppen, einer oder zwei Cyanogruppen, einer oder zwei C₃-C₇-Gruppen, einer OSO₂R₇₃-Gruppe, einer oder zwei C(O)R₇₄-Gruppen, einer oder zwei CO₂R₇₅-Gruppen, einer oder zwei C (O)SR₇₆-Gruppen, einer oder zwei C(O)NR₇₇R₇₈-Gruppen, einer bis drei OR₇₉-Gruppen, einer oder zwei P(O)(OR₈₀)₂-Gruppen, einem 1,3-Dioxolan, das gegebenenfalls durch eine bis drei C₁-C₄-Alkylgruppen substituiert ist, oder einem 1,3-Dioxan, das gegebenenfalls durch ein bis drei C₁-C₄-Alkylgruppen substituiert ist, substituiert ist, oder
Phenyl oder Benzyl, die gegebenenfalls durch eine Kombination aus einem bis drei Halogenatomen, einer bis drei C₁-C₆-Alkylgruppen, einer bis drei C₁-C₆-Alkoxygruppen, einer C₃-C₇-Cycloalkylgruppe, einer C₁-C₄-Halogenalkylgruppe, einer C₁-C₄-Alkylthiogruppe, einer Cyanogruppe, einer Nitrogruppe, einer C(O)R₈₁-Gruppe, einer CO₂R₈₂-Gruppe, einer OR₈₃-Gruppe, einer SR₈₄-Gruppe, einer C₁-C₆-Alkoxymethylgruppe, einer Hydroxymethylgruppe, einer C₃-C₈-Alkenyloxymethylgruppe oder einer C₁-C₈-Halogenalkoxymethylgruppe substituiert ist, bedeuten,
R₄₃, R₄₄, R₄₇ und R₄₈ jeweils unabhängig Wasserstoff, C₁-C₄-Alkyl, C₁-C₄-Halogenalkyl, C₃-C₆-Alkenyl, C₃-C₆-Alkinyl oder C₃-C₇-Cycloalkyl bedeuten oder R₄₃ und R₄₄ bzw. R₄₇ und R₄₈ gemeinsam mit dem Atom, an das sie gebunden sind, eine C₃-C₇-Cycloalkylgruppe bilden können,
R₄₉, R₅₀ und R₈₆ jeweils unabhängig C₁-C₆-Alkyl, NR₉₃R₉₄, C₁-C₄-Halogenalkyl, C₃-C₆-Alkenyl, C₃-C₆-Alkinyl oder Benzyl bedeuten,
R₅₁, R₅₂, R₅₃, R₅₄, R₅₅, R₅₇, R₅₈, R₅₉, R₆₀, R₆₁, R₆₂, R₇₁, R₇₃, R₇₄, R₇₈, R₈₇ und R₉₂ jeweils unabhängig Wasserstoff, C₁-C₆-Alkyl, C₃-C₇-Cycloalkyl, C₁-C₆-Halogenalkyl, C₃-C₆-Alkenyl, C₃-C₆-Alkinyl, Phenyl oder Benzyl bedeuten,
R₇₉, R₈₃ und R₈₄ jeweils unabhängig Wasserstoff, C(O)R₈₅, SO₂R₈₆, C₁-C₆-Halogenalkyl, C₂-C₆-Alkenyl, C₅-C₈-Cycloalkenyl, C₂-C₆-Alkinyl, Phenyl, Benzyl oder C₁-C₁₀-Alkyl, das gegebenenfalls durch eine Hydroxy-, Benzyloxy-, OC(O)R₈₇-, C₁-C₆-Alkoxy-, CO₂R₈₈-, C(O)R₈₉-, C(OR₉₀)₂-, C(O)NR₉₁R₉₂- oder Cyanogruppe substituiert ist, bedeuten,
R₉₃ und R₉₄ jeweils unabhängig Wasserstoff, C₁-C₄-Halogenalkyl, C₂-C₆-Alkenyl, C₃-C₈-Cycloalkyl, C₁-C₈-Alkyl, das gegebenenfalls durch eine oder zwei C₁-C₄-Alkoxygruppen oder eine Cyanoalkylgruppe substituiert ist, oder
Benzyl oder Phenyl, die gegebenenfalls durch eine beliebige Kombination aus einem bis drei Halogenatomen, einer bis drei C₁-C₄-Alkylgruppen, einer bis drei C₁-C₄-Halogenalkylgruppen, einer bis drei C₁-C₄-Alkoxygruppen, einer bis drei C₁-C₄-Halogenalkoxygruppen, einer Cyanogruppe oder einer Nitrogruppe substituiert sind, bedeuten, und
R₉₃ und R₉₄ gemeinsam mit dem Atom, an das sie gebunden sind, einen 5- bis 12gliedrigen monocyclischen oder anellierten bicyclischen Heterocyclus bilden, der gegebenenfalls durch eine oder mehrere Gruppen, die unabhängig aus der Reihe Halogen-, Cyano-, Nitro-, Amino-, Hydroxy-, C₁-C₄-Alkyl-, C₁-C₄-Halogenalkyl-, C₁-C₄-Alkoxy-, C₁-C₄-Halogenalkoxy- und C₁-C₄-Halogenalkylsulfonylgruppen ausgewählt sind, substituiert ist,
p und q jeweils unabhängig 0, 1 oder 2 bedeuten und
Z und Z₁ jeweils unabhängig C₁-C₄-Alkyl bedeuten.

10. Verfahren nach Anspruch 9, wobei
Y Wasserstoff oder Methyl bedeutet,
X₁ Wasserstoff, Fluor oder C₁-C₃-Alkyl, das gegebenenfalls durch eine Epoxygruppe substituiert ist, bedeutet,
X₂ Wasserstoff, Halogen NRR₁, CO₂R₂, C(O)R₃, OR₄, SO₂R₅, SO₂NR₆R₇, C(R₈)(OR₉)₂, C(R₁₀)=NOR₁₁, C(R₁₂)=C(R₁₃)-C(OR₁₄)=NOR₁₅, CH₂O-NCO₂R₁₆,
1,3-Dioxolan, das gegebenenfalls durch eine C₁-C₆-Alkoxygruppe oder eine oder zwei C₁-C₄-Alkylgruppen substituiert ist,
1,3-Dioxolinon, das gegebenenfalls durch eine C₁-C₆-Alkoxygruppe oder eine oder zwei C₁-C₄-Alkylgruppen substituiert ist, oder
C₁-C₄-Alkyl, das gegebenenfalls durch eine CO₂R₂-Gruppe und ein Halogenatom substituiert ist, bedeutet, und
X₃ Wasserstoff, Halogen, C₁-C₄-Halogenalkyl, Co₂R₁₇, Cyano, C₁-C₄-Halogenalkoxy, OR₁₈ oder C₁-C₄-Alkyl bedeutet, oder,
X₁ und X₂ gemeinsam mit den Atomen, an die sie gebunden sind, einen fünf- oder sechsgliedrigen Ring bilden können, in dem X₁X₂ bzw. X₂X₁ -OC(R₂₀)(R₂₁)O-, -CH₂S(O)ₚN(R₂₂)-, -SC(R₂₃)=N-, -CH=CH-CH(R₁₁)O-, -OC(O)N-, -SC(R₂₄)=N-, -ON(R₂₅)C(O)-, -OC(CO₂R₂₆)=CH-, -NC(R₂₈)=C(SR₂₉)-, -CH=C(CO₂R₃₀)O-, -CH₂CH(R₃₁)O- oder -OC(R₃₂)(R₃₃)C(O)- bedeutet, oder
X₂ und X₃ gemeinsam mit den Atomen, an die sie gebunden sind, einen fünf- oder sechsgliedrigen Ring bilden können, in dem X₂X₃ bzw. X₃X₂ -NC(R₃₄)=NC(S)-, -N(R₃₅)N=C(R₃₆)-, - N (R₃₇)C(R₃₈)=N-, -N (R₃₈)C(O)CH₂O-, -N(R₃₉)C(O)CH=CH-, -S-N=C(R₄₀)-, -O-N=C(R₄₁)-, -N=N-N(R₄₂)-, -C(R₄₃)(R₄₄)C(O)N(R₄₅)- oder -N(R₄₆)C(O)C(R₄₇)(R₄₈)- bedeutet,
X₄ Wasserstoff, Halogen oder OR₁₉ bedeutet,
X₅ Wasserstoff oder Halogen bedeutet,
R, R₆₄, R₆₉ und R₇₇ jeweils unabhängig Wasserstoff, SO₂R₄₉ oder C₁-C₄-Alkyl bedeuten,
R₁ Wasserstoff, SO₂R₅₀, C(O)R₅₁, Amino oder C₁-C₄-Alkyl, das gegebenenfalls durch CO₂R₅₂ oder C(O)R₅₃ substituiert ist, bedeutet,
R₂, R₁₆, R₁₇, R₂₆, R₃₀, R₆₈, R₇₅, R₇₆, R₈₂ und R₈₈ jeweils unabhängig Wasserstoff, C₃-C₆-Alkenyl oder C₁-C₄-Alkyl, das gegebenenfalls durch CO₂R₅₄, Morpholin oder C(O)R₅₅ substituiert ist, bedeuten,
R₃, R₆₆, R₆₇, R₈₅ und R₈₉ jeweils unabhängig Wasserstoff, C₁-C₄-Alkyl oder NR₅₆R₅₇ bedeuten,
R₄, R₁₈ und R₁₉ jeweils unabhängig Wasserstoff, C₁-C₄-Alkyl, C₁-C₄-Halogenalkyl, C(O)R₅₈, C₃-C₄-Alkenyl oder C₃-C₄-Alkinyl bedeuten,
R₅₆ SO₂R₄₉ bedeutet,
R₅₇ Wasserstoff oder C₁-C₄-Alkyl bedeutet,
R₅ und R₇₂ jeweils unabhängig NR₆₀R₆₁ oder Indazol bedeuten,
R₆, R₁₁, R₁₂, R₁₄, R₁₅, R₂₀, R₂₁, R₂₂, R₂₅, R₂₈, R₂₉, R₃₁, R₃₂, R₃₃, R₃₅, R₄₅, R₄₆ und R₈₀ jeweils unabhängig Wasserstoff oder Methyl bedeuten,
R₇ C₁-C₄-Alkyl, das gegebenenfalls durch Cyano oder C(O)R₆₂ substituiert ist, bedeutet,
R₈ Wasserstoff oder C₁-C₄-Alkoxy bedeutet,
R₉ und R₉₀ jeweils unabhängig C₁-C₄-Alkyl bedeuten,
R₁₀ Wasserstoff oder C₁-C₃-Alkyl bedeutet,
R₁₃, R₂₄ und R₃₆ jeweils unabhängig Wasserstoff oder Chlor bedeuten,
R₂₃ NR₆₃R₆₄ bedeutet,
R₃₄ C₁-C₃-Halogenalkyl bedeutet,
R₃₇ C₂-C₄-Alkoxyalkyl bedeutet,
R₃₈ und R₃₉ jeweils unabhängig C₁-C₃-Halogenalkyl, C₁-C₃-Alkyl oder Propargyl bedeuten,
R₄₀, R₄₁ und R₄₂ jeweils unabhängig Wasserstoff, C(O)R₆₆, C(S)R₆₇, CO₂R₆₈, C(=NOR₆₉),
C₁-C₃-Alkyl, das gegebenenfalls durch eine beliebige Kombination aus einem oder zwei Halogenatomen, einer oder zwei C₁-C₃-Alkoxygruppen, einer oder zwei C₁-C₃-Halogenalkoxygruppen, einer SO₂R₇₂-Gruppe, einer oder zwei Cyanogruppen, einer C₃-C₅-Cycloalkylgruppe, einer OSO₂R₇₃-Gruppe, einer C(O)R₇₄-Gruppe, einer CO₂R₇₅-Gruppe, einer C(O)SR₇₆-Gruppe, einer C(O)NR₇₇R₇₈-Gruppe, einer oder zwei OR₇₉-Gruppen, einer P(O)(OR₈₀)₂-Gruppe, einer 1,3-Dioxolangruppe oder einer 1,3-Dioxangruppe substituiert ist, oder
Phenyl, das gegebenenfalls durch eine beliebige Kombination aus einem Halogenatom, einer oder zwei Methylgruppen, einer Methoxygruppe, einer Halogenmethylgruppe oder einer OR₈₃-Gruppe substituiert ist, bedeuten,
R₄₃, R₄₄, R₄₇ und R₄₈ jeweils unabhängig Wasserstoff oder Methyl bedeuten, oder R₄₃ und R₄₄ bzw. R₄₇ und R₄₈ gemeinsam mit dem Atom, an das sie gebunden sind, eine Cyclopropylgruppe bilden können,
R₄₉, R₅₀ und R₈₆ jeweils unabhängig C₁-C₄-Alkyl oder NR₉₃R₉₄ bedeuten,
R₅₁, R₅₂, R₅₃, R₅₄, R₅₅, R₅₈, R₆₀, R₆₁, R₆₂, R₇₃, R₇₄, R₇₈ und R₈₇ jeweils unabhängig Wasserstoff, C₁-C₄-Alkyl oder C₁-C₄-Halogenalkyl bedeuten,
R₇₉ und R₈₃ jeweils unabhängig Wasserstoff, C(O)R₈₅, SO₂R₈₆, C₁-C₄-Halogenalkyl, C₃-C₄-Alkenyl oder C₁-C₃-Alkyl, das durch eine OC(O)R₈₇-, CO₂R₈₈-, C(O)R₈₉-, C(OR₉₀)₂- oder Cyanogruppe substituiert ist, bedeuten,
R₉₃ und R₉₄ jeweils unabhängig Wasserstoff oder C₁-C₈-Alkyl bedeuten,
p 0, 1 oder 1 bedeutet und
Z und Z₁ jeweils unabhängig Methyl oder Ethyl bedeuten.

11. Verfahren nach Anspruch 1 zur Herstellung einer 6-(Trifluormethyl)uracil-Verbindung der Struktur- formel VI 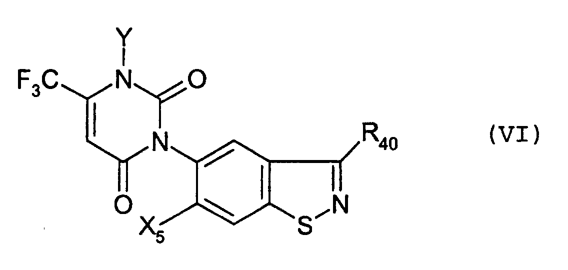 in der
Y Wasserstoff oder Methyl bedeutet,
X₅ Wasserstoff oder Halogen bedeutet,
R₄₀ Wasserstoff, C(O)R₆₆, C(S)R₆₇, CO₂R₆₈,
C₁-C₃-Alkyl, das gegebenenfalls durch eine beliebige Kombination aus einem oder zwei Halogenatomen, einer oder zwei C₁-C₃-Alkoxygruppen, einer oder zwei C₁-C₃-Halogenalkoxygruppen, einer SO₂R₇₂-Gruppe, einer oder zwei Cyanogruppen, einer C₃-C₅-Cycloalkylgruppe, einer OSO₂R₇₃-Gruppe, einer oder zwei OR₇₉-Gruppe, einer P(O)(OR₈₀)₂-Gruppe, einer 1,3-Dioxolangruppe oder einer 1,3-Dioxangruppe substituiert ist, oder
Phenyl, das gegebenenfalls durch eine beliebige Kombination aus einem Halogenatom, einer oder zwei Methylgruppen, einer Methoxygruppe, einer Halogenmethylgruppe oder einer OR₈₃-Gruppe substituiert ist, bedeutet,
R₆₆, R₆₇, R₈₅ und R₈₉ jeweils unabhängig Wasserstoff, C₁-C₄-Alkyl oder NR₅₆R₅₇ bedeuten,
R₅₆ SO₂R₄₉ bedeutet,
R₅₇ Wasserstoff oder C₁-C₄-Alkyl bedeutet,
R₄₉ und R₈₆ jeweils unabhängig C₁-C₄-Alkyl oder NR₉₃R₉₄ bedeuten,
R₉₃ und R₉₄ jeweils unabhängig Wasserstoff oder C₁-C₈-Alkyl bedeuten,
R₆₈ und R₈₈ jeweils unabhängig Wasserstoff, C₃-C₆-Alkenyl oder C₁-C₄-Alkyl, das gegebenenfalls durch CO₂R₅₄, Morpholin oder C(O)R₅₅ substituiert ist, bedeuten,
R₅₄, R₅₅, R₆₀, R₆₁, R₇₃ und R₈₇ jeweils unabhängig Wasserstoff, C₁-C₄-Alkyl oder C₁-C₄-Halogenalkyl bedeuten,
R₇₂ NR₆₀R₆₁ oder Indazol bedeutet,
R₇₉ und R₈₃ jeweils unabhängig Wasserstoff C(O)R₈₅, SO₂R₈₆, C₁-C₄-Halogenalkyl, C₃-C₄-Alkenyl oder C₁-C₃-Alkyl, das durch eine OC(O)R₈₇-, CO₂R₈₈-, C(O)R₈₉-, C(OR₉₀)₂- oder Cyanogruppe substituiert ist, bedeuten,
R₈₀ Wasserstoff oder Methyl bedeutet und
R₉₀ C₁-C₄-Alkyl bedeutet.

## Revendications

1. Procédé de préparation d'un composé 6-(perfluoroalkyl)uracile ayant la formule de structure I : 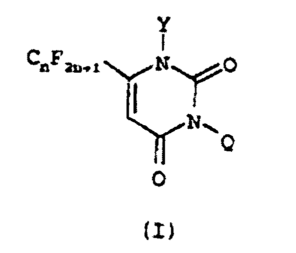 dans laquelle :
n représente un nombre entier équivalent à 1, 2, 3, 4, 5 ou 6 ;
Y représente un atome d'hydrogène ou un groupe alkyle en (C₁-C₆) ; et
Q représente un groupe alkyle en (C₁-C₆) ou un groupe phényle, un groupe benzyle, un groupe hétéroaryle ou un groupe méthylènehétéroaryle éventuellement substitué,
procédé qui comprend :
(a) la réaction d'un composé carbamate ayant la formule de structure II : 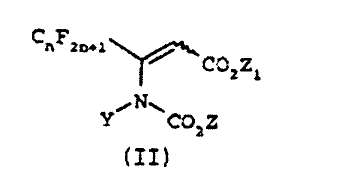 dans laquelle :
Z et Z₁ représentent chacun indépendamment un groupe alkyle en (C₁-C₆) ou un groupe benzyle éventuellement substitué sur le cycle phényle par une quelconque combinaison de un à trois atomes d'halogènes, groupes alkyle en (C₁-C₄) ou groupes halogénoalkyle en (C₁-C₄) ; et
n et Y sont tels que décrits ci-dessus,
avec un composé amine ayant la formule de structure III :
QNH₂ (III)
dans laquelle Q est tel que décrit ci-dessus, en présence d'une base, pour former le composé 6-(perfluoroalkyl)uracile de formule I, dans laquelle Y représente un atome d'hydrogène ou un groupe alkyle en (C₁-C₆) ; et
(b) l'alkylation éventuelle du composé de formule I, dans lequel Y représente un atome d'hydrogène pour former le composé de formule I dans lequel Y représente un groupe alkyle en (C₁-C₄).

2. Procédé selon la revendication 1, dans lequel la double liaison du composé de formule II est de façon prédominante dans la configuration (Z).

3. Procédé selon la revendication 1, dans lequel la base est choisie dans le groupe comprenant une tri(alkyle en (C₁-C₆))amine, une amine tertiaire hétérocyclique et un alcoxyde en (C₁-C₆) de métal alcalin.

4. Procédé selon la revendication 3, dans lequel la base est choisie dans le groupe comprenant le 1,8-diazabicyclo[5-4-0]undec-7-ène et le 1,5-diazabicyclo[4-3-0]non-5-ène.

5. Procédé selon la revendication 1, dans lequel on fait réagir le composé carbamate avec le composé amine en présence d'un solvant.

6. Procédé selon la revendication 5, dans lequel le solvant est choisi dans le groupe comprenant un amide d'acide carboxylique, un sulfoxyde dialkylique, un hydrocarbure aromatique, un hydrocarbure aromatique halogéné, un hydrocarbure aliphatique, un hydrocarbure aliphatique halogéné, un alcool, une cétone, un éther, un nitrile, de l'eau et des mélanges de ceux-ci.

7. Procédé selon la revendication 1, dans lequel on fait réagir le composé carbamate avec le composé amine et la base à une température d'environ 20° C à 150° C.

8. Procédé selon la revendication 1, dans lequel l'étape (b) comprend la réaction du composé de formule I, dans laquelle Y représente un atome d'hydrogène, avec un halogénure d'alkyle ayant la formule de structure IV ou un ester de dialkylsulfate ayant la formule de structure V :
XY (IV)
ou 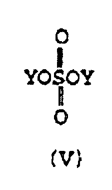 dans laquelle X représente un atome de chlore, de brome ou d'iode, et Y représente un groupe alkyle en (C₁-C₆), en présence d'une base.

9. Procédé selon la revendication 1, dans lequel :
n représente 1 ;
Y représente un atome d'hydrogène ou un groupe alkyle en (C₁-C₄) ;
Q représente 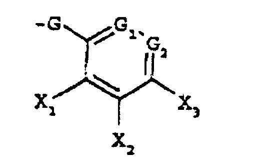
G représente un groupe CH₂ ou une liaison ;
G₁ représente un groupe CX₅ ou un atome d'azote ;
G₂ représente un groupe CX₄ ou un atome d'azote ;
X₁ représente un atome d'hydrogène, un atome d'halogène ou un groupe alkyle en (C₁-C₆) éventuellement substitué par un groupe époxy ,
X₂ représente un atome d'hydrogène, un groupe NRR₁ halogéné, un groupe CO₂R₂, un groupe C(O)R₃, un groupe OR₄, un groupe SO₂R₅, un groupe SO₂NR₆R₇, un groupe C(R₈)(OR₉)₂, un groupe C(R₁₀)=NOR₁₁, un groupe C(R₁₂)=C(R₁₃)-C(OR₁₄)=NOR₁₅, un groupe CH₂O-NCO₂R₁₆,
un groupe 1,3-dioxolane éventuellement substitué par un groupe alcoxy en (C₁-C₆) ou un ou deux groupes alkyle en (C₁-C₄),
un groupe 1,3-dioxolinone éventuellement substitué par un groupe alcoxy en (C₁-C₆) ou un ou deux groupes alkyle en (C₁-C₄),
ou un groupe alkyle en (C₁-C₄) éventuellement substitué par un groupe CO₂R₂ et un atome d'halogène, et
X₃ représente un atome d'hydrogène, un atome d'halogène, un groupe halogénoalkyle en (C₁-C₄), un groupe CO₂R₁₇, un groupe cyano, un groupe halogénoalcoxy en (C₁-C₄), un groupe OR₁₈ ou un groupe alkyle en (C₁-C₄), ou
lorsque X₁ et X₂ sont pris ensemble avec les atomes auxquels ils sont attachés, ils peuvent former un cycle de cinq ou six membres dans lequel X₁X₂ ou X₂X₁ est représenté par : -OC(R₂₀)(R₂₁)O-, -CH₂S(O)_{P}N(R₂₂)-, -SC(R₂₃)=N-, -CH=CH-CH(R₁₁)O-, -OC(O)N-, -SC(R₂₄)=N-, -ON(R₂₅)C(O)-, -OC(CO₂R₂₆)=C(R₂₇)-, -NC(R₂₈)=C(SR₂₉)-, -CH=C(CO₂R₃₀)O-, -CH₂CH(R₃₁)O- ou -OC(R₃₂)(R₃₃)C(O)-, ou
lorsque X₂ et X₃ sont pris ensemble avec les atomes auxquels ils sont attachés, ils peuvent former un cycle de cinq ou six membres dans lequel X₂X₃ ou X₃X₂ est représenté par : -NC(R₃₄)=NC(S)-, -N(R₃₅)N=C(R₃₆)-, -N(R₃₇)C(R₃₈)=N-, -N(R₃₈)C(O)CH₂O-, -N(R₃₉C(O)CH=CH-, -S-N=C(R₄₀)-, -O-N=C(R₄₁)- , -N=N-N(R₄₂)-, -C(R₄₃)(R₄₄)C(O)N(R₄₅)- ou -N(R₄₆)C(O)C(R₄₇)(R₄₈)- ;
X₄ représente un atome d'hydrogène, un atome d'halogène ou un groupe OR₁₉ ;
X₅ représente un atome d'hydrogène ou d'halogène ;
R, R₅₆, R₆₄, R₆₉, R₇₀, R₇₇ et R₉₁ représentent chacun indépendamment un atome d'hydrogène, un groupe SO₂R₄₉, un groupe alkyle en (C₁-C₄), un groupe cycloalkyle en (C₃-C₇), un groupe alcényle en (C₃-C₆), un groupe alcynyle en (C₃-C₆), un groupe phényle ou un groupe benzyle ;
R₁ représente un atome d'hydrogène, un groupe SO₂R₅₀, un groupe C(O)R₅₁, un groupe amino ou un groupe alkyle en (C₁-C₄) éventuellement substitué par un groupe CO₂R₅₂ ou C(O)R₅₃ ;
R₂, R₁₆, R₁₇, R₂₆, R₃₀, R₆₈, R₇₅, R₇₆, R₈₂ et R₈₈ représentent chacun indépendamment un atome d'hydrogène, un groupe halogénoalkyle en (C₁-C₈), un groupe alcényle en (C₃-C₈), un groupe alcynyle en (C₃-C₆), un groupe phényle, un groupe benzyle, un groupe furfuryle, un groupe pyridyle, un groupe thiényle,
un groupe alkyle en(C₁-C₈) éventuellement substitué par un groupe CO₂R₅₄, un groupe morpholine ou un groupe C(O)R₅₅, ou
un métal alcalin, un métal alcalino-terreux, un cation d'ammonium ou d'ammonium organique ;
R₃, R₆₆, R₆₇, R₈₁, R₈₅ et R₈₉ représentent chacun indépendamment un atome d'hydrogène, un groupe alkyle en (C₁-C₆), un groupe alcényle en (C₃-C₆), un groupe alcynyle en (C₃-C₆), un groupe NR₅₆R₅₇, un groupe phényle ou un groupe benzyle ;
R₄, R₁₈, R₁₉ et R₆₅ représentent chacun indépendamment un atome d'hydrogène, un groupe alkyle en (C₁-C₆), un groupe alcényle en (C₃-C₆), un groupe alcynyle en (C₃-C₆), un groupe halogénoalkyle en (C₁-C₄), un groupe C(O)R₅₈, un groupe C(S)R₅₉ ou un groupe benzyle ;
R₅ et R₇₂ représentent chacun indépendamment un groupe alkyle en (C₁-C₆), un groupe halogénoalkyle en (C₁-C₆), un groupe NR₆₀R₆₁, un groupe imidazole ou un groupe indazole ;
R₆, R₁₁, R₁₂, R₁₄, R₁₅, R₂₀, R₂₁, R₂₂, R₂₅, R₂₈, R₂₉, R₃₁, R₃₂, R₃₃, R₃₅, R₄₅, R₄₆, R₆₃ et R₈₀ représentent chacun indépendamment un atome d'hydrogène ou un groupe alkyle en (C₁-C₄) ;
R₇ représente un atome d'hydrogène, un groupe alcényle en (C₃-C₆), un groupe alcynyle en (C₃-C₆), un groupe benzyle ou un groupe alkyle en (C₁-C₄) éventuellement substitué par un groupe cyano ou un groupe C(O)R₆₂ ;
R₈ et R₂₇ représentent chacun indépendamment un atome d'hydrogène, un groupe alkyle en (C₁-C₄) ou un groupe alcoxy en (C₁-C₄) ;
R₉ et R₉₀ représentent chacun indépendamment un groupe alkyle en (C₁-C₆) ;
R₁₀ représente un atome d'hydrogène, un groupe alkyle en (C₁-C₆), un groupe phényle ou un groupe benzyle ;
R₁₃, R₂₄ et R₃₆ représentent chacun indépendamment un atome d'hydrogène, un groupe alkyle en (C₁-C₆) ou un atome d'halogène ;
R₂₃ représente un atome d'hydrogène ou un groupe NR₆₃R₆₄ ;
R₃₄ représente un atome d'hydrogène, un groupe alkyle en (C₁-C₄) ou un groupe halogénoalkyle en (C₁-C₄) ;
R₃₇ représente un atome d'hydrogène, un groupe alkyle en (C₁-C₄) ou un groupe alcoxyalkyle en (C₂-C₈) ;
R₃₈ et R₃₉ représentent chacun indépendamment un atome d'hydrogène, un groupe alkyle en (C₁-C₄), un groupe halogénoalkyle en (C₁-C₄), un groupe alcényle en (C₃-C₆) ou un groupe alcynyle en (C₃-C₆) ;
R₄₀, R₄₁ et R₄₂ représentent chacun indépendamment un atome d'hydrogène, un atome d'halogène, un groupe cyano, un groupe OR₆₅, un groupe C(O)R₆₆, un groupe C(S)R₆₇, un groupe CO₂R₆₈, un groupe C(=NOR₆₉), un groupe alkyle en (C₁-C₈), un groupe cycloalkyle en (C₃-C₇), un groupe alcényle en (C₂-C₈) ou
un groupe alcynyle en (C₂-C₈), dans lequel chaque groupe est éventuellement substitué par une quelconque combinaison d'un à six atomes d'halogène, d'un à trois groupes alcoxy en (C₁-C₁₀), d'un à deux groupes halogénoalcoxy en (C₁-C₆), d'un à deux groupes NR₇₀R₇₁, d'un à deux groupes S(O)_{q}R₇₂, d'un à deux groupes cyano, d'un à deux groupes cycloalkyle en (C₃-C₇), d'un groupe OSO₂R₇₃, d'un à deux groupes C(O)R₇₄, d'un à deux groupes CO₂R₇₅, d'un à deux groupes C(O)SR₇₆, d'un à deux groupes C(O)NR₇₇R₇₈, d'un à trois groupes OR₇₉, d'un à deux groupes P(O)(OR₈₀)₂, d'un groupe 1,3-dioxolane éventuellement substitué par un à trois groupes alkyle en (C₁-C₄), ou d'un groupe 1,3-dioxane éventuellement substitué par un à trois groupes alkyle en (C₁-C₄),
ou
un groupe phényle ou un groupe benzyle éventuellement substitué par une quelconque combinaison d'un à trois atomes d'halogène, d'un à trois groupes alkyles en (C₁-C₆), d'un à trois groupes alcoxy en (C₁-C₆), d'un groupe cycloalkyle en (C₃-C₇), d'un groupe halogénoalkyle en (C₁-C₄), d'un groupe alkylthio en (C₁-C₄) , d'un groupe cyano, d'un groupe nitro, d'un groupe C(O)R₈₁, d'un groupe C(O)R₈₂, d'un groupe OR₈₃, d'un groupe SR₈₄, d'un groupe (alcoxy en (C₁-C₆))méthyle, d'un groupe hydroxyméthyle, d'un groupe (alcényl en (C₃-C₈))oxyméthyle ou d'un groupe (halogénoalcoxy en (C₁-C₈))méthyle ;
R₄₃, R₄₄, R₄₇ et R₄₈ représentent chacun indépendamment un atome d'hydrogène, un groupe alkyle en (C₁-C₄), un groupe halogénoalkyle en (C₁-C₄), un groupe alcényle en (C₃-C₆), un groupe alcynyle en (C₃-C₆) ou un groupe cycloalkyle en (C₃-C₇), ou bien R₄₃ et R₄₄, ou R₄₇ et R₄₈ peuvent être pris ensemble avec l'atome auquel ils sont attachés pour former un groupe cycloalkyle en (C₃-C₇) ;
R₄₉, R₅₀ et R₈₆ représentent chacun indépendamment un groupe alkyle en (C₁-C₆), un groupe NR₉₃R₉₄, un groupe halogénoalkyle en (C₁-C₄), un groupe alcényle en (C₃-C₆), un groupe alcynyle en (C₃-C₆) ou un groupe benzyle ;
R₅₁, R₅₂, R₅₃, R₅₄, R₅₅, R₅₇, R₅₈, R₅₉, R₆₀, R₆₁, R₆₂, R₇₁, R₇₃, R₇₄, R₇₈, R₈₇ et R₉₂ représentent chacun indépendamment un atome d'hydrogène, un groupe alkyle en (C₁-C₆), un groupe cycloalkyle en (C₃-C₇), un groupe halogénoalkyle en (C₁-C₆), un groupe alcényle en (C₃-C₆), un groupe alcynyle en (C₃-C₆), un groupe phényle ou un groupe benzyle ;
R₇₉, R₈₃ et R₈₄ représentent chacun indépendamment un atome d'hydrogène, un groupe C(O)R₈₅, un groupe SO₂R₈₆, un groupe halogénoalkyle en (C₁-C₆), un groupe alcényle en (C₂-C₆), un groupe cycloalcényle en (C₅-C₈), un groupe alcynyle en (C₂-C₆), un groupe phényle, un groupe benzyle, ou un groupe alkyle en (C₁-C₁₀) éventuellement substitué par un groupe hydroxyle, un groupe benzyloxy, un groupe OC(O)R₈₇, un groupe alcoxy en (C₁-C₆), un groupe CO₂R₈₈, un groupe C(O)R₈₉, un groupe C(OR₉₀)₂, un groupe C(O)NR₉₁R₉₂ ou un groupe cyano ;
R₉₃ et R₉₄ représentent chacun indépendamment un atome d'hydrogène, un groupe halogénoalkyle en (C₁-C₄), un groupe alcényle en (C₂-C₆), un groupe cycloalkyle en (C₃-C₈),
un groupe alkyle en (C₁-C₈) éventuellement substitué par un ou deux groupes alcoxy en (C₁-C₄) ou un groupe cyanoalkyle,
ou un groupe benzyle ou un groupe phényle éventuellement substitué par une quelconque combinaison d'un à trois atomes d'halogène, d'un à trois groupes alkyle en (C₁-C₄), d'un à trois groupes halogénoalkyle en (C₁-C₄), d'un à trois groupes alcoxy en (C₁-C₄), d'un à trois groupes halogénoalcoxy en (C₁-C₄), d'un groupe cyano ou d'un groupe nitro, et
lorsque R₉₃ et R₉₄ sont pris ensemble avec l'atome auquel ils sont attachés, ils forment un cycle hétérocyclique, bicyclique fusionné ou monocyclique de 5 à 12 membres éventuellement substitué par un ou plusieurs groupes choisis indépendamment parmi les groupes halogène, cyano, nitro, amino, hydroxyle, alkyle en (C₁-C₄), halogénoalkyle en (C₁-C₄), alcoxy en (C₁-C₄), halogénoalcoxy en (C₁-C₄) et halogénoalkylsulfonyle en (C₁-C₄) ;
p et q représentent chacun indépendamment 0,1,ou 2 ; et
Z et Z₁ représentent chacun indépendamment un groupe alkyle en (C₁-C₄).

10. Procédé selon la revendication 9, dans lequel
Y représente un atome d'hydrogène ou un groupe méthyle ;
X₁ représente un atome d'hydrogène, un atome de fluor ou un groupe alkyle en (C₁-C₃) éventuellement substitué par un groupe époxy ;
X₂ représente un atome d'hydrogène, un groupe NRR₁ halogéné, un groupe CO₂R₂, un groupe C(O)R₃, un groupe OR₄, un groupe SO₂R₅, un groupe SO₂NR₆R₇, un groupe C(R₈)(OR₉)₂, un groupe C(R₁₀)=NOR₁₁, un groupe C(R₁₂)=C(R₁₃)-C(OR₁₄)=NOR₁₅, un groupe CH₂O-NCO₂R₁₆,
un groupe 1,3-dioxolane éventuellement substitué par un groupe alcoxy en (C₁-C₆) ou un ou deux groupes alkyle en (C₁-C₄),
un groupe 1,3-dioxolinone éventuellement substitué par un groupe alcoxy en (C₁-C₆) ou un ou deux groupes alkyles en (C₁-C₄), ou
un groupe alkyle en (C₁-C₄) éventuellement substitué par un groupe CO₂R₂ et un atome d'halogène, et
X₃ représente un atome d'hydrogène, un atome d'halogène, un groupe halogénoalkyle en (C₁-C₄), un groupe CO₂R₁₇, un groupe cyano, un groupe halogénoalcoxy en (C₁-C₄), un groupe OR₁₈, ou un groupe alkyle en (C₁-C₄), ou
lorsque X₁ et X₂ sont pris ensemble avec les atomes auxquels ils sont attachés, ils peuvent former un cycle de cinq ou six membres dans lequel X₁X₂ ou X₂X₁ est représenté par : -OC(R₂₀)(R₂₁)O-, -CH₂S(O)ₚN(R₂₂)-, -SC(R₂₃)=N-, -CH=CH-CH(R₁₁)O-, -OC(O)N-, -SC(R₂₄)=N-, -ON(R₂₅)C(O)-, -OC(CO₂R₂₆)=CH-, -NC(R₂₈)=C(SR₂₉)-, -CH=C(CO₂R₃₀)O-, -CH₂CH(R₃₁)O- ou -OC(R₃₂)(R₃₃)C(O)-, ou
lorsque X₂ et X₃ sont pris ensemble avec les atomes auxquels ils sont attachés, ils peuvent former un cycle de cinq ou six membres dans lequel X₂X₃ ou X₃X₂ est représenté par : -NC(R₃₄)=NC(S)-, -N(R₃₅)N=C(R₃₆)-, -N(R₃₇)C(R₃₈)=N-, -N(R₃₈)C(O)CH₂O-, -N(R₃₉)C(O)CH=CH-, -S-N=C(R₄₀)-, -O-N=C(R₄₁)-, -N=N-N(R₄₂)-, -C(R₄₃)(R₄₄)C(O)N(R₄₅)- ou -N(R₄₆)C(O)C(R₄₇)(R₄₈)- ;
X₄ représente un atome d'hydrogène, un atome d'halogène ou un groupe OR₁₉ ;
X₅ représente un atome d'hydrogène ou un atome d'halogène ;
R, R₆₄, R₆₉ et R₇₇ représentent chacun indépendamment un atome d'hydrogène, un groupe SO₂R₄₉ ou un groupe alkyle en (C₁-C₄) ;
R₁ représente un atome d'hydrogène, un groupe SO₂R₅₀, un groupe C(O)R₅₁, un groupe amino ou un groupe alkyle en (C₁-C₄) éventuellement substitué par un groupe CO₂R₅₂ ou un groupe C(O)R₅₃ ;
R₂, R₁₆, R₁₇, R₂₆, R₃₀, R₆₈, R₇₅, R₇₆, R₈₂ et R₈₈ représentent chacun indépendamment un atome d'hydrogène, un groupe alcényle en (C₃-C₆) ou un groupe alkyle en (C₁-C₄) éventuellement substitué par un groupe CO₂R₅₄, un groupe morpholine ou un groupe C(O)R₅₅ ;
R₃, R₆₆, R₆₇, R₈₅ et R₈₉ représentent chacun indépendamment un atome d'hydrogène, un groupe alkyle en (C₁-C₄) ou un groupe NR₅₆R₅₇ ;
R₄, R₁₈, R₁₉ représentent chacun indépendamment un atome d'hydrogène, un groupe alkyle en (C₁-C₄), un groupe halogénoalkyle en (C₁-C₄), un groupe C(O)R₅₈, un groupe alcényle en (C₃-C₄) ou un groupe alcynyle en (C₃-C₄) ;
R₅₆ représente un groupe SO₂R₄₉ ;
R₅₇ représente un atome d'hydrogène ou un groupe alkyle en (C₁-C₄) ;
R₅ et R₇₂ représentent chacun indépendamment un groupe NR₆₀R₆₁ ou un groupe indazole ;
R₆, R₁₁, R₁₂, R₁₄, R₁₅, R₂₀, R₂₁, R₂₂, R₂₅, R₂₈, R₂₉, R₃₁, R₃₂, R₃₃, R₃₅, R₄₅, R₄₆ et R₈₀ représentent chacun indépendammant un atome d'hydrogène ou un groupe méthyle ;
R₇ représente un groupe alkyle en (C₁-C₄) éventuellement substitué par un groupe cyano ou un groupe C(O)R₆₂ ;
R₈ représente un atome d'hydrogène ou un groupe alcoxy en (C₁-C₄) ;
R₉ et R₉₀ représentent chacun indépendamment un groupe alkyle en (C₁-C₄) ;
R₁₀ représente un atome d'hydrogène ou un groupe alkyle en (C₁-C₃) ;
R₁₃, R₂₄ et R₃₆ représentent chacun indépendammant un atome d'hydrogène ou un atome de chlore ;
R₂₃ représente un groupe NR₆₃R₆₄ ;
R₃₄ représente un groupe halogénoalkyle en (C₁-C₃) ;
R₃₇ représente un groupe alcoxyalkyle en (C₂-C₄) ;
R₃₈ et R₃₉ représentent chacun indépendamment un groupe halogénoalkyle en (C₁-C₃), un groupe alkyle en (C₁-C₃) ou un groupe propargyle ;
R₄₀, R₄₁ et R₄₂ représentent chacun indépendamment un atome d'hydrogène, un groupe C(O)R₆₆, C(S)R₆₇, CO₂R₆₈, C(=NOR₆₉),
un groupe alkyle en (C₁-C₃) éventuellement substitué par une quelconque combinaison d'un ou deux atomes d'halogène, d'un ou deux groupes alcoxy en (C₁-C₃), d'un ou deux groupes halogénoalcoxy en (C₁-C₃), d'un groupe SO₂R₇₂, d'un ou deux groupes cyano, d'un groupe cycloalkyle en (C₃-C₅), d'un groupe OSO₂R₇₃, d'un groupe C(O)R₇₄, d'un groupe CO₂R₇₅, d'un groupe C(O)SR₇₆, d'un groupe C(O)NR₇₇R₇₈, d'un ou deux groupes OR₇₉, d'un groupe P(O)(OR₈₀)₂, d'un groupe 1,3-dioxolane ou d'un groupe 1,3-dioxane, ou
un groupe phényle éventuellement substitué par une quelconque combinaison d'un atome d'halogène, d'un ou deux groupes méthyle, d'un groupe méthoxy, d'un groupe halogénométhyle ou d'un groupe OR₈₃ ;
R₄₃, R₄₄, R₄₇ et R₄₈ représentent chacun indépendamment un atome d'hydrogène ou un groupe méthyle, ou bien R₄₃ et R₄₄ ou R₄₇ et R₄₈ peuvent être pris ensemble avec l'atome auquel ils sont attachés pour former un groupe cyclopropyle ;
R₄₉, R₅₀ et R₈₆ représentent chacun indépendamment un groupe alkyle en (C₁-C₄) ou un groupe NR₉₃R₉₄ ;
R₅₁, R₅₂, R₅₃, R₅₄, R₅₅, R₅₈, R₆₀, R₆₁, R₆₂, R₇₃, R₇₄, R₇₈ et R₈₇ représentent chacun indépendamment un atome d'hydrogène, un groupe alkyle en (C₁-C₄) ou un groupe halogénoalkyle en (C₁-C₄) ;
R₇₉ et R₈₃ représentent chacun indépendamment un atome d'hydrogène, un groupe C(O)R₈₅, un groupe SO₂R₈₆, un groupe halogénoalkyle en (C₁-C₄), un groupe alcényle en (C₃-C₄) ou un groupe alkyle en (C₁-C₃) substitué par un groupe OC(O)R₈₇, un groupe CO₂R₈₈, un groupe C(O)R₈₉, un groupe C(OR₉₀)₂ ou un groupe cyano ;
R₉₃ et R₉₄ représentent chacun indépendamment un atome d'hydrogène ou un groupe alkyle en (C₁-C₈) ;
p est équivalent à 0, 1 ou 2 ; et
Z et Z₁ représentent chacun indépendamment un groupe méthyle ou un groupe éthyle.

11. Procédé selon la revendication 1 pour la préparation d'un composé 6-(trifluorométhyl)uracile ayant la formule de structure VI : 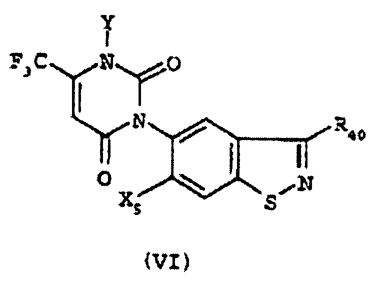 dans laquelle :
Y représente un atome d'hydrogène ou un groupe méthyle ;
X₅ représente un atome d'hydrogène ou un atome d'halogène ;
R₄₀ représente un atome d'hydrogène, un groupe C(O)R₆₆, un groupe C(S)R₆₇, un groupe CO₂R₆₈,
un groupe alkyle en (C₁-C₃) éventuellement substitué par une quelconque combinaison d'un ou deux atomes d'halogène, d'un ou deux groupes alcoxy en (C₁-C₃), d'un ou deux groupes halogénoalcoxy en (C₁-C₃), d'un groupe SO₂R₇₂, d'un ou deux groupes cyano, d'un groupe cycloalkyle en (C₃-C₅), d'un groupe OSO₂R₇₃, d'un ou deux groupes OR₇₉, d'un groupe P(O)(OR₈₀)₂, d'un groupe 1,3-dioxolane ou d'un groupe 1,3-dioxane, ou
un groupe phényle éventuellement substitué par une quelconque combinaison d'un atome d'halogène, d'un ou deux goupes méthyle, d'un groupe méthoxy, d'un groupe halogénométhyle ou d'un groupe OR₈₃ ;
R₆₆, R₆₇, R₈₅ et R₈₉ représentent chacun indépendamment un atome d'hydrogène, un groupe alkyle en (C₁-C₄) ou un groupe NR₅₆R₅₇ ;
R₅₆ représente un groupe SO₂R₄₉ ;
R₅₇ représente un atome d'hydrogène ou un groupe alkyle en (C₁-C₄) ;
R₄₉ et R₈₆ représentent chacun indépendamment un groupe alkyle en (C₁-C₄) ou un groupe NR₉₃R₉₄ ;
R₉₃ et R₉₄ représentent chacun indépendamment un atome d'hydrogène ou un groupe alkyle en (C₁-C₈) ;
R₆₈ et R₈₈ représentent chacun indépendamment un atome d'hydrogène, un groupe alcényle en (C₃-C₆) ou un groupe alkyle en (C₁-C₄) éventuellemnt substitué par un groupe CO₂R₅₄,
un groupe morpholine ou un groupe C(O)R₅₅ ;
R₅₄, R₅₅, R₆₀, R₆₁, R₇₃ et R₈₇ représentent chacun indépendamment un atome d'hydrogène, un groupe alkyle en (C₁-C₄) ou un groupe halogénoalkyle en (C₁-C₄) ;
R₇₂ représente un groupe NR₆₀R₆₁ ou un groupe indazole ;
R₇₉ et R₈₃ représentent chacun indépendamment un atome d'hydrogène, un groupe C(O)R₈₅, un groupe SO₂R₈₆, un groupe halogénoalkyle en (C₁-C₄), un groupe alcényle en (C₃-C₄) ou
un groupe alkyle en (C₁-C₃) substitué par un groupe OC(O)R₈₇, un groupe CO₂R₈₈, un groupe C(O)R₈₉, un groupe C(OR₉₀)₂ ou un groupe cyano ;
R₈₀ représente un atome d'hydrogène ou un groupe méthyle ; et
R₉₀ représente un groupe alkyle en (C₁-C₄).
